# EUROPEAN PATENT SPECIFICATION

(11) **EP 1 747 295 B1**
(45) Date of publication and mention of the grant of the patent: **11.01.2012**
(21) Application number: 05856708.2
(22) Date of filing: 12.05.2005
(51) Int. Cl.: C12Q 1/68

(54) **BIO-BARCODE BASED DETECTION OF TARGET ANALYTES**
BIO-BARCODE ERKENNUNG FÜR ZIELMOLEKÜLE
DETECTION D'ANALYTES CIBLES FONDEE SUR DES CODES A BARRES BIOLOGIQUES

(30) Priority: 12.05.2004 US 570723 P; 01.07.2004 US 585294 P; 19.01.2005 US 645455 P
(43) Date of publication of application: 31.01.2007
(73) Proprietor: Nanosphere, Inc., Northbrook, Illinois 60062 (US)
(72) Inventor: MIRKIN, Chad, A., Wilmette, Illinois 60091 (US); NAM, Jwa-min, Seoul 157-863 (KR); OH, Byung-keun, Seoul 121-742 (KR); THAXTON, C., Shad, Chicago, IL 60614 (US); GEORGANOPOULOU, Dimitra, Chicago, Illinois 60660 (US)
(74) Representative: Grund, Martin
(86) International application number: PCT/US2005/016545
(87) International publication number: WO 2006/078289

(56) References cited:
- EP-A- 1 016 731
- WO-A-90/02205
- WO-A-96/32640
- WO-A-98/04740
- WO-A-03/031591
- WO-A-03/102590
- WO-A-2005/003394
- US-A1- 2004 023 248
- NAM J.-M. ET AL.,: "nanoparticle-based bio-bar-codes for the ultrasensitive detection of proteins" SCIENCE, vol. 301, 26 September 2003 (2003-09-26), pages 1884-1886, XP002396891

## Description

### FIELD OF THE INVENTION:

The present invention relates to a screening method for detecting for the presence or absence of one or more target analytes, e.g., proteins, nucleic acids, or other compounds in a sample. In particular, the present invention relates to a method that utilizes reporter oligonucleotides as biochemical barcodes for detecting one or more analytes in a solution.

### BACKGROUND OF THE INVENTION:

The detection of analytes is important for both molecular biology research and medical applications. Diagnostic methods based on fluorescence, mass spectroscopy, gel electrophoresis, laser scanning and electrochemistry are now available for identifying a variety of protein structures.¹⁻⁴ Antibody-based reactions are widely used to identify the genetic protein variants of blood cells, diagnose diseases, localize molecular probes in tissue, and purify molecules or effect separation processes.⁵ For medical diagnostic applications (e.g. malaria and HIV), antibody tests such as the enzyme-linked immunosorbent assay, Western blotting, and indirect fluorescent antibody tests are extremely useful for identifying single target protein structures. ^{6,7} Rapid and simultaneous sample screening for the presence of multiple antibodies would be beneficial in both research and clinical applications. However, it is difficult, expensive, and time-consuming to simultaneously detect several protein structures under assay conditions using the aforementioned related protocols.

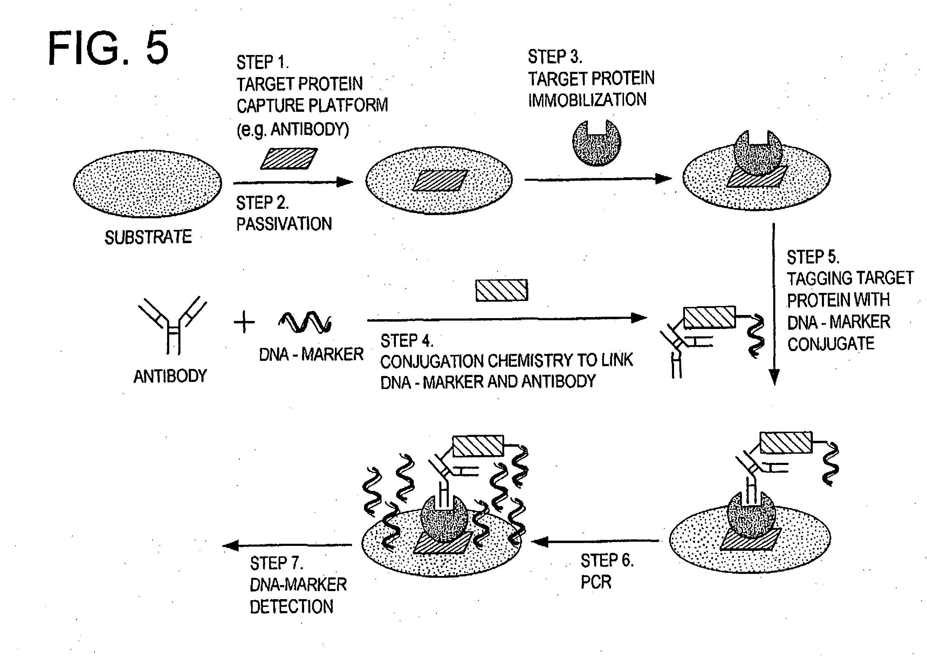

Polymerase chain reaction (PCR) and other forms of target amplification have enabled rapid advances in the development of powerful tools for detecting and quantifying DNA targets of interest for research, forensic, and clinical applications ²⁶⁻³². The development of comparable target amplification methods for proteins could dramatically improve medical diagnostics and the developing field of proteomics ³³⁻³⁶. Although one cannot yet chemically duplicate protein targets, it is possible to tag such targets with oligonucleotide markers that can be subsequently amplified with PCR and then use DNA detection to identify the target of interest ³⁷⁻⁴⁵. This approach, often referred to as immuno-PCR, allows one to detect proteins with DNA labels in a variety of different formats (Figure 5). To date, all immuno-PCR approaches involve heterogeneous assays, which involve initial immobilization of a target analyte to a surface with subsequent detection using an antibody with a DNA label (for example, see U.S. Patent nos. 5,635,602, and 5,665,539). The DNA label is typically strongly bound to the antibody (either through covalent interactions or strepavidin-biotin binding). Although theses approaches are notable advances in protein detection, they have several drawbacks: 1) limited sensitivity because of a low ratio of DNA identification sequence to detection antibody; 2) slow target binding kinetics due to the heterogeneous nature of the target capture procedure, which increases assay time and decreases assay sensitivity (Step 3 in Figure 5); 3) complex conjugation chemistries that are required to chemically link the antibody and DNA-markers (Step 4 in Figure 5); and 4) require a PCR amplification step⁴⁵. Therefore, a sensitive, and rapid method for detecting target analytes in a sample that is amenable to multiplexing and easy to implement is needed.

Various methods and systems for detecting target analytes have been described. For example, WO 03/102590 describes a detection method using two-component conjugate systems comprising a catalytic label and a substrate label; WO 96/32604 discloses a signal amplification system for immunoassays using a DNA labeled antibody as probe; WO 03/031391 relates to methods of target analyte detection using an oligonucleotide ID tag; WO 98/04740 concerns a method of detecting nucleic acid using nanoparticles having oligonucleotides attached thereto, whereby the presence of the target nucleic acid triggers aggregation of the nanoparticles; EP 1016 731 relates to a method of detecting nucleic acid using a labeled, cleavable oligonucleotide probe that is complementary to a portion of the target nucleic acid; Nam et al. (71) discloses a detection method using a nanoparticle having bound to it DNA barcodes; US 2004/0023248 discloses a bipartite dendrimeric capture probe for detecting target nucleic acid; and WO 05/003394 describes a method utilizing reporter oligonucleotides as biochemical barcodes for detecting target analytes in a sample.

For DNA detection methods, many assays have been developed using radioactive labels, molecular fluorophores, chemiluminescence schemes, electrochemical tags, and most recently, nanostructure-based labels.⁶¹⁻⁷⁰ Although some nanostructure-based methods are approaching PCR in terms of sensitivity, none thus far have achieved the 1-10 copy sensitivity level offered by PCR. A methodology that allows for PCR-like signal amplification without the complexity, expense, and time and labor intensive aspects associated with PCR would provide significant advantages over such PCR-based methods.

### SUMMARY OF THE INVENTION

The invention is defined by the appended claims.

The present invention relates to methods, probes, compositions, and kits that utilize oligonucleotides as biochemical barcodes for detecting multiple analytes in one solution. The approach takes advantage of recognition elements of specific binding pairs functionalized either directly or indirectly with nanoparticles, and the previous observation that hybridization events that result in the aggregation of gold nanoparticles can significantly alter their physical properties (e.g. optical, electrical, mechanical). ⁸⁻¹² The general idea is that each recognition element of a specific binding pair can be associated with a different oligonucleotide sequence with discrete and tailorable hybridization and melting properties and a physical signature associated with the nanoparticles. The discrete hybridization and melting properties can be used to decode a series of analytes in a multi-analyte assays by creating a change in a physical signature associated with the nanoparticles or by detection of oligonucleotide sequence(s), through hybridization/dehybridization or melting/annealing events.

Disclosed are, methods for detecting for the presence of one or more target analytes in a sample, each target analyte having at least two binding sites for specific binding interactions with specific binding complements, in a sample comprising the steps of:
a) providing at least one type of capture substrates, the capture substrates having bound thereto at least one specific binding complement of the specific target analyte that binds to at least a first binding site of the specific target analyte;
b) providing at least one type of microparticle detection probes, the microparticle detection probes comprising a microparticle having bound thereto (i) at least one specific binding complement to a specific target analyte and (ii) a plurality of DNA barcodes, the DNA barcodes are labeled with a reporter label, wherein the specific binding complement bound to the microparticle detection probes binds to at least a second binding site of the target analyte;
c) contacting the capture substrates with a sample believed to contain target analytes under conditions effective to allow for binding of the specific target analyte to the specific binding complement bound to the capture substrate so as to immobilize the target analytes onto the capture substrates;
d) contacting the immobilized target analytes with at least one type of microparticle detection probes under conditions effective to allow for binding between the target analytes and the specific binding complement bound to the microparticle detection probes to the analyte and to form a complex in the presence of the target analyte on the capture substrate;
e) optionally isolating and washing the capture substrate to remove unbound microparticle detection probes; and
f) detecting for the presence of the DNA barcode wherein the presence of the DNA barcode is indicative of the presence of a specific target analyte in the sample.

Disclosed are methods for detecting for the presence of one or more target analytes in a sample, each target analyte having at least two binding sites for specific binding interactions with specific binding complements, in a sample comprising the steps of:
a) providing at least one type of capture substrates, the capture substrates having bound thereto at least one specific binding complement of the specific target analyte that binds to at least a first binding site of the specific target analyte;
b) providing at least one type of microparticle detection probes, the microparticle detection probes comprising a microparticle having bound thereto (i) at least one specific binding complement to a specific target analyte and (ii) a plurality of DNA barcodes, the DNA barcodes are fluorescently labeled, wherein the specific binding complement bound to the microparticle detection probes binds to at least a second binding site of the target analyte;
c) contacting the capture substrates with a sample believed to contain target analytes under conditions effective to allow for binding of the specific target analyte to the specific binding complement bound to the capture substrate so as to immobilize the target analytes onto the capture substrates;
d) contacting the immobilized target analytes with at least one type of microparticle detection probes under conditions effective to allow for binding between the target analytes and the specific binding complement bound to the microparticle detection probes to the analyte and to form a complex in the presence of the target analyte on the capture substrate;
e) optionally isolating and washing the capture substrate to remove unbound microparticle detection probes; and
f) detecting for the presence of a fluorescent signal from the DNA barcodes wherein the presence of a fluorescent signal is indicative of the presence of a specific target analyte in the sample.

Disclosed are methods for detecting for the presence of one or more target analytes in a sample, each target analyte having at least two binding sites for specific binding interactions with specific binding complements, in a sample comprising the steps of:
a) providing at least one type of capture substrates, the capture substrates having bound thereto at least one specific binding complement of the specific target analyte that binds to at least a first binding site of the specific target analyte;
b) providing at least one type of microparticle detection probes, the microparticle detection probes comprising a microparticle having bound thereto (i) at least one specific binding complement to a specific target analyte and (ii) a plurality of DNA barcodes, the DNA barcodes are fluorescently labeled, wherein the specific binding complement bound to the microparticle detection probes binds to at least a second binding site of the target analyte;
c) contacting the capture substrates with a sample believed to contain target analytes under conditions effective to allow for binding of the specific target analyte to the specific binding complement bound to the capture substrate so as to immobilize the target analytes onto the capture substrates;
d) contacting the immobilized target analytes with at least one type of microparticle detection probes under conditions effective to allow for binding between the target analytes and the specific binding complement bound to the microparticle detection probes to the analyte and to form a complex in the presence of the target analyte on the capture substrate;
e) optionally isolating and washing the capture substrate to remove unbound microparticle detection probes;
f) subjecting the isolated washed capture substrate to conditions effective to release the DNA barcodes; and
g) detecting for the presence of the fluorescent signal of the DNA barcodes wherein the presence of a fluorescent signal of the DNA barcode is indicative of the presence of a specific target analyte in the sample.

Furthermore, methods for detecting for the presence of one or more target analytes in a sample are disclosed each target analyte having at least two binding sites for specific binding interactions with specific binding complements, in a sample comprising the steps of
a) providing at least one type of capture substrates, the capture substrates having bound thereto at least one specific binding complement of the specific target analyte that binds to at least a first binding site of the specific target analyte;
b) providing at least one type of microparticle detection probes, the microparticle detection probes comprising a microparticle having bound thereto (i) at least one specific binding complement to a specific target analyte and (ii) a plurality of DNA barcodes, the DNA barcodes comprising a reporter label, wherein the specific binding complement bound to the microparticle detection probes binds to at least a second binding site of the target analyte;
c) contacting the capture substrates with a sample believed to contain target analytes under conditions effective to allow for binding of the specific target analyte to the specific binding complement bound to the capture substrate so as to immobilize the target analytes onto the capture substrates;
d) contacting the immobilized target analytes with at least one type of microparticle detection probes under conditions effective to allow for binding between the target analytes and the specific binding complement bound to the microparticle detection probes to the analyte and to form a complex in the presence of the target analyte on the capture substrate;
e) optionally isolating and washing the capture substrate to remove unbound microparticle detection probes;
f) subjecting the isolated washed capture substrate to conditions effective to release the DNA barcodes; and
g) detecting for the presence of the reporter label of the DNA barcodes wherein the presence of the reporter label of the DNA barcode is indicative of the presence of a specific target analyte in the sample.

Disclosed are methods for detecting for the presence of one or more target analytes in a sample, each target analyte having at least two binding sites for specific binding interactions with specific binding complements, in a sample comprising the steps of:
a) providing at least one type of capture substrates, the capture substrates having bound thereto at least one specific binding complement of the specific target analyte that binds to at least a first binding site of the specific target analyte;
b) providing at least one type of particle detection probes, the particle detection probes comprising a microparticle or nanoparticle having bound thereto (i) at least one specific binding complement to a specific target analyte and (ii) a plurality of DNA barcodes, the DNA barcodes are optionally labeled with a reporter label, wherein the specific binding complement bound to the particle detection probes binds to at least a second binding site of the target analyte;
c) contacting the capture substrates with a sample believed to contain target analytes under conditions effective to allow for binding of the specific target analyte to the specific binding complement bound to the capture substrate so as to immobilize the target analytes onto the capture substrates;
d) contacting the immobilized target analytes with at least one type of particle detection probes under conditions effective to allow for binding between the target analytes and the specific binding complement bound to the particle detection probes to the analyte and to form a complex in the presence of the target analyte on the capture substrate;
e) optionally isolating and washing the capture substrate to remove unbound particle detection probes;
f) releasing the DNA barcodes from the particle detection probes by a chemical releasing agent; and
g) detecting for the presence of the DNA barcode wherein the presence of the DNA barcode is indicative of the presence of a specific target analyte in the sample.

Disclosed are methods for detecting for the presence of one or more target analytes in a sample, each target analyte having at least two binding sites for specific binding interactions with specific binding complements, in a sample comprising the steps of:
a) providing at least one type of capture substrates that are separable from the sample, the capture substrates having bound thereto at least one specific binding complement of the specific target analyte that binds to at least a first binding site of the specific target analyte;
b) contacting the capture substrates with a sample believed to contain target analyses under conditions effective to allow for binding of the specific target analyte to the specific binding complement bound to the capture substrate so as to immobilize the target analytes onto the capture substrates;
c) optionally separating the capture substrate and any target analytes bound thereto from the sample;
d) providing at least one type of particle detection probes, the particle detection probes comprising a particle having bound thereto (i) at least one specific binding complement to a specific target analyte and (ii) a plurality of oligonucleotides that are optionally labeled with a reporter label, wherein the specific binding complement bound to the particle detection probes binds to at least a second binding site of the target analyte;
e) contacting the immobilized target analytes with at least one type of particle detection probes under conditions effective to allow for binding between the target analytes and the specific binding complement bound to the particle detection probes to the analyte and to form a complex in the presence of the target analyte on the capture substrate, wherein the complex comprises the target analyte and capture substrate and particle detection probe;
f) optionally separating the complex from unbound particle detection probes; and
g) detecting for the presence of the complex wherein the presence of the complex is indicative of the presence of a specific target analyte in the sample.

As discussed herein, the assay methods are ultrasensitive and can be performed in less than 60 minutes without the need for enzymatic amplification or a scanometric detection scheme. By eliminating both PCR and scanometric method without losing sensitivity, the methods of the invention overcome some of the drawbacks that both of these conventional methods, such as long assay times and assay complexity. Moreover, non-scientists such as nurses, medical doctors, and soldiers should be able to learn how to use the methods of the invention after simple training due to its simplicity. The methods of the invention can be easily multiplexed by using different fluorophores for different target proteins and total assay time can be even more decreased by optimizing probe concentrations and reaction conditions. In addition, the methods of the invention can be used to detect any target that can be detected in a sandwich assay, including proteins, DNA, RNA, small molecules, and metal ions. Furthermore, other types of reporter groups can be attached to the barcodes allowing for methods other than fluorescence-detection to identify the barcodes and amplified detection signal. These include, but are limited to, redox-active groups, groups with electrical signatures, radioactive groups, catalytic groups, groups with distinct absorption characteristics, and groups with Raman signatures. A reporter group can be anything with a distinct and measurable chemical or physical signature.

Disclosed is a method is provided for detecting for the presence or absence of one or more target analytes, the target analyte having at least two binding sites, in a sample comprising the steps of:
providing a substrate; providing one or more types of particle probes, each type of probe comprising a particle having one or more specific binding complements to a specific target analyte and one or more DNA barcodes bound thereto, wherein the specific binding complement of each type of particle probe is specific for a particular target analyte, and the DNA barcode for each type of particle probe serves as a marker for the particular target analyte;
immobilizing the target analytes onto the substrate;
contacting the immobilized target analytes with one or more types of particle probes under conditions effective to allow for binding between the target analyte and the specific binding complement to the analyte and form a complex in the presence of the target analyte;
washing the substrate to remove unbound particle probes; and
optionally amplifying the DNA barcode; and
detecting for the presence or absence of the DNA barcode wherein the presence or absence of the marker is indicative of the presence or absence of a specific target analyte in the sample.

In one aspect, the target analyte is a protein or hapten and its specific binding complement is an antibody comprising a monoclonal or polyclonal antibody.

In another aspect, DNA barcode is amplified by PCR.

In another aspect, the particle is labeled with at least two DNA barcodes.

In another aspect, the substrate is arrayed with one or more types of capture probes for the target analytes.

A method is provided for detecting for the presence or absence of one or more target analytes in a sample, each target analyte having at least two binding sites, the method comprising:
providing one or more types of capture probes bound to a substrate, each type of capture probe comprising a specific binding complement to a first binding site of a specific target analyte;
providing one or more types of detection probes, each type of detection probe comprising a nanoparticle having oligonucleotides bound thereto, one or more specific binding complements to a second binding site of the specific target analyte, and one or more DNA barcodes that serve as a marker for the particular target analyte, wherein at least a portion of a sequence of the DNA barcodes is hybridized to at least some of the oligonucleotides bound to the nanoparticles
contacting the sample, the capture probe, and the detection probe under conditions effective to allow specific binding interactions between the target analyte and the probes and to form an aggregate complex in the presence of the target analyte;
washing the substrate to remove any unbound detection probes;
detecting for the presence or absence of the DNA barcode in any aggregate complex on the substrate, wherein the detection of the presence or absence of the DNA barcode is indicative of the presence or absence of the target analyte in the sample.

In one aspect of this embodiment of invention, the detection probe comprises (i) one or more specific binding complements to the second binding site of a specific target analyte, (ii) at least one type of oligonucleotides bound to the nanoparticle, and a DNA barcode having a predetermined sequence that is complementary to at least a portion of at least one type of oligonucleotides, the DNA barcode bound to each type of detection probe serving as a marker for a specific target analyte;

In another aspect, prior to said detecting step, the method further comprising the steps of:
subjecting the aggregate complex to conditions effective to dehybridize the complex and release the DNA barcodes; and
amplifying the DNA barcode prior to said detecting.

In another aspect of this embodiment, the DNA barcode is amplified by PCR.

In another aspect of this embodiment, the capture probe is bound to a magnetic substrate such as a magnetic particle.

In another aspect, the target analyte is a target nucleic acid having a sequence of at least two portions, the detection probe comprises a nanoparticle having oligonucleotides bound thereto, at least a portion of the oligonucleotides having a sequence that is complementary to the DNA bar code, the specific binding complement of the detection probe comprising a first target recognition oligonucleotide having a sequence that is complementary to a first portion of the target nucleic acid, and the specific binding complement of the capture probes comprises second target recognition oligonucleotide having a sequence that is complementary to at least a second portion of the target nucleic acid.

In another aspect, the target analyte is a target nucleic acid having a sequence of at least two portions, the detection probe comprising a nanoparticle having oligonucleotides bound thereto, the DNA barcode having a sequence that is complementary to at least a portion of the oligonucleotides bound to the detection probe, the specific binding complement comprises a target recognition oligonucleotide having a sequence of at least first and second portions, the first portion is complementary to a first portion of the target nucleic acid and the second portion is complementary to a least a portion of the oligonucleotides bound to the nanoparticles, the specific binding complement of the substrate comprising a target recognition oligonucleotide having at least a portion that is complementary to a second portion of the target nucleic acid.

In another aspect, the detection probe comprises a dendrimer.

A method is provided for detecting for the presence or absence of one or more target analytes in a sample, each target analyte having at least two binding sites, the method comprising:
providing one or more types of capture probes, each type of capture probe comprising (i) a magnetic particle; and (ii) a first member of a first specific binding pair attached to the magnetic particle, wherein the first member of the first specific binding pair binds to a first binding site of a specific target analyte;
providing one or more types of detection probe for each target analyte, each type of detection probe comprising (i) a nanoparticle; (ii) a first member of a second specific binding pair attached to the nanoparticle, wherein the first member of the second specific binding pair binds to a second binding site of the target analyte; (iii) at least one type of oligonucleotides bound to the nanoparticle; and (iv) at least one type of DNA barcodes, each type of DNA barcode having a predetermined sequence that is complementary to at least a portion of a specific type of oligonucleotides and serves as a marker for a specific target analyte;
contacting the sample with the capture probe and the detection probe under conditions effective to allow specific binding interactions between the target analyte and the probes and to form an aggregated complex bound to the magnetic particle in the presence of the target analyte;
washing any unbound detection probes from the magnetic particle; and
detecting for the presence or absence of the DNA barcodes in the complex, wherein the detection of the DNA barcode is indicative of the presence of the target analyte.

The method can further comprise, prior to said detecting step, the steps of:
isolating the aggregated complex by applying a magnetic field; .
subjecting the aggregated complex to conditions effective to dehybridize and release the DNA barcodes from the aggregated complex;
isolating the released DNA barcodes.

In another aspect, the method further comprises amplifying the released DNA barcodes.

In another aspect, the method further comprises:
providing a substrate having oligonucleotides bound thereto, the oligonucleotides having a sequence complementary to at least a portion of the sequence of the DNA barcode;
providing a nanoparticle comprising oligonucleotides bound thereto, wherein at least portion of the oligonucleotides bound to the nanoparticles have a sequence that is complementary to at least a portion of a DNA barcode; and
contacting the DNA barcodes, the oligonucleotides bound to the substrate, and the nanoparticles under conditions effective to allow for hybridization at least a first portion of the DNA barcodes with a complementary oligonucleotide bound to the substrate and a second portion of the DNA barcodes with some of the oligonucleotides bound to the nanoparticles.

In another aspect, the DNA barcode is amplified by PCR prior to detection.

In another aspect, the method further comprises isolating the aggregated complexes prior to analyzing the aggregated complex.

In another aspect, the aggregated complex is isolated by applying a magnetic field to the aggregated complex.

In another aspect, the nanoparticles are metal nanoparticles such as gold nanoparticles or semiconductor nanoparticles.

In another aspect, the specific binding pair is an antibody and an antigen; a receptor and a ligand; an enzyme and a substrate; a drug and a target molecule; an aptamer and an aptamer target; two strands of at least partially complementary oligonucleotides.

In another aspect, the DNA barcode may be biotinylated, radioactively labeled, or fluorescently labeled.

A method is provided for detecting for the presence or absence of one or more target analytes in a sample, the method comprises:
providing at least one or more types of particle complex probes, each type of probe comprising oligonucleotides bound thereto, one or more specific binding complements of a specific target analyte, and one or more DNA barcodes that serves as a marker for the particular target analyte, wherein at least a portion of a sequence of the DNA barcodes is hybridized to at least some of the oligonucleotides bound to the nanoparticles;
contacting the sample with the particle complex probes under conditions effective to allow specific binding interactions between the target analytes and the particle complex probes and to form an aggregate complex in the presence of a target analyte; and observing whether aggregate complex formation occurred.

A method for detecting the presence or absence of one or more target analytes is disclosed analyte having at least two binding sites. The method comprises at least one type of capture probe and at least one type of detection probe for each target analyte used. These probes may be generated prior to conducting the actual assay or in situ while conducting the assay. The capture probe comprises a first member of a first specific binding pair, wherein the first member of the first specific binding pair binds to the first binding site of the target analyte, and wherein the first member of the first specific binding pair optionally binds to a substrate. In one preferred embodiment, the substrate comprises a magnetic particle. The detection probe comprises (1) a nanoparticle; (2) a first member of a second specific binding pair attached to the nanoparticle, wherein the first member of the second specific binding pair binds to a second binding site of the target analyte; and (3) at least one type of oligonucleotides bound to the nanoparticle; and (4) at least one type of DNA barcodes, each type of DNA barcode having a predetermined sequence that is complementary to at least a portion of a specific type of oligonucleotides. When employed in a sample containing the target analyte; the first member of a first specific binding pair on the capture probe binds to the first binding site of the target analyte, and the first member of a second specific binding pair on the detection probe binds to the second binding site of the target analyte. Aggregation occurs when capture probes and detection probes are brought together by the target analyte. The aggregates may be isolated and subjected to further melting analysis to identity the particular target analyte where multiple targets are present as discussed above. Alternatively, the aggregates can be dehybridized to release the DNA barcode.

In one aspect the DNA barcode in each type of particle complex probe has a sequence that is different and that serves as an identifier for a particular target analyte.

In another aspect , the method further comprises the steps of:
isolating aggregated complexes; and
analyzing the aggregated complexes to determine the presence of one or more DNA barcodes having different sequences.

In another aspect, the method further comprises the steps of:
isolating the aggregated complex;
subjecting the aggregated complex to conditions effective to dehybridize the aggregated complex and release the DNA barcode;
isolating the DNA barcode; and
detecting for the presence of one or more DNA barcodes having different sequences, wherein each DNA barcode is indicative of the presence of a specific target analyte in the sample.

In another aspect, the method further comprises the steps of:
isolating the aggregated complex;
subjecting the aggregated complex to conditions effective to dehybridize the aggregated complex and release the DNA barcode;
isolating the DNA barcode;
amplifying the isolated DNA barcode; and
detecting for the presence of one or more amplified DNA barcodes having different sequences, wherein each DNA barcode is indicative of the presence of a specific target analyte in the sample.

In another aspect, target has more than two binding sites and at least two types of particle complex probes are provided, the first type of probe having a specific binding complement to a first binding site on the target analyte and the second type of probe having a specific binding complement to a second binding site on the probe. A plurality of particle complex probes may be provided, each type of probe having a specific binding complement to different binding sites on the target analyte.

In another aspect, the detecting step for the presence of one or more DNA barcodes comprises:
providing a substrate having oligonucleotides bound thereto, the oligonucleotides having a sequence complementary to at least a portion of the sequence of the DNA barcode;
providing a nanoparticle comprising oligonucleotides bound thereto, wherein at least portion of the oligonucleotides bound to the nanoparticles have a sequence that is complementary to at least a portion of a DNA barcode; and
contacting the DNA barcodes, the oligonucleotides bound to the subsbrate, and the nanoparticles under conditions effective to allow for hybridization at least a first portion of the DNA barcodes with a complementary oligonucleotide bound to the substrate and a second portion of the DNA barcodes with some of the oligonucleotides bound to the nanoparticles; and
observing a detectable change.

In another aspect, the substrate comprises a plurality of types of oligonucleotides attached thereto in an array to allow for the detection of one or more different types of DNA barcodes.

In another aspect , the detectable change is the formation of dark areas on the substrate.

In another aspect, the detectable change is observed with an optical scanner.

In another aspect, the substrate is contacted with a silver stain to produce the detectable change.

In another aspect, the DNA barcodes are contacted with the substrate under conditions effective to allow the DNA barcodes to hybridize with complementary oligonucleotides bound to the substrate and subsequently contacting the DNA barcodes bound to the substrate with the nanoparticles having oligonucleotides bound thereto under conditions effective to allow at least some of the oligonucleotides bound to the nanoparticles to hybridize with a portion of the sequence of the DNA barcodes on the substrate.

In another aspect, the DNA barcodes are contacted with the nanoparticles having oligonucleotides bound thereto under conditions effective to allow the DNA barcodes to hybridize with at least some of the oligonucleotides bound to the nanoparticles; and subsequently contacting the DNA barcodes bound to the nanoparticles with the substrate under conditions effective to allow at least a portion of the sequence of the DNA barcodes bound to the nanoparticles to hybridize with complementary oligonucleotides bound to the substrate.

In another aspect , the DNA barcode is amplified prior to the contacting step.

In another aspect, at least two types of particle complex probes are provided, a first type of probe having a specific binding complement to a first binding site of the target analyte and a second type of probe having a specific binding complement to a second binding site of the target analyte.

Particle complex probes are provided. Thus, in one aspect of this embodiment, the particle complex probe comprises a particle having oligonucleotides bound thereto, one or more DNA barcodes, and an oligonucleotide having bound thereto a specific binding complement to a specific target analyte, wherein (i) the DNA barcode has a sequence having at least two portions; (ii) at least some of the oligonucleotides attached to the particle have a sequence that is complementary to a first portion of a DNA barcode; (iii) the oligonucleotide having bound thereto a specific binding complement have a sequence that is complementary to a second portion of a DNA barcode; and (iv) the DNA barcode in each type of particle complex probe has a sequence that is different and that serves as an identifier for a particular target analyte.

In another aspect, the particle complex probe comprises a particle having at least two types of oligonucleotides bound thereto; one or more DNA barcodes, and an oligonucleotide having bound thereto a specific binding complement to a target analyte, wherein a first type of oligonucleotides bound to the probe having a sequence that is complementary to at least a portion of the DNA barcode, the second type of oligonucleotide bound to the probe having a sequence that is complementary to at least a portion of the sequence of the oligonucleotide having a specific binding complement.

In another aspect the particle complex probe comprising a particle having oligonucleotides bound thereto, one or more DNA barcodes, and a specific binding complement to a target analyte, wherein at least a portion of the oligonucleotides bound to the particle have a sequence that is complementary to at least a portion of the sequence of the DNA barcode and where the DNA barcode serves as an identifier for a specific target analyte.

A particle complex probe is provided. Thus, a particle complex probe is provided which comprises a particle having oligonucleotides bound thereto, a DNA barcode, and an oligonucleotide having bound thereto a specific binding complement to a specific target analyte, wherein (i) the DNA barcode has a sequence having at least two portions; (ii) at least some of the oligonucleotides attached to the particle have a sequence that is complementary to a first portion of a DNA barcode; (iii) the oligonucleotide having bound thereto a specific binding complement have a sequence that is complementary to a second portion of a DNA barcode; and (iv) the DNA barcode in each type of particle complex probe has a sequence that is different and that serves as an identifier for a particular target analyte.

A particle complex probe is provided which comprises a particle having at least two types of oligonucleotides bound thereto, a DNA barcode, and an oligonucleotide having bound thereto a specific binding complement to a target analyte, wherein a first type of oligonucleotides bound to the probe having a sequence that is complementary to at least a portion of the DNA barcode, the second type of oligonucleotide bound to the probe having a sequence that is complementary to at least a portion of the sequence of the oligonucleotide having a specific binding complement.

A particle complex probe is provided which comprises a particle having oligonucleotides bound thereto, a DNA barcode, and a specific binding complement to a target analyte, wherein at least a portion of the oligonucleotides bound to the particle have a sequence that is complementary to at least a portion of the sequence of the DNA barcode and where the DNA barcode serves as an identifier for a specific target analyte.

A detection probe is provided which comprises a nanoparticle; a member of a specific binding pair bound to the nanoparticle; at least one type of oligonucleotide bound to the nanoparticle; and at least one type of DNA barcode each having a predetermined sequence, wherein each type of DNA barcode is hybridized to at least a portion of the at least one type of oligonucleotide.

Kits are provided which comprise the particle complex probe described above.

### BRIEF DESCRIPTION OF THE FIGURES

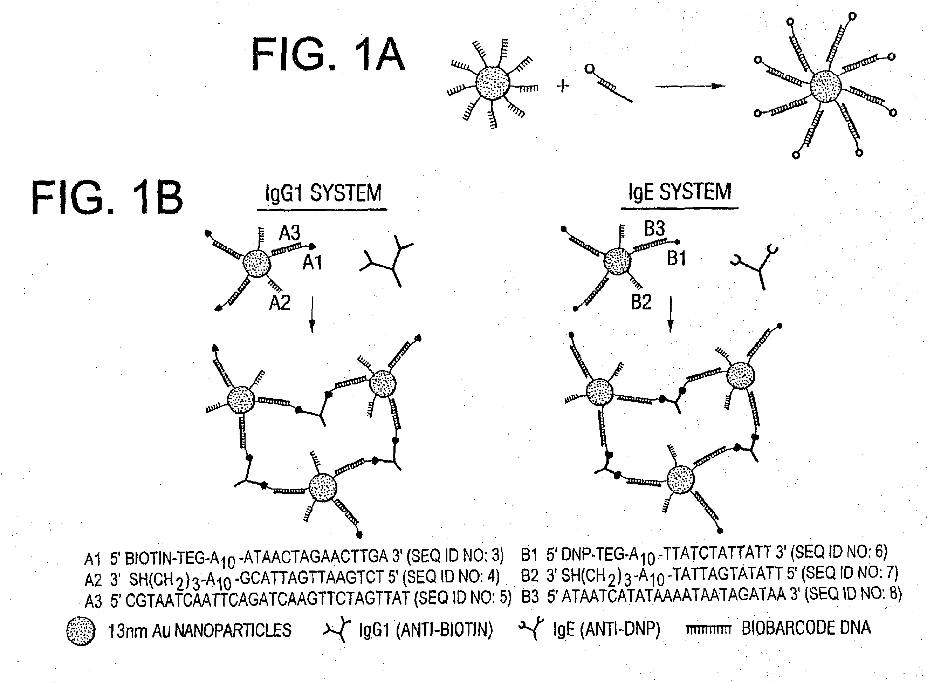

Figure 1 illustrates a DNA/Au nanoparticle-based protein detection scheme. (A) Preparation of hapten-modified nanoparticle probes. (B) Protein detection using protein binding probes. Notice that there are nine G,C pairs in sequence A and there are only two G,C pairs in sequence B.

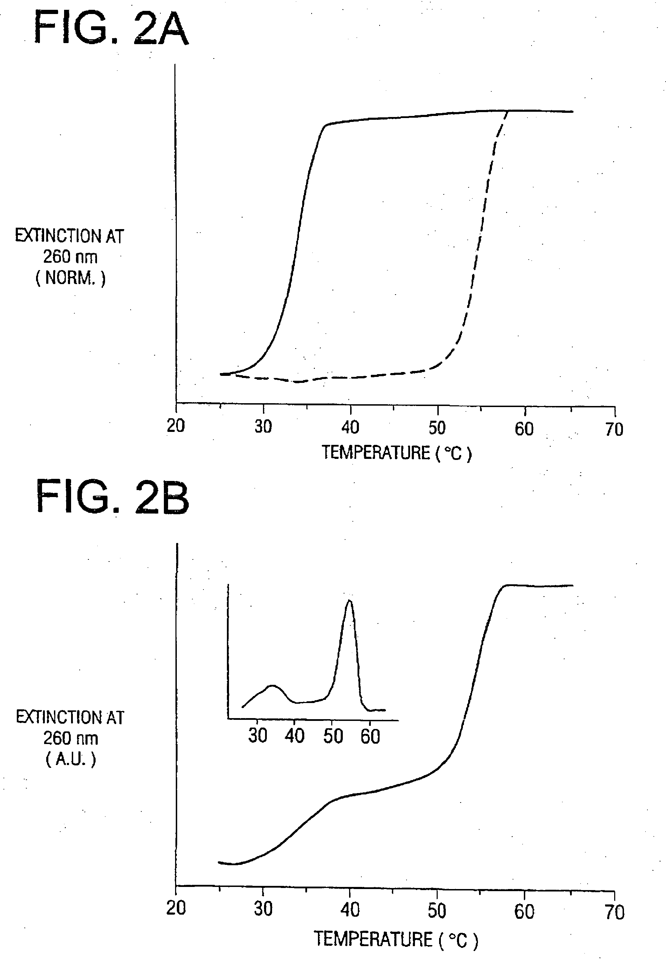

Figure 2 illustrates thermal denaturation profiles for Au nanoparticle aggregates linked by DNA and proteins. Extinction at 260 nm was monitored as a function of increasing temperature (1 °C/min, 1 min holding time). Each UV-Vis spectrum was measured under constant stirring to suspend the aggregates. All the aggregates were suspended in 1 ml of 0.3 M PBS prior to performing the melting analyses. A) Two probes with one target antibody present IgE (——), IgG1 (---)); all data have been normalized; (B) Two probes with both target antibodies present. Inset; first derivative of the thermal denaturation curve.

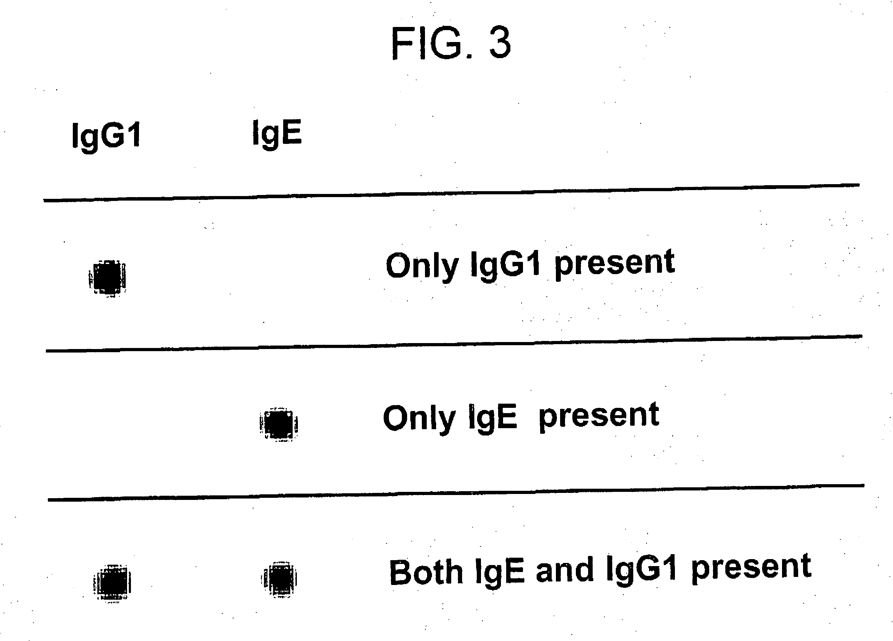

Figure 3 illustrates an array-based protein detection scheme using DNA as a biobarcode for the protein.

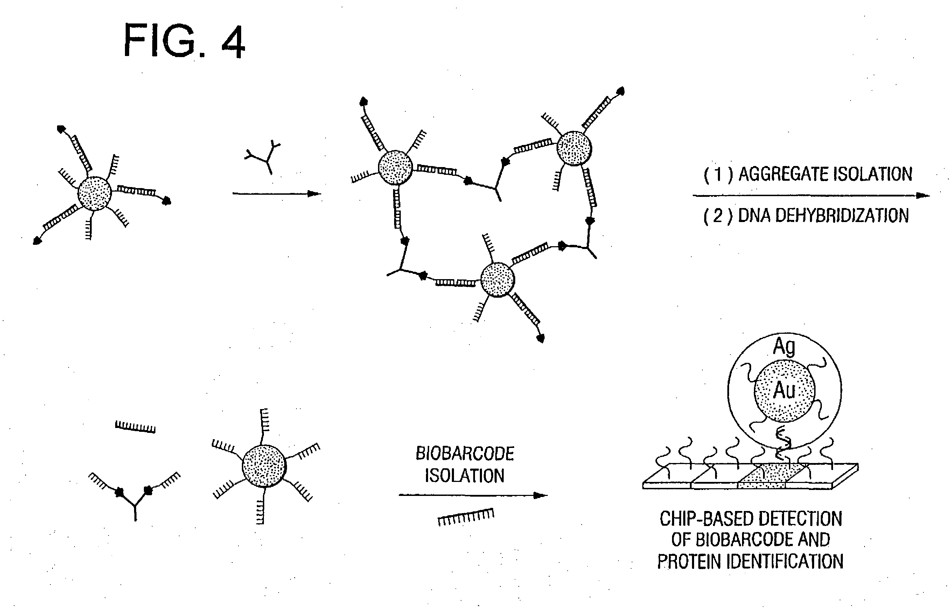

Figure 4 illustrates scanometric DNA array detection of the DNA biobarcodes. Left column is for the detection of the biobarcode associated with IgG1 and the right column is for the biobarcode associated with IgE. The capture oligonucleotides are 5' -thiol-modified ataactagaacttga (SEQ ID NO:1) for the IgG1 system. and 5' -thiol-modified ttatctattatt (SEQ ID NO:2) for the IgE system. Each spot is approximately 250 um in diameter and read via gray-scale with an Epson Expression 1640XL flatbed scanner (Epson America, Longbeach, California). These assays have been studied and work comparably well over the 20 nM to 700 nM target concentration range.
Figure 5 illustrates a common type of immuno-PCR-based analyte detection scheme.

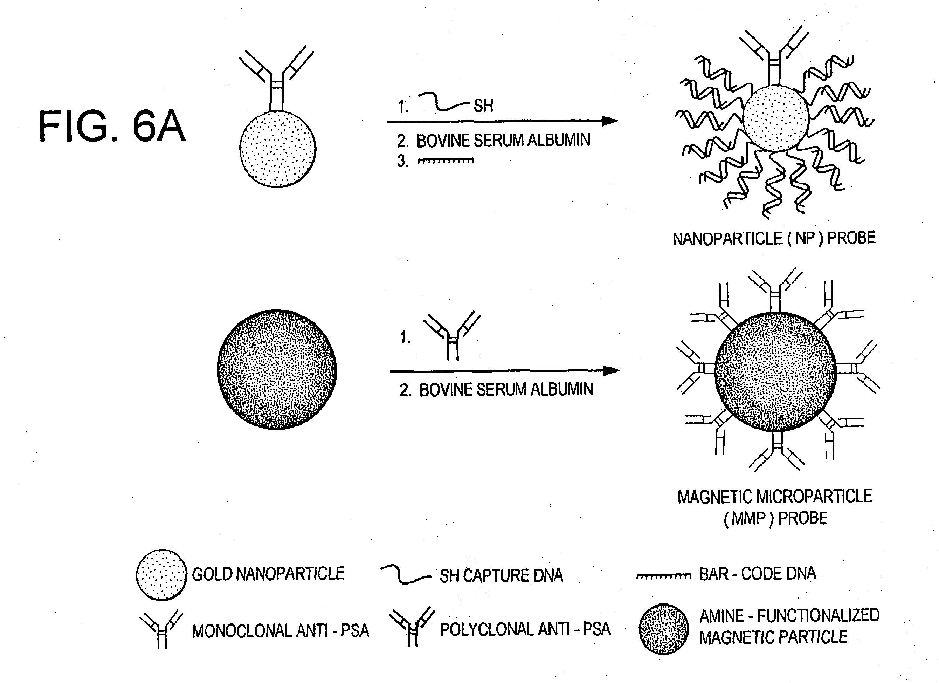

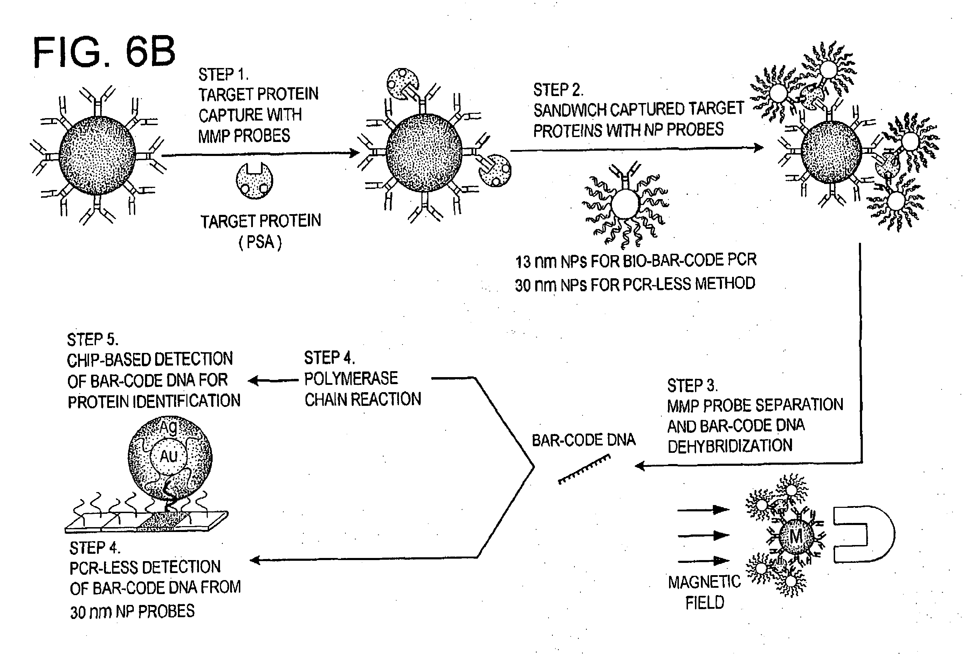

Figure 6 depicts the use of Barcode PCR (*B*PCR) protocol to detect a target analyte, prostate specific antigen (PSA). Panel A illustrates probe design and preparation. Panel B depicts PSA detection and barcode DNA amplification and identification.

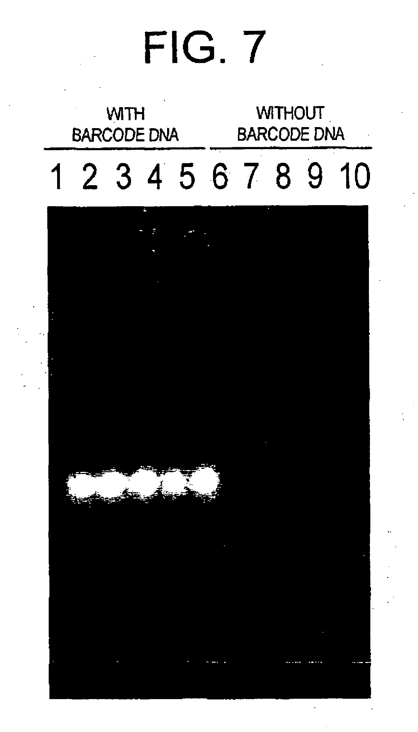

Figure 7 illustrates the control experiment to assess primer-dimer formation, DNA barcode amplification, and the effect of increasing DMSO concentration using 25 thermal cycles. Lanes 1 through 5 are those with DNA barcode present in the PCR reaction mixture while there is no DNA barcode in lanes 6 through 10. Note that DMSO is increased from lane 1 to 5 and 6 to 10 (0 to 2 % in 0.5 % increments).

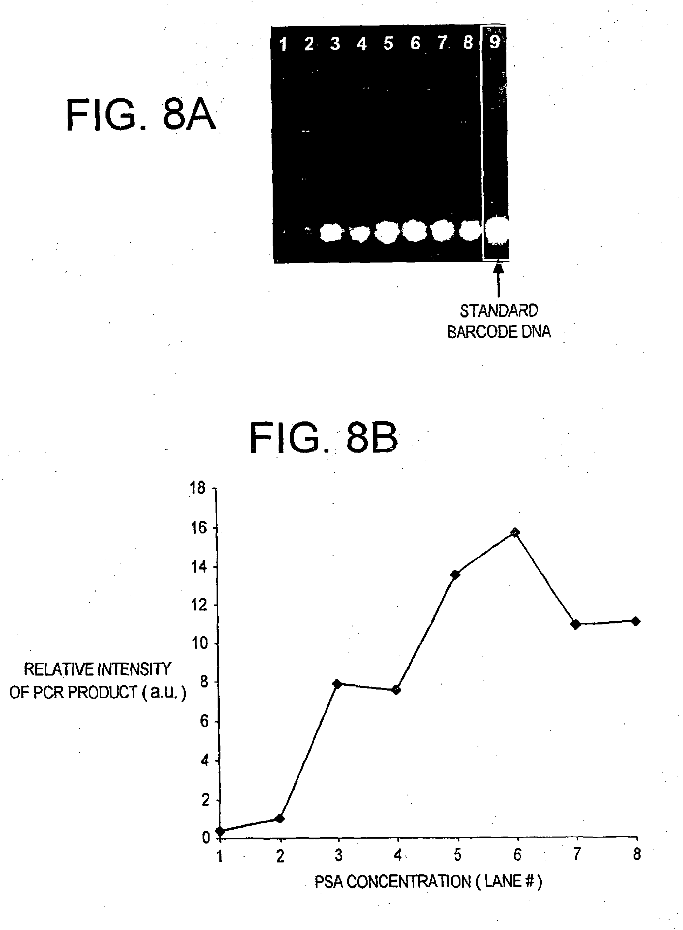

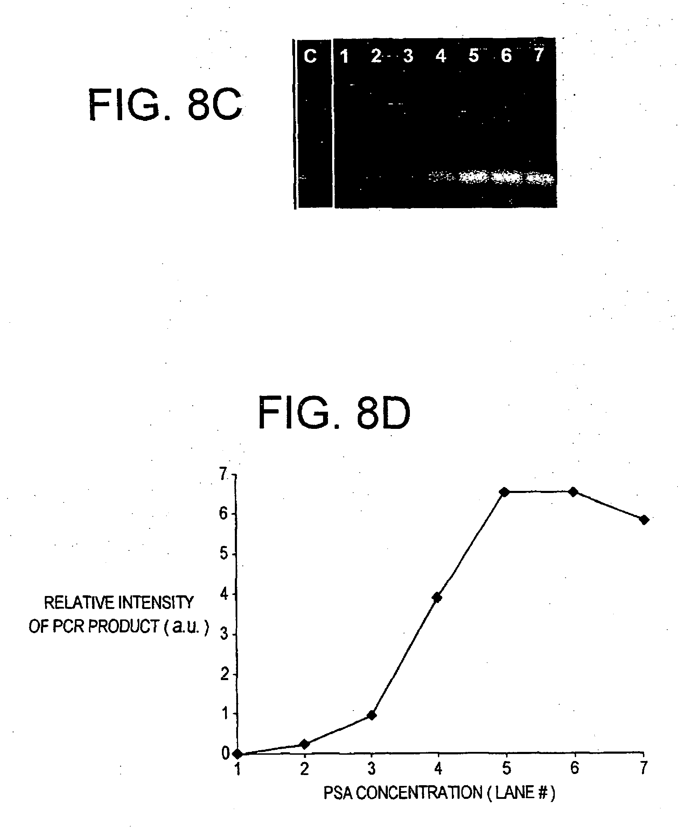

Figure 8 shows gel electrophoresis images and relative band intensity graph of barcode DNA amplified by PCR after PSA detection. Panel A, lanes 1 and 2 are control experiments (lane 1: with background proteins anti-dinitrophenyl and β-galactosidase without PSA, lane 2: with no protein). From lanes 3 to 8, PSA concentrations in the sample (10µl) are 300 aM, 3 fM, 30 fM, 300 fM, 3 pM, and 30 pM, respectively. The standard biobarcode DNA 40-mer for PSA is run in lane 9 to compare with other gel bands after PCR. Panel B, relative gel electrophoresis band intensity graph after *B*PCR. Panel C, low concentration detection of PSA. Concentrations are from 3 aM to 300 fM in 10x dilutions from lane 2 (3 aM) to lane 7 (300 fM). A negative control with only background proteins is shown in lane 1, and the standard biobarcode 40-mer (6 µM, biobarcode duplex) is shown in the fist lane (lane C). Panel D, relative gel electrophoresis band intensity after *B*PCR.

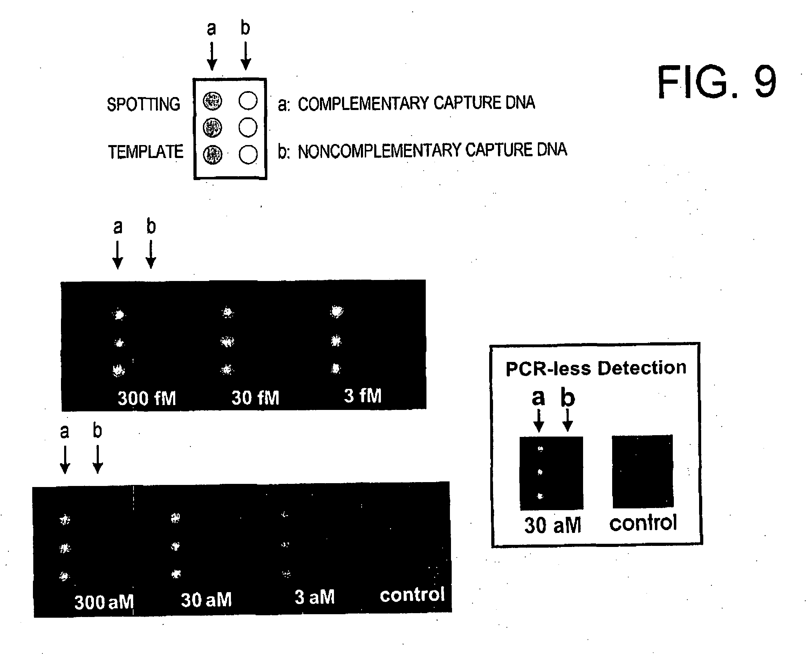

Figure 9 illustrates scanometric detection of PSA-specific barcode DNA. PSA concentration (sample volume of 10 ml) was varied from 300 fM to 3 aM and a negative control sample where no PSA was added (control) is shown. For all seven samples, 2 ml of anti-dinitrophenyl (10 pM) and 2 ml of β-galactosidase (10 pM) were added as background proteins. Also shown is PCR-less detection of PSA (30 aM and control) with 30 nm NP probes (inset). Chips were imaged with the Verigene ID system.

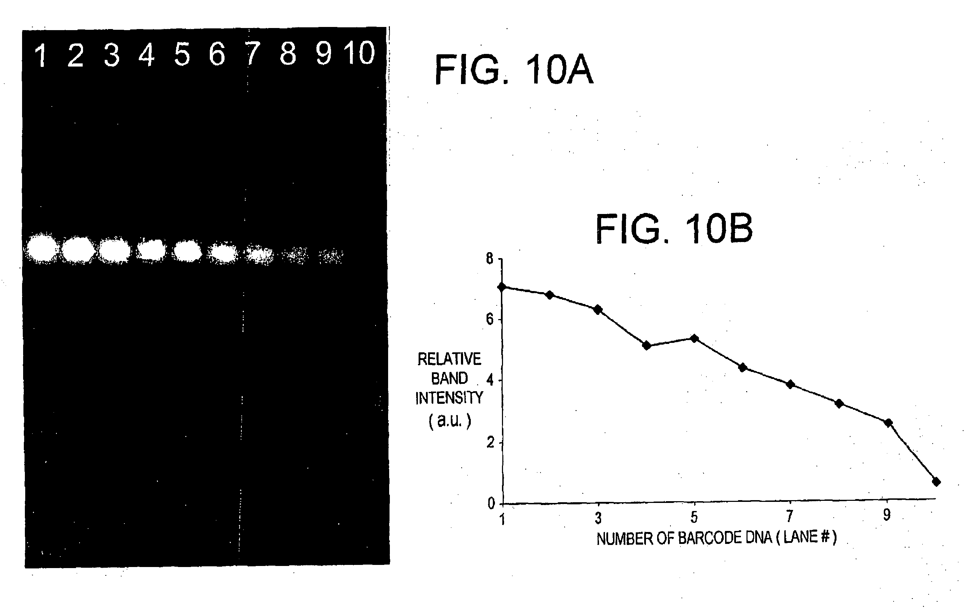

Figure 10 illustrates theoretical detection limit of *B*PCR. Left panel, The gel image shows bands after PCR at decreasing starting barcode DNA concentrations. Lane 1: 3 x 10⁹ copies, lane 2: 3 x 10⁸ copies, lane 3: 3 x 10⁷ copies, lane 4: 3 x 10⁶ copies, lane 5: 3 x 10⁵ copies, lane 6: 3 x 10⁴ copies, lane 7: 3 x 10³ copies, lane 8: 3 x 10² copies, lane 9: 3 x 10¹ copies, and lane 10: no barcode DNA. Right panel, relative gel electrophoresis band intensity graph.

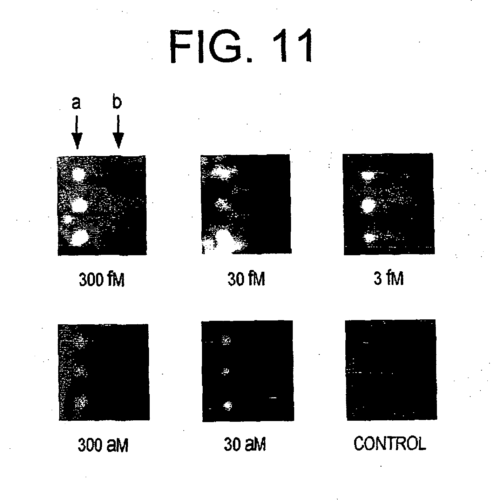

Figure 11 illustrates detection of PSA-specific barcode DNA where the PSA is dissolved in a complex goat serum medium. Each panel shows the signal generated by the BPCR amplified barcode DNA at various concentrations of analyte (3 pM to 3 aM).

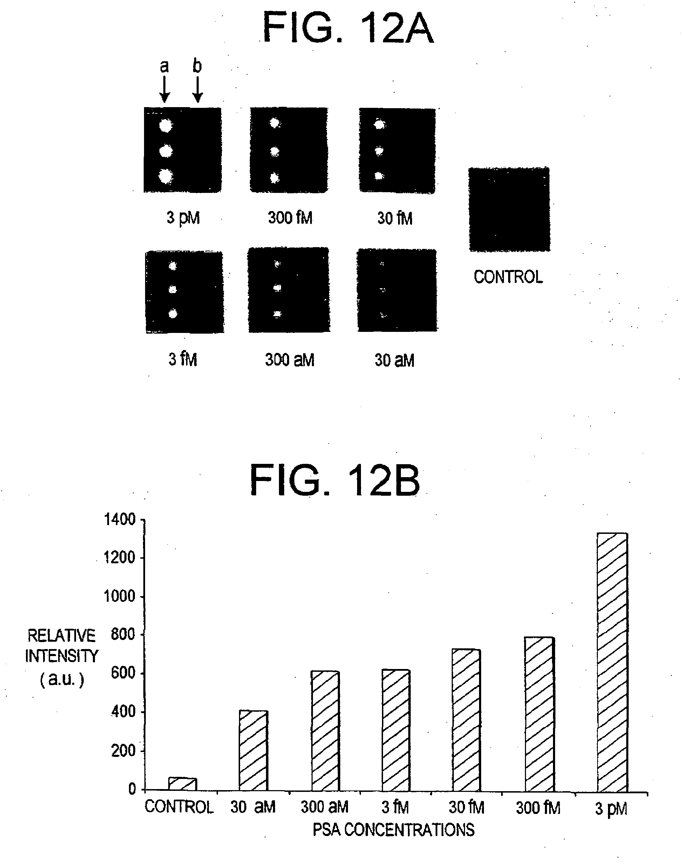

Figure 12 illustrates PCR-less detection of PSA with 30 nm NP probes. Each panel and associated relative intensity value on the bar graph shows the signal generated by direct detection of barcode DNA (*i.e*., non-*B*PCR amplified) at various concentrations (30 aM to 3 pM, and control). Chips were imaged with the Verigene ID system (Nanosphere, Inc., Northbrook, IL).

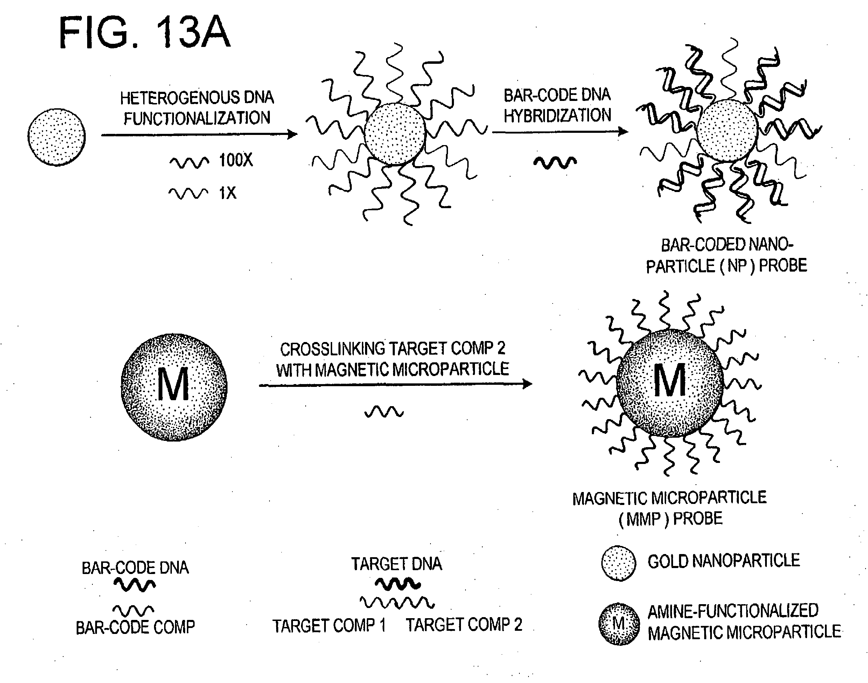

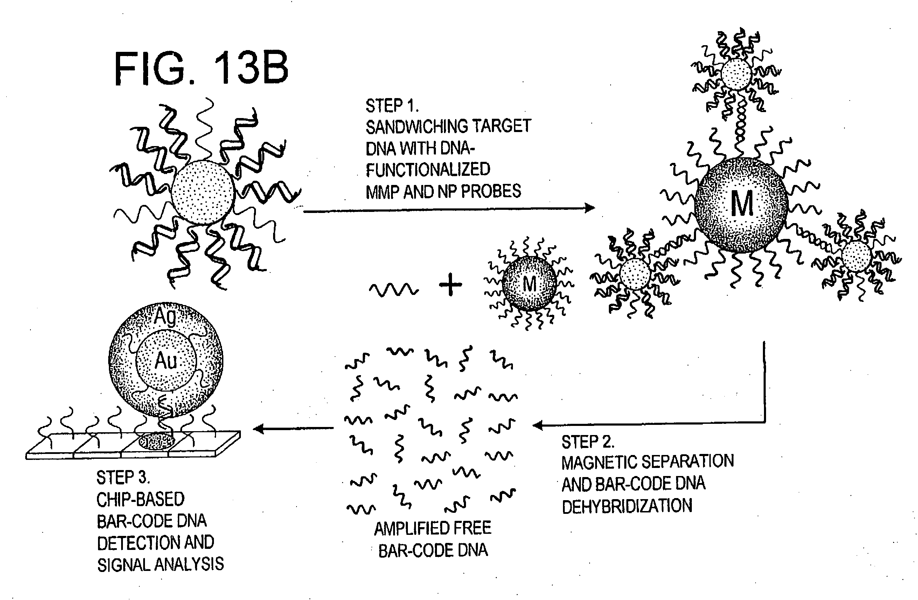

Figure 13 illustrates the DNA-BCA assay. A. Nanoparticle and Magnetic Microparticle Probe Preparation. B. Nanoparticle-Based PCR-less DNA Amplification Scheme.

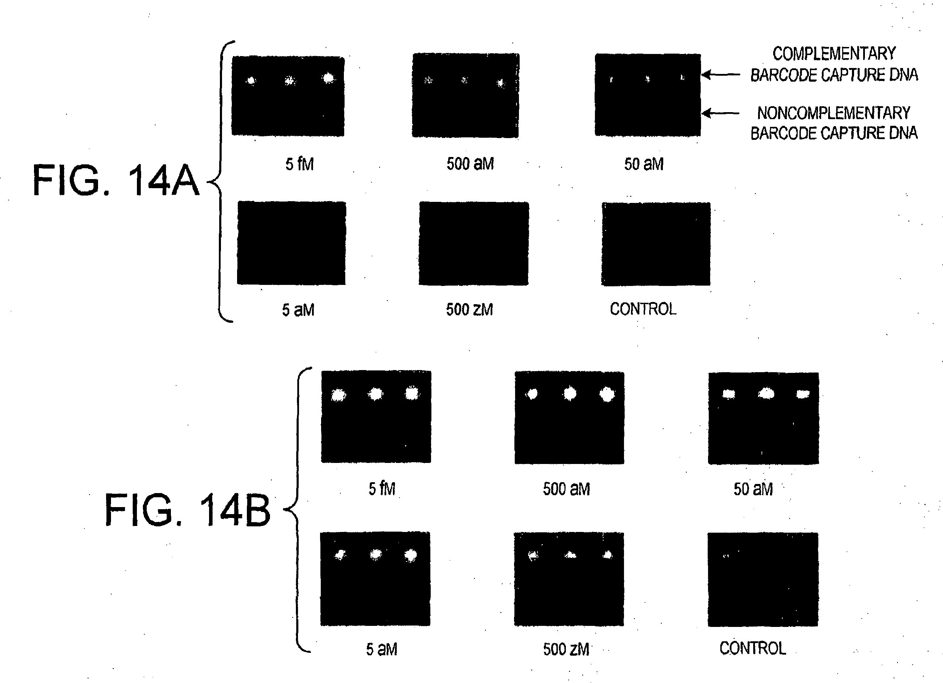

Figure 14 illustrates amplified Anthrax Bar-Code DNA Detection with Verigene ID system. A. Anthrax Bar-Code DNA Detection with 20 nm NP Probes. B. Anthrax Bar-Code DNA Detection with 30 nm NP Probes.

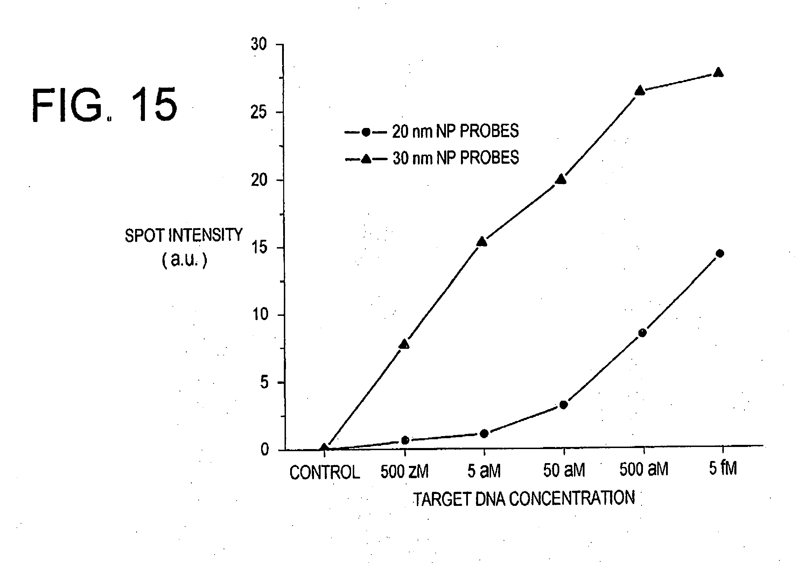

Figure 15 illustrates intensity graph of the bar-code DNA and NP probe sandwiched spots after silver enhancement for 20 nm and 30 nm NP probes.

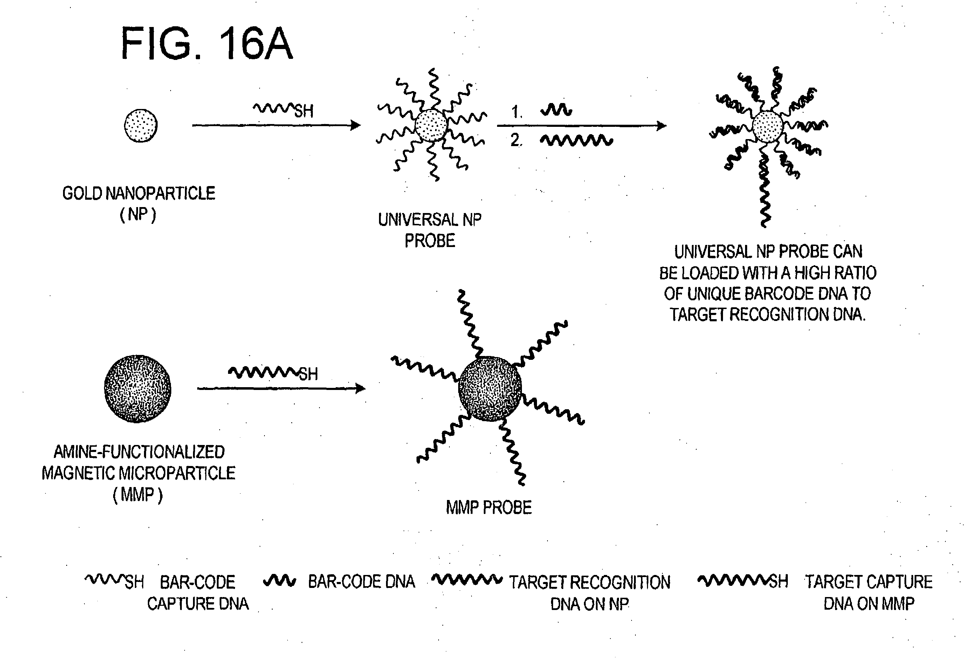

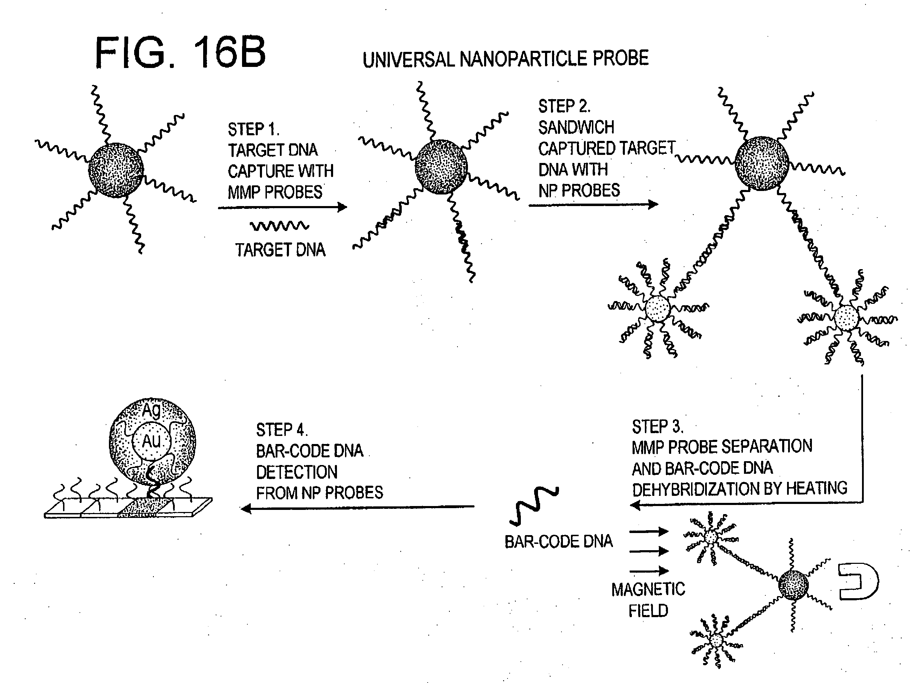

Figure 16 illustrates one embodiment of the "universal" nanoparticle probe detection scheme. A. The universal probes are synthesized for specificity to one or more target nucleic acid sequences. The target recognition DNA can be used to control specificity of the probe for a target. The probes can be used in assay systems where single or multiple target nucleic acid sequences are present in a given test solution. B. The universal probes are used in conjunction with a second type of recognition oligonucleotide bound to a substrate, such as a magnetic microparticle or glass slide. The second type of recognition oligonucleotide, the universal probe, and the test solution thought to contain the target nucleic acid are mixed and reacted under conditions that allow for hybridization and complex formation. The complex is separated from the unreacted universal probes and test solution components, and the reporter oligonucleotides are detected.

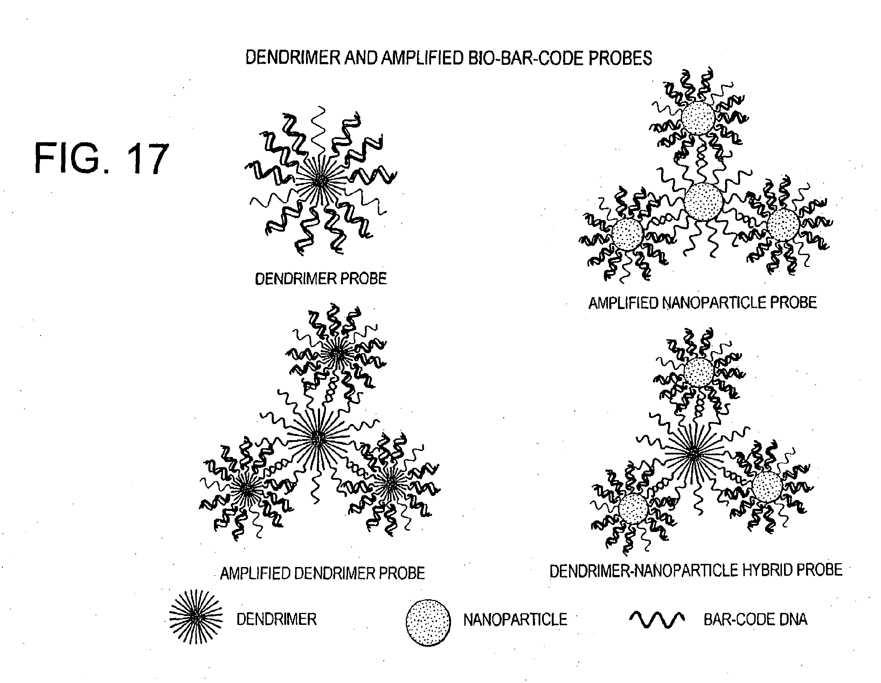

Figure 17 illustrates another embodiment of the universal nanoparticle probe with dendrimer probes, amplified dendrimer probes, and dendrimer-nanoparticle hybrid probes. The first type of dendrimer probes comprise a recognition oligonucleotide sequence and a nucleic acid sequence that is complementary to a reporter oligonucleotide, such as a barcode DNA. The recognition oligonucleotide sequence on the first type of dendrimer probe can hybridize to a second type of dendrimer probe. Under hybridization conditions, this generates a dendrimer probe complex (or matrix) that can be extended as desired. The second type of dendrimer probe can bind a plurality of dendrimer probes and functions to amplify the amount of reporter oligonucleotide contained within the entire matrix. Similarly, the amount of recognition oligonucleotide present on the first type of dendrimer can be increased or decreased, in order to provide more sites of complexation with the second type of dendrimer, or in order to provide more reporter oligonucleotide. The first or second type of dendrimer probes can be used with other particle probes, such as gold nanoparticle probes, or magnetic particle probes in order to generate a hybrid probe complex (or matrix) system, as required by the particular assay.

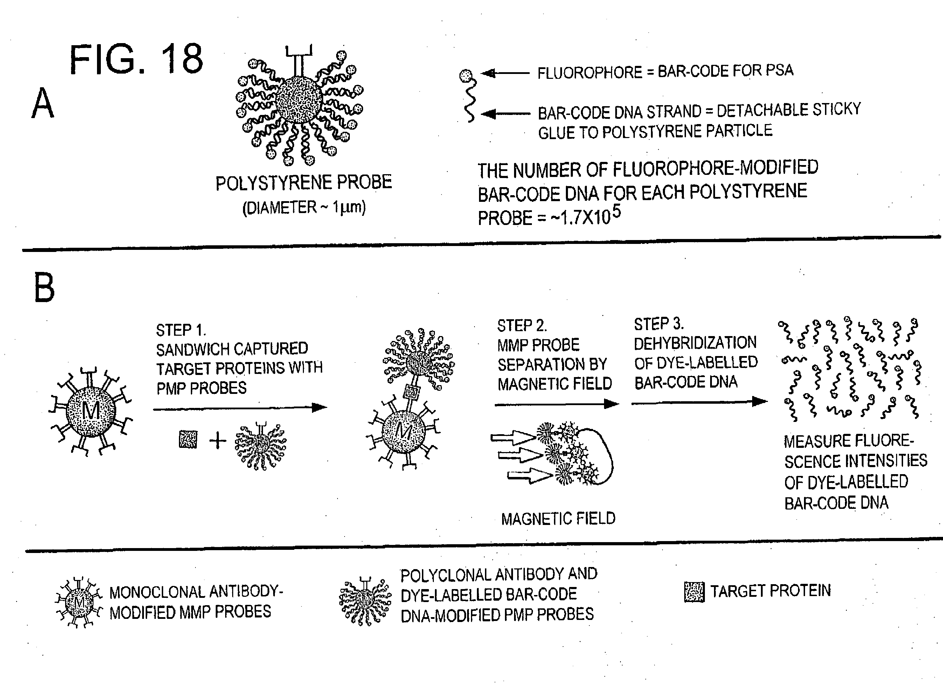

Figure 18 illustrates a fluorophore-based bio-barcode amplification assay. Barcode DNA for each polystyrene probe was about 1.1x10⁵ and total assay time was about 50 minutes.

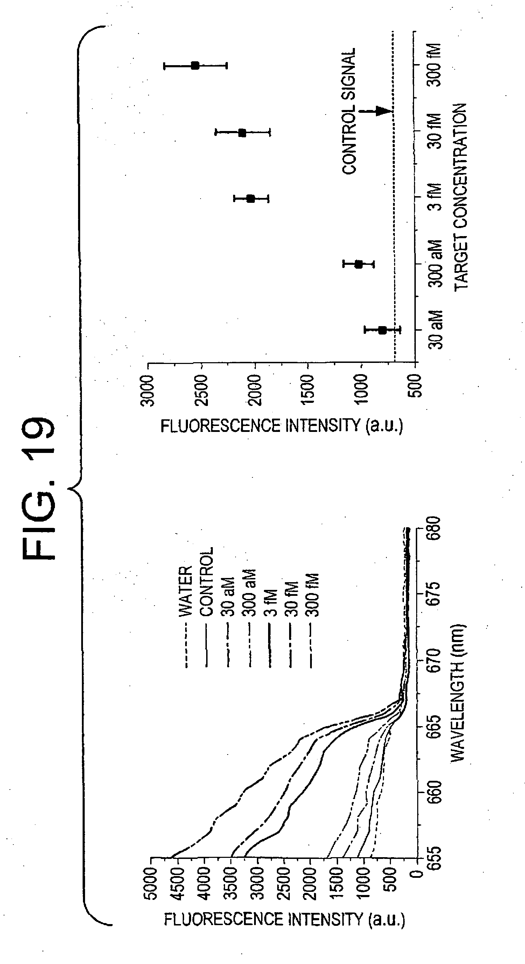

Figure 19 shows fluorescence signal (Alexa-647) for prostate specific antigen (PSA) (10 µL sample). Sensitivity was about 30 aM PSA. The micro-fluorometer cell (50 µL capacity) was used with Alexa-647.

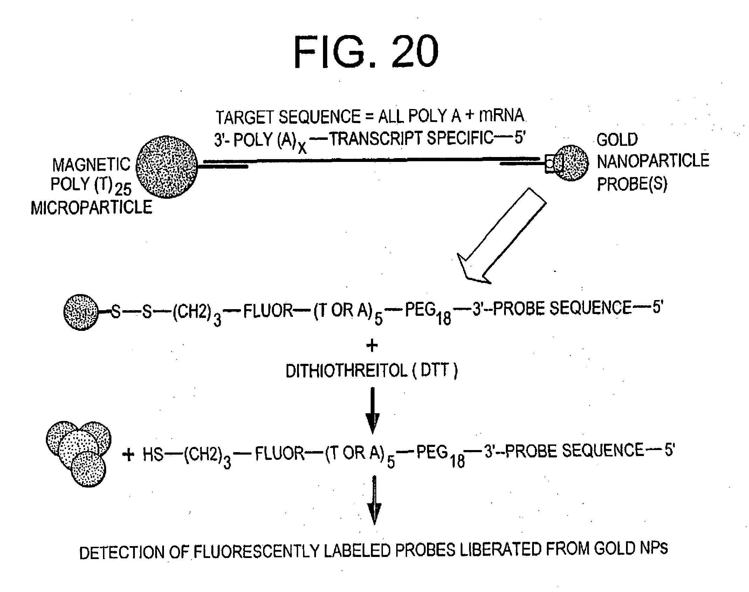

Figure 20 shows a schematic representation of an exemplary assay of the present invention wherein the probe strands on the surface of the gold nanoparticle probes also represent the reporter moieties. Thiolated oligonucleotides doubling as probes for mRNA specific target sequences and reporter moieties were covalently immobilized to the surface of the gold nanoparticle. As shown, the sphere next to the gold nanoparticle surface represents either a fluorophore for fluorescent readout or a "universal" DNA sequence that can be used for scanometric gold nanoparticle labeling.

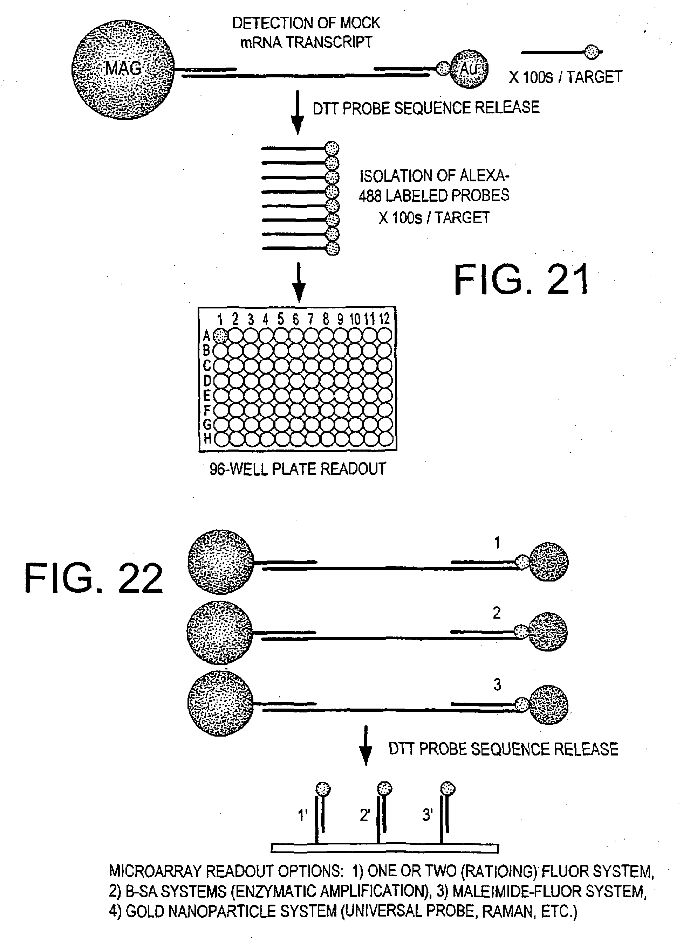

Figure 21 shows a schematic representation of the detection of mock mRNA target. Probes are as in Figure 20. Fluorescent readout was used to detect DTT-liberated fluorescently-labeled probes specific to the detected mRNA target.
Figure 22 shows a schematic representation of multiplexed detection of numerous nucleic acid targets where they are sorted out on a high density microarray which is subsequently readout using any standard method. Importantly, the sphere can represent a fluorescent readout method or can represent a universal capture sequence that can be probed using a "universal" gold nanoparticle probe in the Scanometric technique.

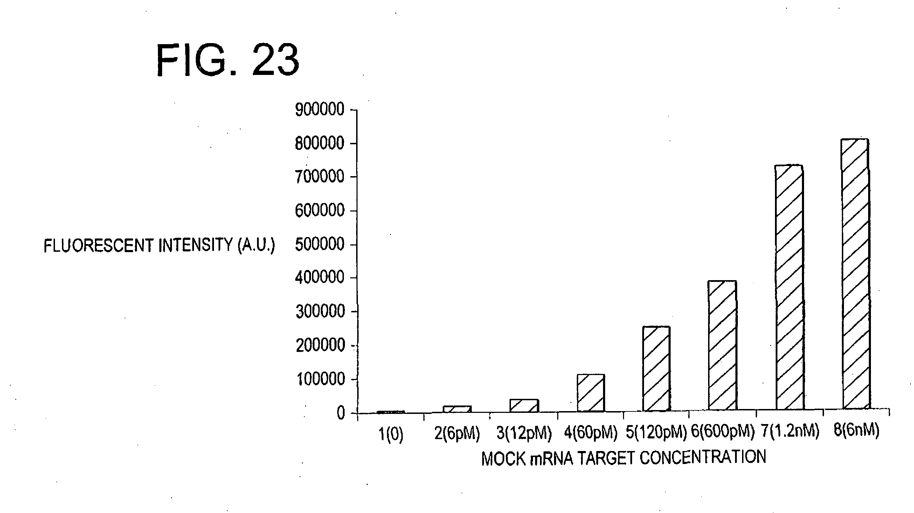

Figure 23 shows fluorimetric detection of fluorescently-labeled probe strands liberated after the detection of a mock mRNA sequence as shown in Figure 20. The 6 pM fluorescent 4 detection limit is as low as is possible using 13 nm probes liberating approximately 100 fluorescently-tagged reporter moieties per detection event in light of the fluorescent detection limit of approximately 1 nM for Alexa 488.

### DETAILED DESCRIPTION OF THE INVENTION

As used herein, a "type of" nanoparticles, conjugates, particles, latex microspheres, etc. having oligonucleotides attached thereto refers to a plurality of that item having the same type(s) of oligonucleotides attached to them. "Nanoparticles having oligonucleotides attached thereto" or "Nanoparticles having oligonucleotides attached thereto" are also sometimes referred to as "nanoparticle-oligonucleotide conjugates" or, in the case of the detection methods of the invention, "nanoparticle-oligonucleotide probes," "nanoparticle probes," or just "probes."

As used herein, the term "particle" refers to a small piece of matter that can preferably be composed of metals, silica, silicon-oxide, or polystyrene. A "particle" can be any shape, such as spherical or rod-shaped. The term "particle" as used herein specifically encompasses both nanoparticles and microparticles as defined and described hereinbelow.

As used throughout the invention "barcode", "biochemical barcode", "biobarcode", "barcode DNA", "DNA barcode", "reporter barcode", "reporter barcode DNA", etc. are all interchangeable with each other and have the same meaning. The DNA barcode may be a nucleic acid such as deoxynucleic acid or ribonucleic acid. Preferably, the DNA barcode is an oligonucleotide of a predefined sequence. If desired, the DNA barcode may be labeled, for instance, with biotin, a radiolabel, or a fluorescent label.

The term "nanoparticle complex" or "nanoparticle complex probe" refers to a conjugate comprised of nanoparticle-oligonucleotide conjugates, a reporter oligonucleotide, and an oligonucleotide having bound thereto a specific binding complement to a target analyte.

The term "analyte" or "target analyte" refers to the compound or composition to be detected, including drugs, metabolites, pesticides, pollutants, and the like. The analyte can be comprised of a member of a specific binding pair (sbp) and may be a ligand, which is monovalent (monoepitopic) or polyvalent (polyepitopic), preferably antigenic or haptenic, and is a single compound or plurality of compounds, which share at least one common epitopic or determinant site. The analyte can be a part of a cell such as bacteria or a cell bearing a blood group antigen such as A, B, D, etc., or an HLA antigen or a microorganism, e.g., bacterium, fungus, protozoan, or virus. If the analyte is monocpitopic, the analyte can be further modified, e.g. chemically, to provide one or more additional binding sites. In practicing this invention, the analyte has at least two binding sites.

The polyvalent ligand analytes will normally be larger organic compounds, often of polymeric nature, such as polypeptides and proteins, polysaccharides, nucleic acids, and combinations thereof. Such combinations include components of bacteria, viruses, chromosomes, genes, mitochondria, nuclei, cell membranes and the like.

For the most part, the polyepitopic ligand analytes to which the subject invention can be applied will have a molecular weight of at least about 5,000, more usually at least about 10,000. In the polymeric molelccule category, the polymers of interest will generally be from about 5,000 to 5,000,000 molecular weight, more usually from about 20,000 to 1,000,000 molecular weight; among the hormones of interest, the molecular weights will usually range from about 5,000 to 60,000 molecular weight.

A wide variety of proteins may be considered as belonging to the family of proteins having similar structural features, proteins having particular biological functions, proteins related to specific microorganisms, particularly disease causing microorganisms, etc. Such proteins include, for example, immunoglobulins, cytokines, enzymes, hormones, cancer antigens, nutritional markers, tissue specific antigens, etc.

The types of proteins, blood clotting factors, protein hormones, antigenic polysaccharides, microorganisms and other pathogens of interest in the present invention are specifically disclosed in U,S. Pat. No. 4,650,770.

The monoepitopic ligand analytes will generally be from about 100 to 2,000 molecular weight, more usually from 125 to 1,000 molecular weight.

The analyte may be a molecule found directly in a sample such as a body fluid from a host. The sample can be examined directly or may be pretreated to render the analyte more readily detectible. Furthermore, the analyte of interest may be determined by detecting an agent probative of the analyte of interest such as a specific binding pair member complementary to the analyte of interest, whose presence will be detected only when the analyte of interest is present in a sample. Thus, the agent probative of the analyte becomes the analyte that is detected in an assay. The body fluid can be, for example, urine, blood, plasma, serum, saliva, semen, stool, sputum, cerebral spinal fluid, tears, mucus, and the like.

The term "specific binding pair (sbp) member" refers to one of two different molecules, which specifically binds to and can be defined as complementary with a particular spatial and/or polar organization of the other molecule. The members of the specific binding pair can be referred to as ligand and receptor (antiligand). These will usually be members of an immunological pair such as antigen-antibody, although other specific binding pairs such as biotin-avidin, enzyme-substrate, enzyme-antagonist, enzyme-agonist, drug-target molecule, hormones-hormone receptors, nucleic acid duplexes, IgG-protein A/protein G, polynucleotide pairs such as DNA-DNA, DNA-RNA, protein-DNA, lipid-DNA, lipid-protein, polysaccharide-lipid, protein-polysaccharide, nucleic acid aptamers and associated target ligands (*e.g*., small organic compounds, nucleic acids, proteins, peptides, viruses, cells, etc.), and the like are not immunological pairs but are included in the invention and the definition of sbp member. A member of a specific binding pair can be the entire molecule, or only a portion of the molecule so long as the member specifically binds to the binding site on the target analyte to form a specific binding pair.

The term "ligand" refers to any organic compound for which a receptor naturally exists or can be prepared. The term ligand also includes ligand analogs, which are modified ligands, usually an organic radical or analyte analog, usually of a molecular weight greater than 100, which can compete with the analogous ligand for a receptor, the modification providing means to join the ligand analog to another molecule. The ligand analog will usually differ from the ligand by more than replacement of a hydrogen with a bond, which links the ligand analog to a hub or label, but need not. The ligand analog can bind to the receptor in a manner similar to the ligand. The analog could be, for example, an antibody directed against the idiotype of an antibody to the ligand.

The term "receptor" or "antiligand" refers to any compound or composition capable of recognizing a particular spatial and polar organization of a molecule, e.g., epitopic or determinant site. Illustrative receptors include naturally occurring receptors, e.g., thyroxine binding globulin, antibodies, enzymes, Fab fragments, lectins, nucleic acids, nucleic acid aptamers, avidin, protein A, barstar, complement component Clq, and the like. Avidin is intended to include egg white avidin and biotin binding proteins from other sources, such as streptavidin.

The term "specific binding" refers to the specific recognition of one of two different molecules for the other compared to substantially less recognition of other molecules. Generally, the molecules have areas on their surfaces or in cavities giving rise to specific recognition between the two molecules. Exemplary of specific binding are antibody-antigen interactions, enzyme-substrate interactions, polynucleotide interactions, and so forth.

The term "non-specific binding" refers to the binding between molecules that is relatively independent of specific surface structures. Non-specific binding may result from several factors including hydrophobic interactions between molecules.

The term "antibody" refers to an immunoglobulin which specifically binds to and is thereby defined as complementary with a particular spatial and polar organization of another molecule. The antibody can be monoclonal or polyclonal and can be prepared by techniques that are well known in the art such as immunization of a host and collection of sera (polyclonal) or by preparing continuous hybrid cell lines and collecting the secreted protein (monoclonal), or by cloning and expressing nucleotide sequences or mutagenized versions thereof coding at least for the amino acid sequences required for specific binding of natural antibodies. Antibodies may include a complete immunoglobulin or fragment thereof, which immunoglobulins include the various classes and isotypes, such as IgA, IgD, IgE, IgG1, IgG2a, IgG2b and IgG3, IgM, etc. Fragments thereof may include Fab, Fv and F(ab')₂, Fab', and the like. In addition, aggregates, polymers, and conjugates of immunoglobulins or their fragments can be used where appropriate so long as binding affinity for a particular molecule is maintained.

In certain embodiments, the invention provides a novel detection reagent containing a reporter particle of a convenient size that has been derivatized to include on its surface some number of selective binding moieties that specifically bind a pre-selected target chemical species and a large number of copies of a pre-selected reporter moiety. The reporter moiety is covalently attached to the surface of the reporter particle using a standard chemistry.

The preferred detection method utilizing this amplification material is similar to that used in a sandwich immunoassay. In particular, the sample being analyzed is exposed to a capture phase capable of selectively and specifically binding to species of interest, the capture phase being immobilized on an insoluble material. Any unbound materials are then separated from the immobilized analyte through standard means. Immobilized analyte is then exposed to the detection reagent of this invention. The detection reagent binds to the immobilized analyte through the selective binding moieties incorporated thereon. The "sandwich" structure thus formed (insoluble substrate - analyte - detection reagent) therefore effectively immobilizes the detection reagent on the insoluble substrate. Unbound detection reagent can be separated from this immobilized structure through standard methods. Amplification can be performed by exposing the immobilized insoluble substrate - analyte - detection reagent sandwich to a means of rupturing the bond between the reporter moiety and the reporter particle, resulting in the release of the reporter moieties into the medium. As the ratio of the numbers of reporter moieties and selective binding moieties initially bound to the reporter particle can be established at greater than one during preparation of the detection reagent, release of the reporter moieties from a particle results in more reporter moieties entering the medium than there are target analyte molecules abound to the insoluble substrate. Detection, and optionally quantitation, of the released reporter moieties can be performed using any method that is appropriate to the chemical nature of the reporter moiety. The significant amplification of the detected signal of the reporter moiety from the detection of individual target analyte molecules results in an extremely sensitive, reliable and adaptable chemical detection assay.

In certain embodiments, a detection reagent of the present invention is composed of a reporter particle of a convenient size that has been derivatized to include on its surface selective binding moieties that specifically bind to the target chemical or target analyte and a plurality of pre-selected reporter moieties. The reporter particle can be any material that is compatible with the sample containing the target analyte and capable of binding both the selective binding moieties and the reporter moieties. Examples of suitable reporter particles include, but are not limited to, metals, silica, silicon-oxide, and polystyrene.

The selective binding compound may be any compound capable of selectively recognizing and binding to the target analyte without interfering with the binding between the target analyte and the capture phase. Example of suitable selective binding compounds include, but are not limited to, antibodies, enzymes, proteins, oligonucleotides and inorganic compounds.

The reporter particles are selected for a functional size typically having a diameter in the nanometer to micrometer range. As the ratio of the numbers of reporter moieties and selective binding moieties initially bound to a reporter particle can be established at greater than one during preparation of the detection reagent of the present invention, release of the reporter moieties from a particle will result in more reporter moieties entering the medium than there are analyte molecules bound to the reporter particle. This ratio establishes the amplification of the signal from the detection of a target analyte molecule. For example, the release of the reporter moieties from one reporter particle bearing 1000 copies of the reporter moiety that is bound to one molecule of immobilized analyte will result in 1000 molecules of reporter moiety appearing in the medium for each molecule of analyte in the original sandwich. This results in the chemical signal represented by the target analyte being amplified by a factor of 1000. This amplification can be adjusted during the synthesis of the detection reagent by manipulating parameters such as the surface area of the reporter particle and the ratio between and the packing densities of the selective binding and reporter moieties on the surface of the reporter particle. Thus, the size of the reporter particle dictates the number of reporter moieties that can be released, and the ultimate amplification factor that is obtained with regard to labeled target molecules. For example, 13 nm gold particles can carry with them up to 200 thiolated surface oligomers serving as reporter moieties thereby achieving an amplification factor of 200 reporter moieties to a single target molecule bound by a single reporter particle in the sandwich formed in the detection assay. Larger size (e.g. micron-sized) particles will obviously lead to larger amplification factors.

The reporter moiety is preferably attached to the surface of the reporter particle by a means sufficiently strong enough to prevent significant non-specific release of the reporter moiety during the steps of the detection method but simultaneously susceptible to separation and release of the reporter moiety immediately prior to the detection step. Preferably, the reporter moiety is attached to the surface of the reporter particle through a covalent bond that can be broken when contacted with a specific reagent added to the solid support immediately prior to the detection step. In the embodiment in which a covalent bond is used to anchor the reporter moiety to the reporter particle, the chemical used to release the reporter moiety from the reporter particle is a molecule that effectively takes the place of the reporter moiety on the surface of the reporter particle by preferentially binding to, or reacting with, the reporter particle while displacing the reporter moiety.

Examples of the reporter moieties may include, but are not limited to, fluorophores, chromophores, oligonucleotides with or without attached fluorophores or chromophores, proteins including enzymes and porphyrins, lipids, carbohydrates, synthetic polymers and tags such as isotopic or radioactive tags. These chemicals can be linked to the reporter particle through a covalent link between sulfur atoms such as thiols, disulfides, phosphorylthiolates and the like. In this instance, contacting the reporter particle with a chemical that displaces the reporter moiety by preferentially binding a sulfur atom in the covalent link liberates the reporter moiety for subsequent detection. Chemicals that would effectively displace the reporter moiety include any molecule that will preferentially bind to the reporter particle through the thiol link such as other thiol- or disulfide-containing molecules, dithiothreitol (DTT), dithioerythritol (DTE), mercaptoethanol and the like. Similarly, reducing agents such as sodium borohydride will cleave a disulfide linkage releasing the reporter moieties.

In a specific embodiment of the present invention, the reporter moiety and the selective binding compound are the same chemical entity. For example, in a preferred embodiment, the reporter moiety and the selective binding compound are described by a single oligonucleotide that is complementary to the target analyte and can additionally be detected after removal from the reporter particle (for example through a fluorescent tag present on the oligonucleotide).

In the first step of a detection method of the present invention, the sample being analyzed for the presence of the target molecule is exposed to a capture phase such as an antibody, oligonucleotide, lectin or similar material that is capable of selectively and specifically binding to the target specie of interest. The capture phase is immobilized on an insoluble material that is compatible with the assay chemistry and that it can readily be separated from the reaction medium. The immobilized capture phase is constructed such that it specifically binds, captures and immobilizes the analyte of interest, but preferably does not bind any other materials that may be present in the sample. Examples of the insoluble material suitable for use in the methods of the present invention include, but are not limited to, wells of a microtiter plate, a microparticle, fibrous or membrane filters, or other such insoluble materials.

The capture phase is preferably selected such that it binds to a different determinant on the analyte than does the selective binding moiety component of the detection reagent. Any unbound materials are then separated from the immobilized target analyte by any suitable means including, for example, decantation, sedimentation, washing, centrifuging or combinations of these processes. The net result of this process is that the analyte of interest is present in a purified and concentrated state on the surface of the insoluble material.

In a subsequent step of a method of the present invention, the immobilized target analyte is exposed to the detection reagent of this invention which contains the reporter particle bound to the reporter moiety and at least one selective binding moiety. The selective binding moiety specifically binds to the target analyte forming a "sandwich" structure including the insoluble substrate bound to the target analyte which is, in turn, bound to the detection reagent. This sandwich structure effectively immobilizes the detection reagent on the insoluble substrate, and any unbound detection reagent can be separated from this immobilized structure by any suitable methods such as decantation, sedimentation, washing, centrifuging or combinations of these processes as noted above.

In another step of a present method the signal from the binding and detection of the target analyte is amplified by exposing the immobilized insoluble substrate - target analyte -detection reagent sandwich to a medium containing a chemical that can liberate the reporter moiety from the reporter particle. The liberated reporter moiety then enters the media surrounding the detection reagent bound to the target analyte as described in detail above.

The media containing the released reporter moiety can be analyzed for the presence of the released reporter moieties using any method that is appropriate to the chemical nature of the reporter moiety. For example, a fluorescently-labeled reporter moiety may be detected and even quantitated by measurement of the fluorescence intensity or fluorescence depolarization of the medium while the presence of a chemiluminescent-labeled reporter can be determined by measuring the luminescence that occurs upon addition of an appropriate trigger reagent. Oligonucleotide-based reporter moieties can further be amplified by the polymerase chain reaction or captured by complimentary oligonucleotides immobilized on an insoluble substrate and detected and quantitated using methods commonly used in conjunction with nucleic acid-based microarrays. Numerous other options including electrochemical, impedance, enzymatic and radioactivity detection are also available.

Therefore, in certain embodiments, the invention provides methods for detecting for the presence of one or more target analytes in a sample, each target analyte having at least two binding sites for specific binding interactions with specific binding complements, in a sample comprising the steps of:
a) providing at least one type of capture substrates, the capture substrates having bound thereto at least one specific binding complement of the specific target analyte that binds to at least a first binding site of the specific target analyte;
b) providing at least one type of microparticle detection probes, the microparticle detection probes comprising a microparticle having bound thereto (i) at least one specific binding complement to a specific target analyte and (ii) a plurality of DNA barcodes, the DNA barcodes are optionally labeled with a reporter label, wherein the specific binding complement bound to the microparticle detection probes binds to at least a second binding site of the target analyte;
c) contacting the capture substrates with a sample believed to contain target analytes under conditions effective to allow for binding of the specific target analyte to the specific binding complement bound to the capture substrate so as to immobilize the target analytes onto the capture substrates;
d) contacting the immobilized target analytes with at least one type of microparticle detection probes under conditions effective to allow for binding between the target analytes and the specific binding complement bound to the microparticle detection probes to the analyte and to form a complex in the presence of the target analyte on the capture substrate;
e) optionally isolating and washing the capture substrate to remove unbound microparticle detection probes; and
f) detecting for the presence of the DNA barcode wherein the presence of the DNA barcode is indicative of the presence of a specific target analyte in the sample.

In other embodiments, the invention provides methods for detecting for the presence of one or more target analytes in a sample, each target analyte having at least two binding sites for specific binding interactions with specific binding complements, in a sample comprising the steps of:
a) providing at least one type of magnetic substrates, the magnetic substrates having bound thereto at least one specific binding complement of the specific target analyte that binds to at least a first binding site of the specific target analyte;
b) providing at least one type of microparticle detection probes, the microparticle detection probes comprising a microparticle having bound thereto (i) at least one specific binding complement to a specific target analyte and (ii) a plurality of DNA barcodes, the DNA barcodes are fluorescently labeled, wherein the specific binding complement bound to the microparticle detection probes binds to at least a second binding site of the target analyte;
c) contacting the magnetic substrates with a sample believed to contain target analytes under conditions effective to allow for binding of the specific target analyte to the specific binding complement bound to the magnetic substrate so as to immobilize the target analytes onto the magnetic substrates;
d) contacting the immobilized target analytes with at least one type of microparticle detection probes under conditions effective to allow for binding between the target analytes and the specific binding complement bound to the microparticle detection probes to the analyte and to form a complex in the presence of the target analyte on the magnetic substrate;
e) optionally isolating and washing the magnetic substrate to remove unbound microparticle detection probes; and
f) detecting for the presence of a fluorescent signal from the DNA barcodes wherein the presence of a fluorescent signal is indicative of the presence of a specific target analyte in the sample.

In additional embodiments, the invention provides methods for detecting for the presence of one or more target analytes in a sample, each target analyte having at least two binding sites for specific binding interactions with specific binding complements, in a sample comprising the steps of:
a) providing at least one type of magnetic substrates, the magnetic substrates having bound thereto at least one specific binding complement of the specific target analyte that binds to at least a first binding site of the specific target analyte;
b) providing at least one type of microparticle detection probes, the microparticle detection probes comprising a microparticle having bound thereto (i) at least one specific binding complement to a specific target analyte and (ii) a plurality of DNA barcodes, the DNA barcodes are fluorescently labeled, wherein the specific binding complement bound to the microparticle detection probes binds to at least a second binding site of the target analyte;
c) contacting the magnetic substrates with a sample believed to contain target analytes under conditions effective to allow for binding of the specific target analyte to the specific binding complement bound to the magnetic substrate so as to immobilize the target analytes onto the magnetic substrates;
d) contacting the immobilized target analytes with at least one type of microparticle detection probes under conditions effective to allow for binding between the target analytes and the specific binding complement bound to the microparticle detection probes to the analyte and to form a complex in the presence of the target analyte on the magnetic substrate;
e) optionally isolating and washing the magnetic substrate to remove unbound microparticle detection probes;
f) subjecting the isolated washed magnetic substrate to conditions effective to release the DNA barcodes; and
g) detecting for the presence of the fluorescent signal of the DNA barcodes wherein the presence of a fluorescent signal of the DNA barcode is indicative of the presence of a specific target analyte in the sample.

In still other embodiments, the invention provides methods for detecting for the presence of one or more target analytes in a sample, each target analyte having at least two binding sites for specific binding interactions with specific binding complements, in a sample comprising the steps of:
a) providing at least one type of magnetic substrates, the magnetic substrates having bound thereto at least one specific binding complement of the specific target analyte that binds to at least a first binding site of the specific target analyte;
b) providing at least one type of microparticle detection probes, the microparticle detection probes comprising a microparticle having bound thereto (i) at least one specific binding complement to a specific target analyte and (ii) a plurality of DNA barcodes, the DNA barcodes comprising a reporter label, wherein the specific binding complement bound to the microparticle detection probes binds to at least a second binding site of the target analyte;
c) contacting the magnetic substrates with a sample believed to contain target analytes under conditions effective to allow for binding of the specific target analyte to the specific binding complement bound to the magnetic substrate so as to immobilize the target analytes onto the magnetic substrates;
d) contacting the immobilized target analytes with at least one type of microparticle detection probes under conditions effective to allow for binding between the target analytes and the specific binding complement bound to the microparticle detection probes to the analyte and to form a complex in the presence of the target analyte on the magnetic substrate;
e) optionally isolating and washing the magnetic substrate to remove unbound microparticle detection probes;
f) subjecting the isolated washed magnetic substrate to conditions effective to release the DNA barcodes; and
g) detecting for the presence of the reporter label of the DNA barcodes wherein the presence of the reporter label of the DNA barcode is indicative of the presence of a specific target analyte in the sample.

In further embodiments, the invention provides methods for detecting for the presence of one or more target analytes in a sample, each target analyte having at least two binding sites for specific binding interactions with specific binding complements, in a sample comprising the steps of:
a) providing at least one type of capture substrates, the capture substrates having bound thereto at least one specific binding complement of the specific target analyte that binds to at least a first binding site of the specific target analyte;
b) providing at least one type of particle detection probes, the particle detection probes comprising a microparticle or nanoparticle having bound thereto (i) at least one specific binding complement to a specific target analyte and (ii) a plurality of DNA barcodes, the DNA barcodes are optionally labeled with a reporter label, wherein the specific binding complement bound to the particle detection probes binds to at least a second binding site of the target analyte;
c) contacting the capture substrates with a sample believed to contain target analytes under conditions effective to allow for binding of the specific target analyte to the specific binding complement bound to the capture substrate so as to immobilize the target analytes onto the capture substrates;
d) contacting the immobilized target analytes with at least one type of particle detection probes under conditions effective to allow for binding between the target analytes and the specific binding complement bound to the particle detection probes to the analyte and to form a complex in the presence of the target analyte on the capture substrate;
e) optionally isolating and washing the capture substrate to remove unbound particle detection probes;
f) releasing the DNA barcodes from the particle detection probes by a chemical releasing agent; and
g) detecting for the presence of the DNA barcode wherein the presence of the DNA barcode is indicative of the presence of a specific target analyte in the sample.

In certain embodiments, the methods of the invention can further comprise the step of washing the capture substrate after the target analytes are immobilized thereon and prior to contacting the capture substrate with particle detection probes.

A target analyte can be a protein or hapten and its specific binding complement can be an antibody comprising a monoclonal or polyclonal antibody. A target analyte can also be a nucleic acid molecule.

A microparticle can be labeled with a plurality of DNA barcodes.

In other embodiments, the methods of the invention can comprise prior to said detecting step and subsequent to said isolating and washing step, the steps of: subjecting the complex to conditions effective to release the DNA barcodes from the microparticle detection probes; and optionally amplifying the DNA barcode prior to said detecting.

Released DNA barcodes can be immobilized on a substrate prior to said detecting.

DNA barcodes can be directly bound to the microparticles or can be bound to the microparticles through any attachment moiety. The microparticles can further comprise oligonucleotides bound thereto and the DNA barcodes can be hybridized to at least a portion of the oligonucleotides bound to the microparticles.

A microparticle used in a method of the invention can be polymeric (such as polystyrene), glass, metallic, semiconductor, or ceramic.

A specific binding pair can be, for example: an antibody and an antigen; a receptor and a ligand; an enzyme and a competitive inhibitor; a drug and a target molecule; or two strands of at least partially complementary oligonucleotides.

In certain embodiments, a DNA barcode is biotinylated, radioactively labeled, or fluorescently labeled.

A target analyte can have more than two binding sites. In some instantces, in addition to its first binding site, a target analyte can be modified to include a second binding site.

In some embodiments, at least two types of microparticle detection probes are provided in a method of the invention, the first type of probe having a specific binding complement to a first binding site on the target analyte and the second type of probe having a specific binding complement to a second binding site on the probe.

In some instances, a plurality of microparticle detection probes are provided, each type of probe having a specific binding complement to different binding sites on the target analyte.

A specific binding complement and target analyte can be members of a specific binding pair. A "member of a specific binding pair" can comprise nucleic acid, oligonucleotide, peptide nucleic acid, polypeptide, antibody, antigen, carbohydrate, protein, peptide, amino acid, hormone, steroid, vitamin, drug, virus, polysaccharides, lipids, lipopolysaccharides, glycoproteins, lipoproteins, nucleoproteins, oligonucleotides, antibodies, immunoglobulins, albumin, hemoglobin, coagulation factors, peptide and protein hormones, non-peptide hormones, interleukins, interferons, cytokines, peptides comprising a tumor-specific epitope, cells, cell-surface molecules, microorganisms, fragments, portions, components or products of microorganisms, small organic molecules, nucleic acids and oligonucleotides, metabolites of or antibodies to any of the above substances. Nucleic acid and oligonucleotide comprise genes, viral RNA and DNA, bacterial DNA, fungal DNA, mammalian DNA, cDNA, mRNA, RNA and DNA fragments, oligonucleotides, synthetic oligonucleotides, modified oligonucleotides, single-stranded and double-stranded nucleic acids, and natural and synthetic nucleic acids.

The following are non-limiting examples of target analyte:specific binding complements. A target analyte can be a nucleic acid and the specific binding complement can be an oligonucleotide. A target analyte can be a protein or hapten and the specific binding complement can be an antibody comprising a monoclonal or polyclonal antibody. A target analyte can be a sequence from a genomic DNA sample and the specific binding complements can be oligonucleotides, the oligonucleotides having a sequence that is complementary to at least a portion of the genomic sequence. Genomic DNA can be eukaryotic, bacterial, fungal or viral DNA. A target analyte can be a sequence from episomal DNA sample and the specific binding complements can be oligonucleotides, the oligonucleotides having a sequence that is complementary to at least a portion of the episomal DNA sequence. A specific binding complement and the target analyte can be members of an antibody-ligand pair.

A "capture substrate" can be any insoluble material to which analytes can be immobilized as described above and throughout this disclosure. A "capture substrate" as used herein captures target analytes from a sample, and can facilitate the separation of these captured target analytes (both before and after treatment with the detection probe) from the sample. Such substrates are typically physically large relative to the analyte and are preferably insoluble in the sample. In particular instances, the methods of the invention comprise the use of magnetic substrates, as described herein, which can be isolated by subjecting the magnetic substrate to a magnetic field.

Additional isolating steps in methods of the invention can comprise any separation methods known to those of skill in the art. For example, separation methods include, but are not limited to, filtration, sedimentation, flotation, hydrodynamics, or gradient methods. A filtration step can comprise, for example, a membrane that removes sample components that do not comprise DNA barcodes.

The DNA barcodes can be released from the microparticles to which they are attached by a chemical releasing agent that will disrupt binding of the barcode to a surface as described above and elsewhere in this disclosure. Such agents, as described herein, include, but are not limited to, any molecule that will preferentially bind to a microparticle through a thiol link such as other thiol- or disulfide-containing molecules, dithiothreitol (DTT), dithioerythritol (DTE), mercaptoethanol and the like, and reducing agents such as sodium borohydride that will cleave a disulfide linkage thereby releasing DNA barcodes from the microparticles to which they are attached.

DNA barcodes can also be released from microparticles by exposing the barcodes to conditions under which the DNA barcodes will dehybridize from oligonucleotides by which the barcodes were attached to the microparticles.

As described herein, a barcode can be labeled with a detectable reporter group. Suitable reporter groups include, but are not limited to, a fluorophore, a chromophore, a redox-active group, a group with an electrical signature, radioactive group, a catalytic group, or Raman label.

The Raman labels can be any one of a number of molecules with distinctive Raman scattering spectra. Unlike the enzymes used in enzyme immunoassays, these label species can be stable, simple, inexpensive molecules which can be chemically modified as required. The following attributes enhance the effectiveness of the label in this application: (a) A strong absorption band in the vicinity of the laser excitation wavelength (extinction coefficient near 10⁴; (b) A functional group which will enable covalent attachment to a specific binding member; (c) Photostability; (d) Sufficient surface and resonance enhancement to allow detection of analyte in the subnanogram range; (e) Minimal interference in the binding interaction between the labeled and unlabeled specific binding members; (f) Minimal exhibition of strong fluorescence emission at the excitation-wavelength used; (g) A relatively simple scattering pattern with a few intense peaks; and/or (h) Labels with scattering patterns which do not interfere with each other so several indicator molecules may be analyzed simultaneously.

The following is a listing of some, but not all potential candidates for these Raman-active label: 4-(4-Aminophenylazo)phenylarsonic acid monosodium salt, arsenazo I, basic fuchsin, Chicago sky blue, direct red 81, disperse orange 3, HABA (2-(4-hydroxyphenylazo)-benzoic acid), crythrosin B, trypan blue, ponceau S, ponceau SS, 1,5-difluoro-2,4-dinitrobenzene, cresyl violet and p-dimethylaminoazobenzene. The chosen labels may be covalently attached to the specific binding members of interest or attached or associated with.

An important aspect is that multiple Raman labels may be bound to the particle to provide a multicoding Raman labels for indexing different particles. Thus, the invention includes a reagent which has multiple Raman dyes and a specific binding substance, such as DNA, RNA, antibody, antigen, small molecule bound to the particle. For particle-based detection probes, the Raman labels or dyes can be attached directly or indirectly to the particle. The Raman label can be modified with a functional group, e.g., a thiol, amine, or phosphine that can bind to the surface of the particle such as a metallic nanoparticle. If desired, the Raman dye can be further functionalized with a molecule such as oligonucleotides (e.g., polyadenosine, polythymidine) for enhanced nanoparticle stability or with a specific binding pair member (such as an oligonucleotide having a sequence that is complementary to at least a portion of a nucleci acid target or a receptor for a particular ligand). Alternatively, the Raman label can be conjugated with a molecule or any linker, e.g., polyA or polyT oligonucleotide, that bears a functional group for binding to the particle. Raman labels can be detected as described, for example, in US Patent Application Serial No. 10/431,341 filed May 7, 2003.

Examples 1-10 provided herein demonstrate the ability to detect simultaneously and, optionally, to quantitate very low concentrations of a large number of different target analytes in a clinical specimen, an environmental sample, an industrial process stream, or other such medium that potentially contains the target analytes. Examples 11 and 12 demonstrate the ability to more rapidly detect fewer different target analytes at similar to higher concentrations. Specifically, Examples 11 and 12 provide modifications of the methods presented and described in Examples 1-10 that allow the methods to be used in less demanding applications while retaining the advantageous sensitivity of the methods. Such modifications also facilitate the use of alternative detection techniques.

One example of a less demanding application requires the simultaneous detection of only a small number, typically between one and ten, of target analytes rather than the tens to hundreds that are within the scope of the methods described in Examples 1-10. In this instance, Examples 11 and 12 conveniently differentiate between and identify target analytes on the basis of the emission wavelengths of fluorescent labels attached to the oligonucleotides of the detection probes rather than by recovering and separating the oligonucleotides prior to detection as practiced in Examples 1-10. Furthermore, the methods described herein permit the detection step to be performed directly upon the ternary sandwich complexes formed between the substrate, target antigen and detection probe, or in solution depending upon the particular end use of the invention. These methods can additionally be extended to emulate the recovery and separation of oligonucleotides as practiced in Examples 1-10, but in a faster and simpler manner.

The methods of the invention require that a target have at least a first and a second feature that differ from each other and that permit the target analyte to be differentiated from all other materials that are present in the medium containing the target analyte. For illustrative purposes, it will be assumed that the target analyte is a protein and that the different features are different epitopes, each of which can be selectively bound by the corresponding antibody. However, any of the types of features and complementary chemical binding moieties, also referred to below as specific binding complements and binding pair members, described herein may be utilized in a similar manner in the practice of these improvements. Where it is desired to detect multiple target analytes that may be present in the medium, one preferred arrangement is for the pair of distinctive features that characterize any one of the target analytes that may be present to be different than the pairs of distinctive features that characterize all of the other target analytes that might be present in the same medium. Alternatively, one distinctive feature may be the same for all of the target analytes that may be present while the second distinctive feature is unique to each type of target analyte. In either case, it is desirable that none of the other materials that may be present in the medium have any of the distinctive features associated with the target analytes.

In addition, the methods of the invention utilize a substrate to which the complementary chemical binding moieties corresponding to the first feature on each target analyte are attached. In the certain methods described herein, a separate substrate bearing the complementary chemical binding moiety that binds the first feature of a target analyte is provided for each target analyte. The invention also provides methods that further allow the use of additional substrate configurations. In one such configuration, the complementary chemical binding moieties corresponding to the first feature of each of the target analytes are bound to the same substrate. In another such configuration, the complementary chemical binding moieties corresponding to the first feature of each of the target analytes are bound to the same substrate in a manner such that the complementary chemical binding moieties corresponding to one target analyte are localized to a different spatial region of the substrate than are the complementary chemical binding moieties corresponding to the other target analytes. These alternative configurations are beneficial in some uses and implementations of the invention.

The substrate used in the methods of the invention serve three primary purposes. One such purpose is to provide a surface upon which the target analyte can be selectively immobilized while materials in the medium that are not target analytes are not so immobilized. This is accomplished by attaching complementary chemical binding moieties that correspond to the first feature of a target analyte to the surface of the substrate and allowing the target analyte to bind to the complementary chemical binding moiety and thus become immobilized on the surface of the substrate. A second such purpose is to facilitate the separation of the target analyte immobilized on the surface of the substrate from the medium. In certain embodiments of the invention, the substrate can be made of a magnetic material that can, along with any material bound to its surface, be separated from the medium by the application of a magnetic field. Numerous other means of separating a substrate from a medium are known and can be likewise employed. Two of many possible illustrative examples are substrates made of materials that are either more or less dense than the medium and which can, therefore, be separated from the medium by sedimentation or floatation, respectively; and substrates that are sufficiently large that they can be separated from the medium by filtration. A substrate can be suspendable in the medium as described herein. Alternatively, the methods of the invention can benefit from the use of a macroscopic substrate such as, but not limited to a microscope slide where spatially separated regions containing the different complementary chemical binding moieties can be formed. The third such purpose of the substrate is that, by virtue of its ability to bind target analytes, it causes the local concentration of the target analytes at the surface of the substrate to be substantially higher than the concentration of the target analytes in the medium. Taken together, these three purposes significantly improve the sensitivity and specificity of the analysis and provide a broad range of options from which the optimum implementation of the invention for any specific use or purpose can be selected.

The methods described herein can utilize a detection probe that comprises a gold nanoparticle approximately 10 to 50 nM in diameter to which are attached a complementary chemical binding moiety that selectively binds to the second feature of a target analyte and a plurality of oligonucleotide pairs. The identity of the target analyte to which any particular detection probe responds can be encoded in the base sequence of the oligonucleotide that is attached to the gold nanoparticle. The oligonucleotide encoding the identity of a particular target analyte has variably been referred to as the "barcode" or the "biobarcode" for that target analyte.

In certain embodiments, the methods of the invention may alternatively utilize detections probes such as described as follows in order to derive benefits such as simple procedures and fast results while retaining sensitivity of methods utilizing gold nanoparticles as described herein. In particular, the surface area of a spherical particle scales as the square of its diameter. The surface area of a one micron diameter sphere is, for example, approximately 1100 times greater than that of a 30nM diameter particle. This means that a one micron diameter spherical particle can accommodate approximately 1100 times as many oligonucleotides as can a 30nM particle, and, as the measurable signal in these inventions is a function of this number of oligonucleotides, a detection probe constructed using a one micron particle can be expected to yield approximately 1100 times the measurable signal per binding event than is available from a 30nM particle. This increased "gain" or "amplification" resulting from the use of a larger particle permits considerable simplifications of the analysis procedure without sacrificing sensitivity. However, the rate of diffusion of the particle and, thus, the frequency with which they move into contact with the target analyte to be detected and the time required to achieve binding to any particular fraction of the target analyte that is present scales inversely with its diameter. The time available for these binding events, therefore, imposes a practical limit upon the largest size of particle that may be employed while still meeting the time constraints that are imposed by any particular use or implementation of the invention. The optimum particle size for any particular use or implementation is, therefore, determined by the balance between the foregoing factors and is typically in the range of between 0.5 and 5.0 microns.

One type of detection probe can be structured identically to the previously described probes based upon 30nM gold particles except that a 1 micron (1000nM) polystyrene microparticle is employed. A second type of detection probe has particular utility when the presence of only a small number of target analytes is to be simultaneously determined. These detection probes of the second type encode the identity of the target analyte in the characteristics of a "detectable entity" bound to an oligonucleotide that is, in turn, bound to the probe particle rather than in the sequence of bases comprising the oligonucleotide. In this context, a "detectable entity" may, for example, be a fluorophore with the identity of the target analyte being encoded in the emission wavelength of a fluorophore. Other suitable detectable entities may include, but are not limited to radioisotopes, electrochemically active species, Raman active species, chemiluminescent species, and the like. Detection probes of this type are particularly suitable for detection by flow cytometric methods and for the in-situ detection of target analytes. A third type of detection probe further allows the oligonucleotides bearing detectable entities to be chemically cleaved from the microparticle to facilitate detection in solution. These improved detection probes provide additional options for optimizing the implementation of the invention for any specific use or purpose.

The present invention relates to a method that utilizes oligonucleotides as biochemical barcodes for detecting multiple analytes in a sample. The approach takes advantage of recognition elements (e.g., proteins or nucleic acids) functionalized either directly or indirectly with nanoparticles and the previous observation that hybridization events that result in the aggregation of gold nanoparticles can significantly alter their physical properties (e.g. optical, electrical, mechanical).⁸⁻¹² The general idea is that each recognition element can be associated with a different oligonucleotide sequence (a DNA barcode) with discrete and tailorable hybridization and melting properties and a physical signature associated with the nanoparticles that changes upon melting to decode a series of analytes in a multi-analyte assay. Therefore, one can use the melting temperature of a DNA-linked aggregate and a physical property associated with the nanoparticles that changes upon melting to decode a series of analytes in a multi-analyte assay. The barcodes herein are different from the ones based on physical diagnostic markers such as nanorods,²³ flourophore-labeled beads,²⁴ and quantum dots,²⁵ in that the decoding information is in the form of chemical information stored in a predesigned oligonucleotide sequence.

The present invention provides several broadly applicable strategies for using nanoparticle probes (preferably gold nanoparticle probes), heavily functionalized with oligonucleotides, to detect a variety of biomolecules, for example, single or multiple polyvalent proteins, in one sample. In particular known methods for detection of multiple proteins in one sample is complicated and often requires time consuming, expensive assay protocols. In this regard, others have recently used fluorophore-labeled peptidonucleic acids and DNA microarrays to recognize multiple protein targets in one solution. ¹⁵⁻¹⁷ However, this method relies on the binding of the proteins labeled with oligonucleotides to a microarray surface. The final step of the method described herein is based solely on the surface chemistry of ordinary DNA. Therefore, it can incorporate many of the high sensitivity aspects of state-of-the-art nanoparticle DNA detection methods, ^{9,11} but allows one to detect a variety of biomolecules, such as proteins, rather than DNA without having the proteins present during the detection event. For surface assays, proteins are typically more difficult to work with than short oligonucleotides because they tend to exhibit greater nonspecific binding to solid supports, which often leads to higher background signals. Finally, for the homogeneous assay, the unusually sharp melting profiles associated with these nanoparticle structures will allow one to design more biobarcodes than what would be possible with probes that exhibit normal and broad DNA melting behavior.

The present invention contemplates the use of any suitable particle having oligonucleotides attached thereto that are suitable for use in detection assays. In practicing this invention, however, microparticles and nanoparticles are preferred. The size, shape and chemical composition of the particles will contribute to the properties of the resulting probe including the DNA barcode. These properties include optical properties, optoelectronic properties, electrochemical properties, electronic properties, stability in various solutions, pore and channel size variation, ability to separate bioactive molecules while acting as a filter, etc. The use of mixtures of particles having different sizes, shapes and/or chemical compositions, as well as the use of nanoparticles having uniform sizes, shapes and chemical composition, are contemplated. Examples of suitable particles include, without limitation, nano- and microsized core particles, aggregate particles, isotropic (such as spherical particles) and anisotropic particles (such as nonspherical rods, tetrahedral, prisms) and core-shell particles such as the ones described in U.S. Patent application no. 10/034,451, filed December 28, 2002 and International application no. PCT/USO 1/50825, filed December 28, 2002. In practicing the invention, the detection probes are preferably generated prior to conducting the actual assay. Alternatively, the detection probes may be generated in situ while conducting the assay.

Thus, in one embodiment of the invention, nanoparticle-conjugate probes are provided. Nanoparticles useful in the practice of the invention include metal (e.g., gold, silver, copper and platinum), semiconductor (e.g., CdSe, CdS, and CdS or CdSe coated with ZnS) and magnetic (e.g., ferromagnetite) colloidal materials. Other nanoparticles useful in the practice of the invention include ZnS, ZnO, TiO₂, AgI, AgBr, HgI₂, PbS, PbSe, ZnTe, CdTe, In₂S₃, In₂Se₃, Cd₃P₂, Cd₃As₂. InAs, and GaAs. The size of the nanoparticles is preferably from about 5 nm to about 150 nm (mean diameter), more preferably from about 30 to about 100 nm, most preferably from about 40 to about 80 nm. The size of microparticles of the invention is preferably from about 1 µm to about 150 µm (mean diameter), more preferably from about 1 µm to about 50 µm, most preferably about 1 µm. The size of the microparticles or nanoparticles used in a method of the invention can be varied as required by their particular use or application. The variation of size can be advantageously used to optimize certain physical characteristics of the nanoparticles, for example, optical properties or amount surface area that can be derivatized. The nanoparticles may also be rods, prisms, or tetrahedra.

Methods of making metal, semiconductor and magnetic nanoparticles are well known in the art. See, *e.g*., Schmid, G. (ed.) Clusters and Colloids (VCH, Weinheim, 1994): Hayat, M.A. (ed.) Colloidal Gold: Principles, Methods, and Applications (Academic Press, San Diego, 1991); Massart, R., IEEE Taransactions On Magnetics, 17, 1247 (1981); Ahmadi, T.S. el al., Science, 272, 1924 (1996); Henglein, A. et al., J. Phys. Chem., 99, 14129 (1995); Curtis, A.C., et al., Angew. Chem. Int. Ed. Engl., 27, 1530 (1988).

Methods of making ZnS, ZnO, TiO₂, AgI, AgBr, HgI₂, PbS, PbSe, ZnTe, CdTe, In₂S₃, In₂Se₃, Cd₃P₂, Cd₃As₂, InAs, and GaAs nanoparticles are also known in the art. See, *e.g.,* Weller, Angew. Chem. Int. Ed. Engl., 32, 41 (1993); Henglein, Top. Curr. Chem., 143, 113 (1988); Henglein, Chem. Rev., 89, 1861 (1989); Brus, Appl. Phys. A., 53, 465 (1991); Bahncmann, in Photochemical Conversion and Storage of Solar Energy (eds. Pelizetti and Schiavello 1991), page 251; Wang and Herron, J. Phys. Chem., 95, 525 (1991); Olshavsky et al., J. Am. Chem. Soc., 112, 9438 (1990); Ushida et al., J. Phys. Chem., 95, 5382 (1992).

Suitable nanoparticles are also commercially available from, *e.g*., Ted Pella, Inc. (gold), Amersham Corporation (gold) and Nanoprobes, Inc. (gold).

Presently preferred for use in detecting nucleic acids are gold nanoparticles. Gold colloidal particles have high extinction coefficients for the bands that give rise to their beautiful colors. These intense colors change with particle size, concentration, interparticle distance, and extent of aggregation and shape (geometry) of the aggregates, making these materials particularly attractive for colorimetric assays. For instance, hybridization of oligonucleotides attached to gold nanoparticles with oligonucleotides and nucleic acids results in an immediate color change visible to the naked eye (see, *e.g*., the Examples).

The nanoparticles, the oligonucleotides or both are functionalized in order to attach the oligonucleotides to the nanoparticles. Such methods are known in the art. For instance, oligonucleotides functionalized with alkanethiols at their 3'-termini or 5'-termini readily attach to gold nanoparticles. See Whitesides, Proceedings of the Robert A. Welch Foundation 39th Conference On Chemical Research Nanophase Chemistry, Houston, TX, pages 109-121 (1995). See also, Mucic et al. Chem. Common. 555-557 (1996) (describes a method of attaching 3' thiol DNA to flat gold surfaces; this method can be used to attach oligonucleotides to nanoparticles). The alkanethiol method can also be used to attach oligonucleotides to other metal, semiconductor and magnetic colloids and to the other nanoparticles listed above. Other functional groups for attaching oligonucleotides to solid surfaces include phosphorothioate groups (see, *e.g*., U.S. Patent No. 5,472,881 for the binding of oligonucleotide-phosphorothioates to gold surfaces), substituted alkylsiloxanes (see, *e.g.* Burwell, Chemical Technology, 4, 370-377 (1974) and Matteucci and Caruthers, J. Am. Chem. Soc., 103, 3185-3191 (1981) for binding of oligonucleotides to silica and glass surfaces, and Grabar et al., Anal. Chem., 67, 735-743 for binding of aminoalkylsiloxanes and for similar binding of mercaptoaklylsiloxanes). Oligonucleotides terminated with a 5' thionucleoside or a 3' thionucleoside may also be used for attaching oligonucleotides to solid surfaces. The following references describe other methods which may be employed to attached oligonucleotides to nanoparticles: Nuzzo et al., J. Am. Chem. Soc., 109, 2358 (1987) (disulfides on gold); Allara and Nuzzo, Langmuir, 1, 45 (1985) (carboxylic acids on aluminum); Allara and Tompkins, J. Colloid Interface Sci., 49, 410-421 (1974) (carboxylic acids on copper); Iler, The Chemistry Of Silica, Chapter 6, (Wiley 1979) (carboxylic acids on silica); Timmons and Zisman, J. Phys. Chem., 69, 984-990 (1965) (carboxylic acids on platinum); Soriaga and Hubbard, J. Am. Chem. Soc., 104, 3937 (1982) (aromatic ring compounds on platinum); Hubbard, Acc. Chem. Res., 13, 177 (1980) (sulfolanes, sulfoxides and other functionalized solvents on platinum); Hickman et al., J. Am. Chem. Soc., 111, 7271 (1989) (isonitriles on platinum); Maoz and Sagiv, Langmuir, 3, 1045 (1987) (silanes on silica); Maoz and Sagiv, Langmuir, 3, 1034 (1987) (silanes on silica); Wasserman et al., Langmuir, 5, 1074 (1989) (silanes on silica); Eltekova and Eltekov, Langmuir, 3, 951 (1987) (aromatic carboxylic acids, aldehydes, alcohols and methoxy groups on titanium dioxide and silica); Lec et al., J. Phys. Chem., 92, 2597 (1988) (rigid phosphates on metals).

In one aspect of this embodiment of the invention, nanoparticles conjugated with dendrimers labeled with at least two types of oligonucleotides are provided. Dendritic molecules are structures comprised of multiple branching unit monomers, and are used in various applications. See, e.g., Barth et al., Bioconjugate Chemistry 5:58-66 (1994); Gitsov & Frechet, Macromolecules 26:6536-6546 (1993); Hawker & Frechet, J. Amer. Chem. Soc. 112:7638-7647 (1990a); Hawker & Frechet, Macromolecules 23:4726-4729 (1990b); Hawker et al., J. Chem. Soc. Perkin Trans. 1:1287-1297 (1993); Lochmann et al. J. Amer. Chem. Soc. 115:7043-7044 (1993); Miller et al., J. Amer. Chem. Soc. 114:1018-1025 (1992); Mousy et al., Macromolecules 25:2401-2406 (1992); Naylor et al., J. Amer. Chem. Soc. 111:2339-2341 (1989); Spindler & Frechet, Macromolecules Macromolecules 26:4809-4813 (1993); Turner et al., Macromolecules 26:4617-4623 (1993); Wiener et al., Magnetic Resonance Med. 31(1):1-8 (1994); Service, 267:458-459 (1995); Tomalia, Sci. Amer. 62-66 (1995); and U.S. Pat. Nos. 4,558,120; 4,507,466; 4,568,737; 4,587,329; 4,857,599; 5,527,524; 5,338,532 to Tomalia, and U.S. Patent 6,274,723 to Nilsen. Dendritic molecules provide important advantages over other types of supermolecular architectures, such as contacting a maximum volume a minimum of structural units, ability to more easily control size, weight, and growth properties, and the multiple termini can be derivatized to yield highly labeled molecules with defined spacing between the labels, or provide sites of attachment for other molecules, or mixtures thereof. See generally U.S. Patent 6,274,723 and the above cited references for methods of synthesis.

Nucleic acid dendrimers that are useful in the methods of the invention are any of those known in the art that can be functionalized with nucleic acids or generated from nucleic acids/oligonucleotides. Such dendrimers can be synthesized according to disclosures such as Hudson et al., "Nucleic Acid Dendrimers: Novel Biopolymer Structures," Am. Chem. Soc. 115:2119-2124 (1993); U.S. Patent 6,274,723; and U.S. Pat. No. 5,561,043 to Cantor.

In another aspect of this embodiment the invention, a universal nanoparticle-oligonucleotide conjugate is provided. The universal provide may be used in an assay for any target nucleic acid that comprises at least two portions. This "universal probe" comprises (i) oligonucleotides of a single "capture" sequence attached to it that are complementary to at least a portion of a reporter oligonucleotide (e.g., barcode DNA), and to a portion of a target recognition oligonucleotide (one embodiment of the universal probe is described in Figures 16A and 16B). The target recognition oligonucleotides comprise a sequence having at least two portions; the first portion comprises complementary sequence to the capture sequence attached to the nanoparticle, and the second portion comprises complementary sequence to the first portion of the particular target nucleic acid sequence. Various types of target recognition oligonucleotides can be used to great advantage with the universal probe, such that a library of target recognition oligonucleotides can be switched or interchanged in order to select for particular target nucleic acid sequences in a particular test solution. A second type of oligonucleotide, which comprises sequence complementary to the second portion of the target nucleic acid, is attached to a support surface, such as a magnetic particle or glass slide.

Contacting the universal probe with a solution comprising the reporter oligonucleotide (barcode DNA) and the target recognition oligonucleotide under conditions that allow for hybridization create a universal probe that is "activated" for contacting with a solution that may contain the target nucleic acid (Fig. 16A, top reaction scheme). The test solution can be contacted under conditions that allow for hybridization, in sequence or in combination with either or both of the "activated" universal probe or the second type of oligonucleotide, which is attached to a support. Once adequate time is allowed for complex formation, the uncomplexed test solution components are removed from the complex, and the reporter oligonucleotides are detected. One embodiment of this assay is depicted in Fig. 16B.

These universal probes can be manipulated for increased advantage, which depend on the particular assay to be conducted. The probes can be "tuned" to various single target nucleic acid sequences, by simply substituting or interchanging the target recognition oligonucleotides such that the second portion comprises complementary sequence to the target nucleic acid of interest. Similarly, if multiple target nucleic acid sequences are to be assayed in a single test solution, the reporter oligonucleotides can comprise a sequence that is specific for each target nucleic acid. Thus, detection of the reporter oligonucleotide of known and specific sequence, would indicate the presence of the particular target nucleic acid in the test solution.

In other aspect of this embodiment of the invention, the oligonucleotides are bound to nanoparticles using sulfur-based functional groups. U.S. patent application nos. 09/760,500 and 09/820,279 and international application nos. PCT/US01/01190 and PCT/US01/10071 describe oligonucleotides functionalized with a cyclic disulfide which are useful in practicing this invention. The cyclic disulfides preferably have 5 or 6 atoms in their rings, including the two sulfur atoms. Suitable cyclic disulfides are available commercially or may be synthesized by known procedures. The reduced form of the cyclic disulfides can also be used.

Preferably, the linker further comprises a hydrocarbon moiety attached to the cyclic disulfide. Suitable hydrocarbons are available commercially, and are attached to the cyclic disulfides. Preferably the hydrocarbon moiety is a steroid residue. Oligonucleotide-nanoparticle conjugates prepared using linkers comprising a steroid residue attached to a cyclic disulfide have unexpectedly been found to be remarkably stable to thiols (e.g., dithiothreitol used in polymerase chain reaction (PCR) solutions) as compared to conjugates prepared using alkanethiols or acyclic disulfides as the linker. Indeed, the oligonucleotide-nanoparticle conjugates of the invention have been found to be 300 times more stable. This unexpected stability is likely due to the fact that each oligonucleotide is anchored to a nanoparticle through two sulfur atoms, rather than a single sulfur atom. In particular, it is thought that two adjacent sulfur atoms of a cyclic disulfide would have a chelation effect which would be advantageous in stabilizing the oligonucleotide-nanoparticle conjugates. The large hydrophobic steroid residues of the linkers also appear to contribute to the stability of the conjugates by screening the nanoparticles from the approach of water-soluble molecules to the surfaces of the nanoparticles.

In view of the foregoing, the two sulfur atoms of the cyclic disulfide should preferably be close enough together so that both of the sulfur atoms can attach simultaneously to the nanoparticle. Most preferably, the two sulfur atoms are adjacent each other. Also, the hydrocarbon moiety should be large so as to present a large hydrophobic surface screening the surfaces of the nanoparticles.

The oligonucleotide-cyclic nanoparticle conjugates that employ cyclic disulfide linkers may be used as probes in diagnostic assays for detecting target analytes in a sample as described in U.S. patent application nos. 09/760,500 and 09/820,279 and international application nos. PCT/US01/01190 and PCT/US01/10071. These conjugates have been found to improve the sensitivity of diagnostic assays in which they are used. In particular, assays employing oligonucleotide-nanoparticle conjugates prepared using linkers comprising a steroid residue attached to a cyclic disulfide have been found to be about 10 times more sensitive than assays employing conjugates prepared using alkanethiols or acyclic disulfides as the linker.

Each nanoparticle will have a plurality of oligonucleotides attached to it. As a result, each nanoparticle-oligonucleotide conjugate can bind to a plurality of oligonucleotides or nucleic acids having the complementary sequence.

Oligonucleotides of defined sequences are used for a variety of purposes in the practice of the invention. Methods of making oligonucleotides of a predetermined sequence are well known. See, *e.g*., Sambrook et al., Molecular Cloning: A Laboratory Manual (2nd ed. 1989) and F. Eckstein (ed.) Oligonucleotides and Analogues, 1st Ed. (Oxford University Press, New York, 1991). Solid-phase synthesis methods are preferred for both oligoribonucleotides and oligodeoxyribonucleotides (the well-known methods of synthesizing DNA are also useful for synthesizing RNA), Oligoribonucleotides and oligodeoxyribonucleotides can also be prepared enzymatically. For oligonuoleotides having bound thereto a specific binding complement to a target analyte, any suitable method for attaching the specific binding complement such as proteins to the oligonucleotide may be used.

Any suitable method for attaching oligonucleotides onto the particle, nanoparticle, or nanosphere surface may be used. A particularly preferred method for attaching oligonucleotides onto a surface is based on an aging process described in U.S. application nos. 09/344,667, filed June 25, 1999; 09/603,830, filed June 26, 2000; 09/760,500, filed January 12, 2001; 09/820,279, filed March 28, 2001; 09/927,777, filed August 10, 2001; and in International applications nos. PCT/US97/12783, filed July 21, 1997; PCT/USOO/17507, filed June 26, 2000; PCT/US01/01190, filed January 12, 2001; PCT/US01/10071, filed March 28, 2001. The aging process provides nanoparticle-oligonucleotide conjugates with unexpected enhanced stability and selectivity.

The method comprises providing oligonucleotides preferably having covalently bound thereto a moiety comprising a functional group which can bind to the nanoparticles. The moieties and functional groups are those that allow for binding (*i.e*., by chemisorption or covalent bonding) of the oligonucleotides to nanoparticles. For instance, oligonucleotides having an alkanethiol, an alkanedisulfide or a cyclic disulfide covalently bound to their 5' or 3' ends can be used to bind the oligonucleotides to a variety of nanoparticles, including gold nanoparticles.

The oligonucleotides are contacted with the nanoparticles in water for a time sufficient to allow at least some of the oligonucleotides to bind to the nanoparticles by means of the functional groups. Such times can be determined empirically. For instance, it has been found that a time of about 12-24 hours gives good results. Other suitable conditions for binding of the oligonucleotides can also be determined empirically. For instance, a concentration of about 10-20 nM nanoparticles and incubation at room temperature gives good results.

Next, at least one salt is added to the water to form a salt solution. The salt can be any suitable water-soluble salt. For instance, the salt may be sodium chloride, magnesium chloride, potassium chloride, ammonium chloride, sodium acetate, ammonium acetate, a combination of two or more of these salts, or one of these salts in phosphate buffer. Preferably, the salt is added as a concentrated solution, but it could be added as a solid. The salt can be added to the water all at one time or the salt is added gradually over time. By "gradually over time" is meant that the salt is added in at least two portions at intervals spaced apart by a period of time. Suitable time intervals can be determined empirically.

The ionic strength of the salt solution must be sufficient to overcome at least partially the electrostatic repulsion of the oligonucleotides from each other and, either the electrostatic attraction of the negatively-charged oligonucleotides for positively-charged nanoparticles, or the electrostatic repulsion of the negatively-charged oligonucleotides from negatively-charged nanoparticles. Gradually reducing the electrostatic attraction and repulsion by adding the salt gradually over time has been found to give the highest surface density of oligonucleotides on the nanoparticles. Suitable ionic strengths can be determined empirically for each salt or combination of salts. A final concentration of sodium chloride of from about 0.1 M to about 1.0 M in phosphate buffer, preferably with the concentration of sodium chloride being increased gradually over time, has been found to give good results.

After adding the salt, the oligonucleotides and nanoparticles are incubated in the salt solution for an additional period of time sufficient to allow sufficient additional oligonucleotides to bind to the nanoparticles to produce the stable nanoparticle-oligonucleotide conjugates. As will be described in detail below, an increased surface density of the oligonucleotides on the nanoparticles has been found to stabilize the conjugates. The time of this incubation can be determined empirically. A total incubation time of about 24-48, preferably 40 hours, has been found to give good results (this is the total time of incubation; as noted above, the salt concentration can be increased gradually over this total time). This second period of incubation in the salt solution is referred to herein as the "aging" step. Other suitable conditions for this "aging" step can also be determined empirically. For instance, incubation at room temperature and pH 7.0 gives good results.

>

The conjugates produced by use of the "aging" step have been found to be considerably more stable than those produced without the "aging" step. As noted above, this increased stability is due to the increased density of the oligonucleotides on the surfaces of the nanoparticles which is achieved by the "aging" step. The surface density achieved by the "aging" step will depend on the size and type of nanoparticles and on the length, sequence and concentration of the oligonucleotides. A surface density adequate to make the nanoparticles stable and the conditions necessary to obtain it for a desired combination of nanoparticles and oligonucleotides can be determined empirically. Generally, a surface density of at least 10 picomoles/cm² will be adequate to provide stable nanoparticle-oligonucleotide conjugates. Preferably, the surface density is at least 15 picomoles/cm². Since the ability of the oligonucleotides of the conjugates to hybridize with nucleic acid and oligonucleotide targets can be diminished if the surface density is too great, the surface density is preferably no greater than about 35-40 picomoles/cm². As used herein, "stable" means that, for a period of at least six months after the conjugates are made, a majority of the oligonucleotides remain attached to the nanoparticles and the oligonucleotides are able to hybridize with nucleic acid and oligonucleotide targets under standard conditions encountered in methods of detecting nucleic acid and methods of nanofabrication.

It has been found that the hybridization efficiency of nanoparticle-oligonucleotide conjugates can be increased dramatically by the use of recognition oligonucleotides which comprise a recognition portion and a spacer portion. "Recognition oligonucleotides" are oligonucleotides which comprise a sequence complementary to at least a portion of the sequence of a nucleic acid or oligonucleotide target. In this embodiment, the recognition oligonucleotides comprise a recognition portion and a spacer portion, and it is the recognition portion which hybridizes to the nucleic acid or oligonucleotide target. The spacer portion of the recognition oligonucleotide is designed so that it can bind to the nanoparticles. For instance, the spacer portion could have a moiety covalently bound to it, the moiety comprising a functional group which can bind to the nanoparticles. These are the same moieties and functional groups as described above. As a result of the binding of the spacer portion of the recognition oligonucleotide to the nanoparticles, the recognition portion is spaced away from the surface of the nanoparticles and is more accessible for hybridization with its target. The length and sequence of the spacer portion providing good spacing of the recognition portion away from the nanoparticles can be determined empirically. It has been found that a spacer portion comprising at least about 10 nucleotides, preferably 10-30 nucleotides, gives good results. The spacer portion may have any sequence which does not interfere with the ability of the recognition oligonucleotides to become bound to the nanoparticles or to a nucleic acid or oligonucleotide target. For instance, the spacer portions should not have sequences complementary to each other, to that of the recognition olignucleotides, or to that of the nucleic acid or oligonucleotide target of the recognition oligonucleotides. Preferably, the bases of the nucleotides of the spacer portion are all adenines, all thymines, all cytidines, or all guanines, unless this would cause one of the problems just mentioned. More preferably, the bases are all adenines or all thymines. Most preferably the bases are all thymines.

It has further been found that the use of diluent oligonucleotides in addition to recognition oligonucleotides provides a means of tailoring the conjugates to give a desired level of hybridization. The diluent and recognition oligonucleotides have been found to attach to the nanoparticles in about the same proportion as their ratio in the solution contacted with the nanoparticles to prepare the conjugates. Thus, the ratio of the diluent to recognition oligonucleotides bound to the nanoparticles can be controlled so that the conjugates will participate in a desired number of hybridization events. The diluent oligonucleotides may have any sequence which does not interfere with the ability of the recognition oligonucleotides to be bound to the nanoparticles or to bind to a nucleic acid or oligonucleotide target. For instance, the diluent oligonulceotides should not have a sequence complementary to that of the recognition olignucleotides or to that of the nucleic acid or oligonucleotide target of the recognition oligonucleotides. The diluent oligonucleotides are also preferably of a length shorter than that of the recognition oligonucleotides so that the recognition oligonucleotides can bind to their nucleic acid or oligonucleotide targets. If the recognition oligonucleotides comprise spacer portions, the diluent oligonulceotides are, most preferably, about the same length as the spacer portions. In this manner, the diluent oligonucleotides do not interefere with the ability of the recognition portions of the recognition oligonucleotides to hybridize with nucleic acid or oligonucleotide targets. Even more preferably, the diluent oligonucleotides have the same sequence as the sequence of the spacer portions of the recognition oligonucleotides.

In another embodiment of the invention, particle complex probes are provided. Each type of particle complex probe contains a predetermined reporter oligonucleotide or barcode for a particular target analyte. In the presence of target analyte, aggregates are produced as a result of the binding interactions between the particle complex and the target analyte. These aggregates can be isolated and analyzed by any suitable means, e.g., thermal denaturation, to detect the presence of one or more different types of reporter oligonucleotides. In practicing this invention, nanoparticle complex probes are preferred.

Thus, in one aspect of the invention, the particle complex probe comprises a particle having oligonucleotides bound thereto, one or more DNA barcodes, and an oligonucleotide having bound thereto a specific binding complement to a specific target analyte, wherein (i) the DNA barcode has a sequence having at least two portions; (ii) at least some of the oligonucleotides attached to the particle have a sequence that is complementary to a first portion of a DNA barcode; (iii) the oligonucleotide having bound thereto a specific binding complement have a sequence that is complementary to a second portion of a DNA barcode; and (iv) the DNA barcode in each type of particle complex probe has a sequence that is different and that serves as an identifier for a particular target analyte.

In another aspect of this embodiment, the particle complex probe comprises a particle having at least two types of oligonucleotides bound thereto, one or more DNA barcodes, and an oligonucleotide having bound thereto a specific binding complement to a target analyte, wherein a first type of oligonucleotides bound to the probe having a sequence that is complementary to at least a portion of the DNA barcode, the second type of oligonucleotide bound to the probe having a sequence that is complementary to at least a portion of the sequence of the oligonucleotide having a specific binding complement.

In another aspect of this embodiment the particle complex probe comprising a particle having oligonucleotides bound thereto, one or more DNA barcodes, and a specific binding complement to a target analyte, wherein at least a portion of the oligonucleotides bound to the particle have a sequence that is complementary to at least a portion of the sequence of the DNA barcode and where the DNA barcode serves as an identifier for a specific target analyte.

In yet another embodiment of the invention, a particle complex probe is provided. Thus in one embodiment of the invention, a particle complex probe is provided which comprises a particle having oligonucleotides bound thereto, a DNA barcode, and an oligonucleotide having bound thereto a specific binding complement to a specific target analyte, wherein (i) the DNA barcode has a sequence having at least two portions; (ii) at least some of the oligonucleotides attached to the particle have a sequence that is complementary to a first portion of a DNA barcode; (iii) the oligonucleotide having bound thereto a specific binding complement have a sequence that is complementary to a second portion of a DNA barcode; and (iv) the DNA barcode in each type of particle complex probe has a sequence that is different and that serves as an identifier for a particular target analyte.

In another embodiment of the invention, a particle complex probe is provided which comprises a particle having at least two types of oligonucleotides bound thereto, a DNA barcode, and an oligonucleotide having bound thereto a specific binding complement to a target analyte, wherein a first type of oligonucleotides bound to the probe having a sequence that is complementary to at least a portion of the DNA barcode, the second type of oligonucleotide bound to the probe having a sequence that is complementary to at least a portion of the sequence of the oligonucleotide having a specific binding complement.

In yet another embodiment of the invention, a particle complex probe is provided which comprises a particle having oligonucleotides bound thereto, a DNA barcode, and a specific binding complement to a target analyte, wherein at least a portion of the oligonucleotides bound to the particle have a sequence that is complementary to at least a portion of the sequence of the DNA barcode and where the DNA barcode serves as an identifier for a specific target analyte.

In yet another embodiment of the invention, a detection probe is provided which comprises a nanoparticle; a member of a specific binding pair bound to the nanoparticle; at least one type of oligonucleotide bound to the nanoparticle; and at least one type of DNA barcode each having a predetermined sequence, wherein each type of DNA barcode is hybridized to at least a portion of the at least one type of oligonucleotide.

Preferably the particles comprise nanoparticles as described above such as metal, semiconductor, insulator, or magnetic nanoparticles. Preferably the particles are gold nanoparticles. The the specific binding complement or binding pair member and the target analyte are members of a specific binding pair which comprises nucleic acid, oligonucleotide, peptide nucleic acid, polypeptide, antibody, antigen, carbohydrate, protein, peptide, amino acid, hormone, steroid, vitamin, drug, virus, polysaccharides, lipids, lipopolysaccharides, glycoproteins, lipoproteins, nucleoproteins, oligonucleotides, antibodies, immunoglobulins, albumin, hemoglobin, coagulation factors, peptide and protein hormones, non-peptide hormones, interleukins, interferons, cytokines, peptides comprising a tumor-specific epitope, cells, cell-surface molecules, microorganisms, fragments, portions, components or products of microorganisms, small organic molecules, nucleic acids and oligonucleotides, metabolites of or antibodies to any of the above substances.

In another embodiment of the invention, methods are provided for detecting for the presence or absence of one or more target analytes, the target analyte having at least two binding sites, in a sample. In one aspect of this embodiment of the invention, a method is provided which comprises the steps of:
providing a substrate;
providing one or more types of particle probes, each type of probe comprising a particle having one or more specific binding complements to a specific target analyte and one or more DNA barcodes bound thereto, wherein the specific binding complement of each type of particle probe is specific for a particular target analyte, and the DNA barcode for each type of particle probe serves as a marker for the particular target analyte;
immobilizing the target analytes onto the substrate;
contacting the immobilized target analytes with one or more types of particle probes under conditions effective to allow for binding between the target analyte and the specific binding complement to the analyte and form a complex in the presence of the target analyte;
washing the substrate to remove unbound particle probes; and
optionally amplifying the DNA barcode; and
detecting for the presence or absence of the amplified DNA barcode wherein the presence or absence of the marker is indicative of the presence or absence of a specific target analyte in the sample.

In one aspect of this embodiment of the invention, the target analyte is a protein or hapten and its specific binding complement is an antibody comprising a monoclonal or polyclonal antibody.

In another aspect of the invention, any suitable substrate may be used. The substrate may be arrayed with one or more types of capture probes for the target analytes.

In this aspect of the invention, the barcode may be isolated. Analyte detection occurs indirectly by ascertaining for the presence of reporter oligonucleotide or biobarcode by any suitable means such as a DNA chip.

DNA barcode can optionally be amplified by any suitable means including PCR amplification, and then be detected by any suitable DNA detection system using any suitable detection probes. The particle is preferably labeled with a sufficient amount of DNA barcodes to provide sufficient signal amplification and eliminate the need for DNA barcode amplification. In practicing this invention, amplification by the PCR method is preferred. PCR amplification (herein also referred to as *B*PCR) of the DNA barcode allows one to detect a protein target at attomolar level. The assay as illustrated in Figure 6, utilizes a new type of nanoparticle heavily functionalized with hybridized oligonucleotides (biobarcodes)³⁶ and polyclonal detection antibodies to recognize a target analyte, the prostate specific antigen (PSA) (Example 5). In addition, polyamine microparticles (1µm diameter) with magnetic iron oxide cores are funtionalized with PSA monoclonal antibodies (Figure 6 and Example 5). The gold nanoparticles and the polyamine microparticles sandwich the PSA target, generating a complex with a large ratio of barcode DNA to protein target (for 13 nm particles, each particle can support up to 200 strands of DNA; this represents the upper limit for this size particle). Application of a magnetic field draws the magnetic particles to the wall of the reaction vessel in a matter of seconds, allowing one to separate both reacted and unreacted microparticles but only reacted nanoparticles from the reaction mixture. Washing the aggregate structures in Nanopure water (18 MOhms) dehybridizes barcode DNA from nanoparticle-immobilized complements. Using the magnetic separator, the aggregate can be easily removed from the assay solution, leaving the barcode DNA, which can be amplified using PCR, and subsequently and quickly identified by standard DNA detection methodologies (scanometric¹¹, gel electrophoresis, or fluorophore-labeling approaches). PSA was chosen as the initial target for these studies because of its importance in the early detection of prostate cancer, one of the most common cancers and second leading cause of cancer death in American men ^{47, 48}. Importantly, identification of disease relapse following the surgical treatment of prostate cancer using PSA as a marker present at low levels (10s of copies), could be extremely beneficial and enable the delivery of curative adjuvant therapies ^{46, 49}.

Examples 5-6 demonstrate that *B*PCR is an extremely powerful method for detecting protein analytes, namely PSA, at low attomolar concentrations in the presence of background proteins using either gel electrophoresis or scanometric microarray detection. The work demonstrates several advantages over current protein detection methods. First, the target binding protein of the assay is homogeneous. Therefore, one can add a large quantity of magnetic particles to the reaction vessel to facilitate the binding kinetics between the detection antibody and target analyte. This leads to an assay that is faster than heterogeneous systems and also allows one to increase sensitivity because the capturing step is more efficient. Second, the use of the nanoparticle biobarcodes provides a high ratio of PCR-amplifiable DNA to labeling antibody serving to substantially increase assay sensitivity. For example, the *B*PCR assay reported herein was able to detect PSA at 3 aM concentration while a PCR-based immunoassay has been reported to have a detection limit of 3 fM for the same target analyte ⁴³. Third, this assay obviates the need for complicated conjugation chemistry for attaching DNA to the labeling antibodies. Barcode DNA is bound to the nanoparticle probe through hybridization at the start of the labeling reaction and liberated for PCR amplification using a simple wash step. Ad the labeling antibody and DNA are present on the same particle, there is no need for the addition of further antibodies or DNA-protein conjugates prior to the PCR amplification of barcode DNA. In addition, the barcode DNA is removed from the detection assay, and PCR is carried out on samples of barcode DNA that is free from PSA, most of the biological sample, the microparticles, and nanoparticles. This substantially reduces background signal. Finally, this protein detection scheme has the potential for massive multiplexing and the simultaneous detection of many analytes in one solution. Although the PSA system is used for proof-of-concept, the approach should be general for almost any target with known binding partners, and by using the nanoparticle-based biobarcode approach ³⁶, one can prepare a unique identifiable barcode for virtually every target of interest.

Example 9 demonstrates that the probes of the invention can be used to detect and measure directly the amount of target analyte in a sample. Thus, a step comprising *B*PCR to amplify the barcode DNA is not required to achieve excellent sensitivity and detection limits (Figs. 6B (step 4), 9 (inset), and 12).

Any suitable washing solution that removes unbound probes from the surface of the substrate after complex foliation may be used. A representative example includes, without limitation, PBS (phosphate buffer solution).

In the presence of target analyte, nanoparticle aggregate complexes are produced as a result of the binding interactions between the nanoparticle complex probe and the target analyte. These aggregates are may isolated and subject to conditions effective to dehybridize the aggregate and to release the reporter oligonucleotide. The reporter oligonucleotide is then isolated. If desired, the reporter oligonucleotide may be amplified by any suitable means including PCR amplification. Analyte detection occurs indirectly by ascertaining for the presence of reporter oligonucleotide or biobarcode by any suitable means such as a DNA chip.

The DNA barcodes or reporter oligonucleotides may then be detected by any suitable means. Generally, the DNA barcodes are released via dehybridization from the complex prior to detection. Any suitable solution or media may be used that dehybridize and release the DNA barcode from the complex. A representative medium is water.

The DNA barcodes released by dehybridization of the aggregates can be directly detected using a substrate having capture oligonucleotides bound thereto. The oligonucleotides have a sequence complementary to at least one portion of the reporter oligonucleotides. Some embodiments of the method of detecting the DNA barcodes utilize a substrate having complementary oligonucleotides bound thereto to capture the reporter oligonucleotides. These captured reporter oligonucleotides are then detected by any suitable means. By employing a substrate, the detectable change (the signal) can be amplified and the sensitivity of the assay increased.

Any suitable method for attaching oligonucleotides to a substrate may be used. For instance, oligonucleotides can be attached to the substrates as described in, *e.g*., Chrisey et al., Nucleic Acids Res., 24, 3031-3039 (1996); Chrisey et al., Nucleic Acids Res., 24, 3040-3047 (1996); Mucic et al., Chem. Commun., 555 (1996); Zimmermann and Cox, Nucleic Acids Res., 22, 492 (1994); Bottomley et al., J. Vac. Sci. Technol. A, 10, 591 (1992); and Hegner et al., FEBS Lett., 336, 452 (1993).

The oligonucleotides attached to the substrate have a sequence complementary to a first portion of the sequence of reporter oligonucleotides to be detected. The reporter oligonucleotide is contacted with the substrate under conditions effective to allow hybridization of the oligonucleotides on the substrate with the reporter oligonucleotide. In this manner the reporter oligonucleotide becomes bound to the substrate. Any unbound reporter oligonucleotide is preferably washed from the substrate before adding a detection probe such as nanoparticle-oligonucleotide conjugates.

In one aspect of the invention, the reporter oligonucleotide bound to the oligonucleotides on the substrate is contacted with a first type of nanoparticles having oligonucleotides attached thereto. The oligonucleotides have a sequence complementary to a second portion of the sequence of the reporter oligonucleotide, and the contacting takes place under conditions effective to allow hybridization of the oligonucleotides on the nanoparticles with the reporter oligonucleotide. In this manner the first type of nanoparticles become bound to the substrate. After the nanoparticle-oligonucleotide conjugates are bound to the substrate, the substrate is washed to remove any unbound nanoparticle-oligonucleotide conjugates.

The oligonucleotides on the first type of nanoparticles may all have the same sequence or may have different sequences that hybridize with different portions of the reporter oligonucleotide to be detected. When oligonucleotides having different sequences are used, each nanoparticle may have all of the different oligonucleotides attached to it or, preferably, the different oligonucleotides are attached to different nanoparticles. Alternatively, the oligonucleotides on each of the first type of nanoparticles may have a plurality of different sequences, at least one of which must hybridize with a portion of the reporter oligonucleotide to be detected.

Optionally, the first type of nanoparticle-oligonucleotide conjugates bound to the substrate is contacted with a second type of nanoparticles having oligonucleotides attached thereto. These oligonucleotides have a sequence complementary to at least a portion of the sequence(s) of the oligonucleotides attached to the first type of nanoparticles, and the contacting takes place under conditions effective to allow hybridization of the oligonucleotides on the first type of nanoparticles with those on the second type of nanoparticles. After the nanoparticles are bound, the substrate is preferably washed to remove any unbound nanoparticle-oligonucleotide conjugates.

The combination of hybridizations produces a detectable change. The detectable changes are the same as those described above, except that the multiple hybridizations result in an amplification of the detectable change. In particular, since each of the first type of nanoparticles has multiple oligonucleotides (having the same or different sequences) attached to it, each of the first type of nanoparticle-oligonucleotide conjugates can hybridize to a plurality of the second type of nanoparticle-oligonucleotide conjugates. Also, the first type of nanoparticle-oligonucleotide conjugates may be hybridized to more than one portion of the reporter oligonucleotide to be detected. The amplification provided by the multiple hybridizations may make the change detectable for the first time or may increase the magnitude of the detectable change. This amplification increases the sensitivity of the assay, allowing for detection of small amounts of reporter oligonucleotide.

If desired, additional layers of nanoparticles can be built up by successive additions of the first and second types of nanoparticle-oligonucleotide conjugates. In this way, the number of nanoparticles immobilized per molecule of target nucleic acid can be further increased with a corresponding increase in intensity of the signal.

Also, instead of using first and second types of nanoparticle-oligonucleotide conjugates designed to hybridize to each other directly, nanoparticles bearing oligonucleotides that would serve to bind the nanoparticles together as a consequence of hybridization with binding oligonucleotides could be used.

When a substrate is employed, a plurality of the initial types of nanoparticle-oligonucleotide conjugates or oligonucleotides can be attached to the substrate in an array for detecting multiple portions of a target reporter oligonucleotide, for detecting multiple different reporter oligonucleotides, or both. For instance, a substrate may be provided with rows of spots, each spot containing a different type of oligonucleotide designed to bind to a portion of a target reporter oligonucleotide. A sample containing one or more reporter oligonucleotides is applied to each spot, and the rest of the assay is performed in one of the ways described above using appropriate oligonucleotide-nanoparticle conjugates.

In yet another aspect, the methods of analyte detection by *B*PCR, as well as direct detection, can be adapted for use with methods that comprise analyte detection on a substrate, for example, glass, gold, silicon, nickel, plastics, and the like. These methods can also be adapted to detect other biological and chemical recognition events such as DNA-protein binding events, physiological protein-protein binding or dimerization, and other biomolecular interactions previously described above.

In one embodiment of this aspect, the method comprises attaching one or more types of capture probe for each target analyte to a substrate, contacting the substrate with a test solution, optionally washing the test solution from the substrate, subsequently contacting the substrate with one or more types of detection probe for each target analyte, removing any unbound detection probe, and detecting an observable signal, wherein the detection of an observable signal indicates the presence of the target analyte in the test solution.

In this aspect of the invention, the observable signal can be detected using any method described herein. For example, direct detection of barcode DNAs, detection of *B*PCR-amplified barcode DNAs, detection of aggregation of the one or more types of detection probe (e.g., by visual inspection, fluorescence, colorimetric, electrochemistry, electronic, densitometry, radioactivity, and the like), or by using reporter nucleotides that comprise a sequence that is complementary to at least a portion of the barcode DNAs and a detectable signal moiety (e.g., fluorescent label).

When a substrate is employed, a detectable change can be produced or further enhanced by staining such as silver or gold staining. Silver staining can be employed with any type of nanoparticles that catalyze the reduction of silver. Preferred are nanoparticles made of noble metals (*e.g*., gold and silver). See Bassell, et al., J. Cell Biol., 126, 863-876 (1994); Braun-Howland et al., Biotechniques, 13, 928-931 (1992). If the nanoparticles being employed for the detection of a nucleic acid do not catalyze the reduction of silver, then silver ions can be complexed to the nucleic acid to catalyze the reduction. See Braun et al., Nature, 391, 775 (1998). Also, silver stains are known which can react with the phosphate groups on nucleic acids.

Silver staining can be used to produce or enhance a detectable change in any assay performed on a substrate, including those described above. In particular, silver staining has been found to provide a huge increase in sensitivity for assays employing a single type of nanoparticle so that the use of layers of nanoparticles can often be eliminated.

In assays for detecting reporter oligonucleotides performed on a substrate, the detectable change can be observed with an optical scanner. Suitable scanners include those used to scan documents into a computer which are capable of operating in the reflective mode (*e.g*., a flatbed scanner), other devices capable of performing this function or which utilize the same type of optics, any type of greyscale-sensitive measurement device, and standard scanners which have been modified to scan substrates according to the invention (*e.g*., a flatbed scanner modified to include a holder for the substrate) (to date, it has not been found possible to use scanners operating in the transmissive mode). The resolution of the scanner must be sufficient so that the reaction area on the substrate is larger than a single pixel of the scanner. The scanner can be used with any substrate, provided that the detectable change produced by the assay can be observed against the substrate (*e.g*., a gray spot, such as that produced by silver staining, can be observed against a white background, but cannot be observed against a grey background). The scanner can be a black-and-white scanner or, preferably, a color scanner. Most preferably, the scanner is a standard color scanner of the type used to scan documents into computers. Such scanners are inexpensive and readily available commercially. For instance, an Epson Expression 636 (600 x 600 dpi), a UMAX Astra 1200 (300 x 300 dpi), or a Microtec 1600 (1600 x 1600 dpi) can be used. The scanner is linked to a computer loaded with software for processing the images obtained by scanning the substrate. The software can be standard software which is readily available commercially, such as Adobe Photoshop 5.2 and Corel Photopaint 8.0. Using the software to calculate greyscale measurements provides a means of quantifying the results of the assays. The software can also provide a color number for colored spots and can generate images (*e.g*., printouts) of the scans which can be reviewed to provide a qualitative determination of the presence of a nucleic acid, the quantity of a nucleic acid, or both. The computer can be a standard personal computer which is readily available commercially. Thus, the use of a standard scanner linked to a standard computer loaded with standard software can provide a convenient, easy, inexpensive means of detecting and quantifying nucleic acids when the assays are performed on substrates. The scans can also be stored in the computer to maintain a record of the results for further reference or use. Of course, more sophisticated instruments and software can be used, if desired.

In another embodiment of the invention, a method is provided for detecting for the presence or absence of one or more target analytes in a sample, each target analyte having at least two binding sites, the method comprising:
providing one or more types of capture probes bound to a substrate, each type of capture probe comprising a specific binding complement to a first binding site of a specific target analyte;
providing one or more types of detection probes, each type of detection probe comprising a nanoparticle having oligonucleotides bound thereto, one or more specific binding complements to a second binding site of the specific target analyte, and one or more DNA barcodes that serve as a marker for the particular target analyte, wherein at least a portion of a sequence of the DNA barcodes is hybridized to at least some of the oligonucleotides bound to the nanoparticles
contacting the sample, the capture probe, and the detection probe under conditions effective to allow specific binding interactions between the target analyte and the probes and to form an aggregate complex in the presence of the target analyte;
washing the substrate to remove any unbound detection probes;
detecting for the presence or absence of the DNA barcode in any aggregate complex on the substrate, wherein the detection of the presence or absence of the DNA barcode is indicative of the presence or absence of the target analyte in the sample.

In one aspect of this embodiment of invention, the detection probe comprises (i) one or more specific binding complements to the second binding site of a specific target analyte, (ii) at least one type of oligonucleotides bound to the nanoparticle, and a DNA barcode having a predetermined sequence that is complementary to at least a portion of at least one type of oligonucleotides, the DNA barcode bound to each type of detection probe serving as a marker for a specific target analyte;

In another aspect of this embodiment, prior to said detecting step, the method further comprising the steps of:
subjecting the aggregate complex to conditions effective to dehybridize the complex and release the DNA barcodes; and
optionally amplifying the DNA barcode prior to said detecting.

In another aspect of the invention, the capture probe is bound to a magnetic substrate such as a magnetic particle, e.g., a polystyrene MMP with a magnetic iron oxide. This allows for facile removal of complexes from solution. DNA barcodes can then be detected directly using the substrate-based detection technique described above or indirectly by amplification followed by a detection technique. In the Examples below, a method based on oligonucleotide-modified nanoparticles (NPs), magnetic microparticles (MMPs), and the subsequent detection of barcode DNA that serve as amplifiers of one or more target nucleic acid sequences is described. Preferably, the oligonucleotide-modified nanoparticles comprise gold nanoparticles. The detection of the presence or absence of target DNA signal (via detection of bar-code DNA) is preferably performed using a substrate-based detection method as discussed above.

In one aspect, the invention provides a target nucleic acid amplification method that does not rely on PCR methods, and is based on oligonucleotide-modified nanoparticles (NPs), magnetic microparticles (MMPs), and detection of amplified target nucleic acid in the form of barcode DNA. In one embodiment of this aspect, the oligonucleotide-modified nanoparticles (NPs) comprise gold nanoparticles. In another embodiment of this aspect, the detection of the amplified DNA signal (bar-code DNA specific for a target sequence) is performed using a chip-based detection method. In another embodiment of this aspect, the barcode DNA comprises a sequence specific for each target nucleic acid molecule of interest, allowing for specific detection of multiple target nucleic acid sequences in a test solution.

In another aspection of the invention, the barcode DNA comprises a sequence specific for each particular target analyte of interest in a test sample, allowing for the detection of multiple specific targets in a single assay/test solution. As shown in the Examples, detection limits as low as about 500 zeptomolar (zM) can be achieved (the "zepto" order of magnitude is 10⁻²¹; *e.g*., 10 copies in an entire 20 µL sample). Such detection limits represent a significant increase in the sensitivity of PCR-less detection of target nucleic acid molecules.

In this aspect, two types of probes are provided for target DNA detection (DNA-BCA). In certain embodiments the first type of probe is a polystyrene MMP with a magnetic iron oxide core, functionalized with oligonucleotides that are complementary to at least a portion of a target sequence. The complementary portion of the oligonucleotides of the MMP can have various lengths, depending on the particular assay conditions (e.g., buffer system, target nucleic acid sequence, temperature, etc.).

In another embodiment, the second probe comprises a nanoparticle modified with two types of oligonucleotides, one that comprises a sequence that is complementary to at least a portion of a target sequence that is different from the region on the target that is recognized by the MMP; and the other comprises a sequence that is complementary to at least a portion of barcode DNA sequence, which barcode DNA provides a unique identification tag for the particular target sequence. In certain embodiments the nanoparticle is a gold nanoparticle. In further embodiments the gold nanoparticle comprises 13, 20, or 30 nm gold nanoparticles.

The ratio of barcode DNA to target binding sequence on the nanoparticle surface can be varied to suit each individual assay. In order to provide for PCR-less detection of barcode DNA, the ratio of barcode DNA to target binding DNA should be greater than 1:1, preferably at least about 25:1, more preferably at least about 50:1, most preferably at least about 100:1. The higher ratios provide for PCR-less target amplification because the barcode DNA, not the target sequence, is identified and detected in the DNA-BCA methods. For example, a 13 nm gold nanoparticle can accomodate at least 100 thiolated DNA strands per particle.⁷³ For 20 and 30 nm nanoparticles, assuming comparable oligonucleotide loading and a spherical shape for each particle, the particles can accommodate approximately 240 and 530 immobilized oligonucleotides, respectively.

In another aspect of this embodiments, the specific binding complement bound to the nanoparticle is a monoclonal or polyclonal antibody.

In another aspect of this embodiment, the specific binding complement bound to the capture probe is a monoclonal antibody.

In another aspect of this embodiment, the antibody is an anti-PSA antibody.

In another aspect of this embodiment, prior to said washing step, the method further comprises the step of:
isolating the aggregated complex prior to washing by subjecting the aggregated complex bound to the magnetic particle to a magnetic field.

In another aspect of this embodiment, the method further comprises the step of: subjecting the isolated aggregated complex to conditions effective to dehybridize the aggregated complex and release the DNA barcode.

In another aspect of this embodiment, the released DNA barcode is amplified by any suitable technique such as PCR.

In another aspect of this embodiment, the target analyte is a nucleic acid having at least two portions.

In another aspect of this embodiment, the target analyte is a target nucleic acid having a sequence of at least two portions, the detection probe comprises a nanoparticle having at oligonucleotides having a sequence that is complementary to the DNA bar code, the specific binding complement of the detection probe comprising a first target recognition oligonucleotide having a sequence that is complementary to a first portion of the target nucleic acid, and the specific binding complement of the capture probes comprises second target recognition oligonucleotide having a sequence that is complementary to at least a second portion of the target nucleic acid.

In another aspect of this embodiment, the target analyte is a target nucleic acid having a sequence of at least two portions, the detection probe comprising a nanoparticle having oligonucleotides bound thereto, the DNA barcode having a sequence that is complementary to at least a portion of the oligonucleotides bound to the detection probe, the specific binding complement comprises a target recognition oligonucleotide having a sequence of at least first and second portions, the first portion is complementary to a first portion of the target nucleic acid and the second portion is complementary to a least a portion of the oligonucleotides bound to the nanoparticles, the specific binding complement of the substrate comprising a target recognition oligonucleotide having at least a portion that is complementary to a second portion of the target nucleic acid.

In another aspect of this embodiment, the detection probe comprises a dendrimeric nanoparticle as described above.

In yet another embodiment of this invention, a method is provided for detecting for the presence or absence of one or more target analytes in a sample, each target analyte having at least two binding sites, the method comprising:
providing one or more types of capture probes, each type of capture probe comprising (i) a magnetic particle; and (ii) a first member of a first specific binding pair attached to the magnetic particle, wherein the first member of the first specific binding pair binds to a first binding site of a specific target analyte;
providing one or more types of detection probe for each target analyte, each type of detection probe comprising (i) a nanoparticle; (ii) a first member of a second specific binding pair attached to the nanoparticle, wherein the first member of the second specific binding pair binds to a second binding site of the target analyte; (iii) at least one type of oligonucleotides bound to the nanoparticle; and (iv) at least one type of DNA barcodes, each type of DNA barcode having a predetermined sequence that is complementary to at least a portion of a specific type of oligonucleotides and serves as a marker for a specific target analyte;
contacting the sample with the capture probe and the detection probe under conditions effective to allow specific binding interactions between the target analyte and the probes and to form an aggregated complex bound to the magnetic particle in the presence of the target analyte;
washing any unbound detection probes from the magnetic particle; and
detecting for the presence or absence of the DNA barcodes in the complex, wherein the detection of the DNA barcode is indicative of the presence of the target analyte.

In one aspect of this embodiment, the method further comprises, prior to said detecting step, the steps of:
isolating the aggregated complex by applying a magnetic field;
subjecting the aggregated complex to conditions effective to dehybridize and release the DNA barcodes from the aggregated complex;
isolating the released DNA barcodes.

In another aspect of this embodiment, the method further comprises amplifying the released DNA barcodes.

In another aspect of this embodiment, the method further comprises:
providing a substrate having oligonucleotides bound thereto, the oligonucleotides having a sequence complementary to at least a portion of the sequence of the DNA barcode;
providing a nanoparticle comprising oligonucleotides bound thereto, wherein at least portion of the oligonucleotides bound to the nanoparticles have a sequence that is complementary to at least a portion of a DNA barcode; and
contacting the DNA barcodes, the oligonucleotides bound to the substrate, and the nanoparticles under conditions effective to allow for hybridization at least a first portion of the DNA barcodes with a complementary oligonucleotide bound to the substrate and a second portion of the DNA barcodes with some of the oligonucleotides bound to the nanoparticles.

In another aspect of this embodiment, the DNA barcode is amplified by PCR prior to detection.

In another aspect of this embodiment, the method further comprises isolating the aggregated complexes prior to analyzing the aggregated complex.

In another aspect of this embodiment, the aggregated complex is isolated by applying a magnetic field to the aggregated complex.

In another aspect of this embodiment, the nanoparticles are metal nanoparticles such as gold nanoparticles or semiconductor nanoparticles.

In another aspect of this embodiment, the specific binding pair is an antibody and an antigen; a receptor and a ligand; an enzyme and a substrate; a drug and a target molecule; an aptamer and an aptamer target; two strands of at least partially complementary oligonucleotides.

In another aspect of this embodiment, the DNA barcode may be biotinylated, radioactively labeled, or fluorescently labeled.

In any of the embodiments, at least two types of particle complex probes are provided, the first type of probe having a specific binding complement to a first binding site on the target analyte and the second type of probe having a specific binding complement to a second binding site on the probe. A plurality of particle complex probes are provided, each type of probe having a specific binding complement to different binding sites on the target analyte.

The specific binding complement and the target analyte are members of a specific binding pair which comprise nucleic acid, oligonucleotide, peptide nucleic acid, polypeptide, antibody, antigen, carbohydrate, protein, peptide, amino acid, hormone, steroid, vitamin, drug, virus, polysaccharides, lipids, lipopolysaccharides, glycoproteins, lipoproteins, nucleoproteins, oligonucleotides, antibodies, immunoglobulins, albumin, hemoglobin, coagulation factors, peptide and protein hormones, non-peptide hormones, interleukins, interferons, cytokines, peptides comprising a tumor-specific epitope, cells, cell-surface molecules, microorganisms, fragments, portions, components or products of microorganisms, small organic molecules, nucleic acids and oligonucleotides, metabolites of or antibodies to any of the above substances.

The nucleic acid and oligonucleotide comprise genes, viral RNA and DNA, bacterial DNA, fungal DNA, mammalian DNA, cDNA, mRNA, RNA and DNA fragments, oligonucleotides, synthetic oligonucleotides, modified oligonucleotides, single-stranded and double-stranded nucleic acids, natural and synthetic nucleic acids, and aptamers.

The target analyte is a nucleic acid and the specific binding complement is an oligonucleotide. Alternatively, the target analyte is a protein or hapten and the specific binding complement is an antibody comprising a monoclonal or polyclonal antibody. Alternatively, the target analyte is a sequence from a genomic DNA sample and the specific binding complements are oligonucleotides, the oligonucleotides having a sequence that is complementary to at least a portion of the genomic sequence. The genomic DNA may be eukaryotic, bacterial, fungal or viral DNA.

The specific binding complement and the target analyte are members of an antibody-ligand pair.

In addition to its first binding site, the target analyte has been modified to include a second binding site.

The methods may further comprise a filtration step to remove aggregate complexes, wherein the filtration is performed prior to analyzing the aggregated complex. The filtration step comprises a membrane that removes sample components that do not comprise DNA barcodes.

In another embodiment of the invention, a method is provided for detecting for the presence or absence of one or more target analytes in a sample, the method comprises:
providing at least one or more types of particle complex probes, each type of probe comprising oligonucleotides bound thereto, one or more specific binding complements of a specific target analyte, and one or more DNA barcodes that serves as a marker for the particular target analyte, wherein at least a portion of a sequence of the DNA barcodes is hybridized to at least some of the oligonucleotides bound to the nanoparticles;
contacting the sample with the particle complex probes under conditions effective to allow specific binding interactions between the target analytes and the particle complex probes and to form an aggregate complex in the presence of a target analyte; and observing whether aggregate complex formation occurred.

In this aspect of the invention, the observable signal can be detected using any method described herein. For example, direct detection of barcode DNAs, detection of *B*PCR-amplified barcode DNAs, detection of aggregation of the one or more types of detection probe (e.g., by visual inspection, fluorescence, colorimetric, electrochemistry, electronic, densitometry, radioactivity, and the like), or by using reporter nucleotides that comprise a sequence that is complementary to at least a portion of the barcode DNAs and a detectable signal moiety (e.g., fluorescent label).

If sufficient complex is present, the complex can be observed visually with or without a background substrate. Any substrate can be used which allows observation of the detectable change. Suitable substrates include transparent solid surfaces (*e.g*., glass, quartz, plastics and other polymers), opaque solid surface (*e.g*., white solid surfaces, such as TLC silica plates, filter paper, glass fiber filters, cellulose nitrate membranes, nylon membranes), and conducting solid surfaces (*e.g*., indium-tin-oxide (ITO)). The substrate can be any shape or thickness, but generally will be flat and thin. Preferred are transparent substrates such as glass (*e.g*., glass slides) or plastics (*e.g*., wells of microtiter plates).

In one aspect of the invention, a method for detecting for the presence of a target analyte, e.g., an antibody, in a sample is provided. An antibody such as immunoglobulin E (IgE) or immunoglobulin G1 (IgG1) shown in the Examples below can be detected with olignucleotide-modified probes prehybridized with oligonucleotide strands modified with the appropriate hapten (biotin in the case of IgG1 and dinitrophenyl (DNP) in the case of IgE; Figure 1A). ^{13, 14} The DNA sequences in the proof-of-concept assays presented in the Examples below were designed in a way that would ensure that the two different aggregates formed from the probe reactions with IgG1 and IgE would melt at different temperatures, Figure 1B. The probes for IgG1 have longer sequences and greater G,C base contents than those for IgE. Therefore, the former sequences melt at a higher temperature than the latter ones. These sequence variations allow one to prepare probes with distinct melting signatures that can be used as codes to identify which targets have reacted with them to form nanoparticle aggregates. Three different systems have been studied: (1) two probes with one target antibody present (IgG1 or IgE); (2) two probes with the two different target antibodies present, and (3) a control where no target antibodies are present.

In this aspect of the invention, a method is provided for detecting the presence of a target analyte, e.g., an antibody, in a sample comprises contacting a nanoparticle probe having oligonucleotides bound thereto with a sample which may contain a target analyte. At least some of the oligonucleotides attached to the nanoparticle are bound to a first portion of a reporter oligonucleotide as a result of hybridization. A second portion of the reporter oligonucleotide is bound, as a result of hybridization, to an oligonucleotide having bound thereto a specific binding complement (e.g., antigen) to the analyte. The contacting takes place under conditions effective to allow specific binding interactions between the analyte and the nanoparticle probe. In the presence of target analyte, nanoparticle aggregates are produced. These aggregates may be detected by any suitable means.

In another aspect of the invention, particle complex probes, preferably nanoparticle complex probes, are used. These particle complexes may be generated prior to conducting the actual assay or in situ while conducting the assay. These complexes comprise a particle, preferably a nanoparticle, having oligonucleotides bound thereto, a reporter oligonucleotide bound to at least a portion of the oligonucleotides bound to the nanoparticle, and a specific binding complement of the target analyte. The specific binding complement may be directly or indirectly bound to the nanoparticle. For instance, the specific binding complement can be bound to a linker or oligonucleotide and the labeled linker or oligonucleotide is then bound to the nanoparticle. In one embodiment, the DNA barcode or reporter oligonucleotides has a sequence having at least two portions and joins via hybridization the nanoparticle having oligonucleotides bound thereto and the oligonucleotide having bound thereto the specific binding complement. The oligonucleotides bound to the nanoparticles have a sequence that is complementary to one portion of the reporter oligonucleotide and the oligonucleotide having bound thereto the specific binding complement having a sequence that is complementary to a second portion of the reporter oligonucleotide. The reporter oligonucleotides have at least two portions and joins via hybridization the nanoparticle having oligonucleotides bound thereto and the oligonucleotide having bound thereto the specific binding complement. When employed in a sample containing the target analyte, the nanoparticle complex binds to the target analyte and aggregation occurs. The aggregates may be isolated and subject to further melting analysis to identify the particular target analyte where multiple targets are present as discussed above. Alternatively, the aggregates can be dehybridized to release the reporter oligonucleotides. These reporter oligonucleotides, or DNA barcode can optionally be amplified, and then be detected by any suitable DNA detection system using any suitable detection probes.

In practicing the invention, a nanoparticle complex probes are prepared by hybridizing the nanoparticles having oligonucleotides bound thereto with an oligonucleotide modified with a specific binding complement to a target analyte, and a reporter oligonucleotide. At least some of the oligonucleotides attached to the nanoparticle have a sequence that is complementary to a first portion of a reporter oligonucleotide. The oligonucleotides having bound thereto a specific binding complement have a sequence that is complementary to a second portion of a reporter oligonucleotide. The reporter oligonucleotide hybridizes to the at least some of the oligonucleotides attached to the nanoparticle and to the oligonucleotides having bound thereto the specific binding complement, forming the nanoparticle complex probe under conditions sufficient to allow for hybridization between the components. Any suitable solvent medium and hybridization conditions may be employed in preparing the nanoparticle complex solution that allows for sufficient hybridization of the components. Preferably, the components are hybridized in a phosphate buffered solution (PBS) comprised of 0.3 M NaCl and 10 mM phosphate buffer (pH 7) at room temperature for about 2-3 hours. The concentration of nanoparticle-oligonucleotide conjugates in the hybridization mixture range between about 2 nM and about 50 nM, preferably about 13 nM. The concentration of hapten-modified oligonucleotides generally ranges between about 50 and about 900, preferably about 300 nM. The concentration of reporter oligonucleotide generally ranges between about 50 and about 900, preferably about 300 nM. Unreacted hapten-modified oligonucleotide and reporter oligonucleotides may be optionally, but preferably, removed by any suitable means, preferably via centrifugation (12,000 rpm, 20 minutes) of the hybridization mixture and subsequent decanting of the supernatant. The prepared complexes were stored in 0.3 M NaCl and 10 mM phosphate buffer (pH 7-7.4), 0.01% azide solution at 4-6 °C.

A typical assay for detecting the presence of a target analyte, e.g, antibody, in a sample is as follows: a solution containing nanoparticle complex probe comprising nanoparticles having oligonucleotides bound thereto, a reporter oligonucleotide, and an oligonucleotide having a specific binding complement to the target analyte, is admixed with an aqueous sample solution believed to contain target protein. The total protein content in the aqueous sample solution generally ranges between about 5 and about 100, usually about 43 ug/ml. The concentration of nanoparticles in the reaction mixture generally ranges between about 2 nM and about 20 nM, usually about -13 nM. The total volume of the resulting mixture generally ranges between about 100 uL and about 1000 uL, preferably about 400 uL. Any suitable solvent may be employed in preparing the aqueous sample solution believed to contain target analyte, preferably PBS comprising 0.3 M NaCl and 10 mM phosphate buffer (pH 7-7.4).

The resulting assay mixture is then incubated at a temperature ranging between about 35 and about 40°C, preferably at 37 °C, for a time ranging between about 30 and about 60, preferably about 50 minutes, sufficient to facilitate specific binding pair, e.g., protein-hapten, complexation. If the target protein is present, particle aggregation takes place effecting a shift in the gold nanoparticle plasmon band and a red-to-purple color change along with precipitation. The hybridized products are centrifuged (e.g., 3000 rpm for 2 minutes), and the supernatant containing unreacted elements are decanted prior to analysis.

If desired, the nanoparticle complex probe may be prepared in situ within the assay mixture by admixing all the nanoparticles having oligonucleotides bound thereto, the reporter oligonucleotide, and the hapten-modified oligonucleotide with the sample suspected of containing a target analyte. To ensure complete hybridization among all the components, especially the complementary DNA strands, the assay mixture may be incubated to expedite hybridization at -15 °C for 20 minutes (Boekel Tropicooler Hot/Cold Block Incubator) and stored at 4 °C for 24 hours. In practicing the invention, however, it is preferred that the nanoparticle complex probe is prepared prior to conducting the assay reaction to increase the amount of DNA barcode within the nanoparticle complex probe.

To determine which proteins are present, a melting analysis of the aggregates which monitors the extinction at 260 nm as a function of temperature may carried out in the solution. See, for instance, Figure 2 in Example 3 which describes analysis of a sample containing one or two known target analytes: IgG1 and IgE. As discussed in Example 3, when IgG1 is treated with the probes via the aforementioned protocol, the solution turns pinkish-blue, indicating the formation of nanoparticle aggregates. In a control experiment where no target but background proteins are present, there is no discernible precipitation. A melting analysis of the solution shows a sharp transition with a melting temperature (Tm) of 55 °C. This is the expected transition for the IgG1 target, Figure 2A (---). If IgE is added to a fresh solution of probes, the same color change is observed but the melting analysis provides a curve with a Tm of 36 °C, the expected transition for this target, Figure 2A (—). Significantly, when both protein targets are added to the solution of probes, the solution turns dark purple, and the melting analysis exhibits two distinct transactions. The first derivative of this curve shows two peaks centered at 36 and 55 °C, respectively, Figure 2B. This demonstrates that two distinct assemblies form and their melting properties, which derive from the oligonucleotide barcodes, can be used to distinguish two protein targets.

In another aspect of the invention, a variation of the above aggregation method strategy can be used to increase the sensitivity of the aforementioned system and to increase the number of targets that can be interrogated in one solution. See, for instance, Figure 3 in Example 4. With this strategy, the protein targets can be detected indirectly via the DNA biobarcodes or unique reporter oligonucleotides assigned to specific target analytes. Generally, the suitable length, GC content, and sequence, and selection of the reporter oligonucleotide for the target analyte is predetermined prior to the assay. For instance, a 12-mer oligonucleotide has 4¹² different sequences, many of which can be used to prepare a barcode for a polyvalent protein of interest as shown in Figure 1A. In this variation of the assay, the melting properties of the aggregates that form are not measured in solution but rather the reporter oligonucleotides or DNA biobarcodes within the aggregates are separated via centrifugation (e.g., 3000 rpm for 2 minutes) from the unreacted probes and target molecules. The aggregates are then denatured by any suitable means, e.g., by adding water to the solution, to free the reporter oligonucleotides or biobarcodes. If the reporter oligonucleotide is present in small amounts, it may be amplified by methods known in the art. See, *e.g*., Sambrook et al., Molecular Cloning: A Laboratory Manual (2nd ed. 1989) and B.D. Hames and S.J. Higgins, Eds., Gene Probes 1 (IRL Press, New York, 1995). Preferred is polymerase chain reaction (PCR) amplification. The particles and proteins can be separated from the reporter oligonucleotides by any suitable means, e.g., a centrifugal filter device (Millipore Microcon YM-100, 3500 rpm for 25 min. Once the reporter oligonucleotides are isolated, they can be captured on an oligonucleotide array and can be identified using one of the many suitable DNA detection assays (Figure 3). For the examples described herein involving IgG1 and IgE, the reporter oligonucleotides are captured on a microscope slide that has been functionalized with oligonucleotides (250 µm diameter spots) that are complementary to one half of the barcode of interest (**A3** and **B3** in Figure 1). If the barcode is captured by the oligonucleotide array, a DNA-modified particle that is complementary to the remaining portion of the barcode can be hybridized to the array (see experimental section). When developed via the standard scanometric approach ^{[11]} (which involves treatment with photographic developing solution), a flat bed scanner can be used to quantify the results, Figure 4.¹¹ If IgG1 is present, only the spot designed for IgG1 shows measurable signal. Similarly if IgE is the only protein present, the spot designed for it only exhibits signal. Finally, if both proteins are present, both spots exhibit intense signals.

In one aspect of this embodiment, the DNA barcode in each type of particle complex probe has a sequence that is different and that serves as an identifier for a particular target analyte.

In another aspect of this embodiment, the method further comprises the steps of:
isolating aggregated complexes; and
analyzing the aggregated complexes to determine the presence of one or more DNA barcodes having different sequences.

In another aspect of this embodiment, the method further comprises the steps of:
isolating the aggregated complex;
subjecting the aggregated complex to conditions effective to dehybridize the aggregated complex and release the DNA barcode;
isolating the DNA barcode; and
detecting for the presence of one or more DNA barcodes having different sequences, wherein each DNA barcode is indicative of the presence of a specific target analyte in the sample.

In another aspect of this embodiment, the method further comprises the steps of:
isolating the aggregated complex;
subjecting the aggregated complex to conditions effective to dehybridize the aggregated complex and release the DNA barcode;
isolating the DNA barcode;
amplifying the isolated DNA barcode; and
detecting for the presence of one or more amplified DNA barcodes having different sequences, wherein each DNA barcode is indicative of the presence of a specific target analyte in the sample.

In another aspect of this embodiment, target has more than two binding sites and at least two types of particle complex probes are provided, the first type of probe having a specific binding complement to a first binding site on the target analyte and the second type of probe having a specific binding complement to a second binding site on the probe. A plurality of particle complex probes may be provided, each type of probe having a specific binding complement to different binding sites on the target analyte.

In another aspect of this embodiment, the specific binding complement and the target analyte are members of a specific binding pair comprising nucleic acid, oligonucleotide, peptide nucleic acid, polypeptide, antibody, antigen, carbohydrate, protein, peptide, amino acid, hormone, steroid, vitamin, drug, virus, polysaccharides, lipids, lipopolysaccharides, glycoproteins, lipoproteins, nucleoproteins, oligonucleotides, antibodies, immunoglobulins, albumin, hemoglobin, coagulation factors, peptide and protein hormones, non-peptide hormones, interleukins, interferons, cytokines, peptides comprising a tumor-specific epitope, cells, cell-surface molecules, microorganisms, fragments, portions, components or products of microorganisms, small organic molecules, nucleic acids and oligonucleotides, metabolites of or antibodies to any of the above substances.

The nucleic acid and oligonucleotide comprise genes, viral RNA and DNA, bacterial DNA, fungal DNA, mammalian DNA, cDNA, mRNA, RNA and DNA fragments, oligonucleotides, synthetic oligonucleotides, modified oligonucleotides, single-stranded and double-stranded nucleic acids, natural and synthetic nucleic acids, and aptamers.

In one aspect, the target analytes may be a nucleic acid and the specific binding complement may be an oligonucleotide. Alternatively, the target analyte may be a protein or hapten and the specific binding complement may be an antibody comprising a monoclonal or polyclonal antibody.

In another aspect of this embodiment, the target analyte may be a sequence from a genomic DNA sample and the specific binding complements are oligonucleotides, the oligonucleotides having a sequence that is complementary to at least a portion of the genomic sequence.

In another aspect of this embodiment, the genomic DNA may be eukaryotic, bacterial, fungal or viral DNA.

In another aspect, the specific binding complement and the target analyte are members of an antibody-ligand pair.

In another aspect of this embodiment, detecting step for the presence of one or more DNA barcodes comprises:
providing a substrate having oligonucleotides bound thereto, the oligonucleotides having a sequence complementary to at least a portion of the sequence of the DNA barcode;
providing a nanoparticle comprising oligonucleotides bound thereto, wherein at least portion of the oligonucleotides bound to the nanoparticles have a sequence that is complementary to at least a portion of a DNA barcode; and
contacting the DNA barcodes, the oligonucleotides bound to the substrate, and the nanoparticles under conditions effective to allow for hybridization at least a first portion of the DNA barcodes with a complementary oligonucleotide bound to the substrate and a second portion of the DNA barcodes with some of the oligonucleotides bound to the nanoparticles; and
observing a detectable change.

In another aspect of this embodiment, substrate comprises a plurality of types of oligonucleotides attached thereto in an array to allow for the detection of one or more different types of DNA barcodes.

In another aspect of this embodiment, the detectable change is the formation of dark areas on the substrate.

In another aspect of this embodiment, the detectable change is observed with an optical scanner.

In another aspect of this embodiment, the substrate is contacted with a silver stain to produce the detectable change.

In another aspect of this embodiment, the DNA barcodes are contacted with the substrate under conditions effective to allow the DNA barcodes to hybridize with complementary oligonucleotides bound to the substrate and subsequently contacting the DNA barcodes bound to the substrate with the nanoparticles having oligonucleotides bound thereto under conditions effective to allow at least some of the oligonucleotides bound to the nanoparticles to hybridize with a portion of the sequence of the DNA barcodes on the substrate.

In another aspect of this embodiment, the DNA barcodes are contacted with the nanoparticles having oligonucleotides bound thereto under conditions effective to allow the DNA barcodes to hybridize with at least some of the oligonucleotides bound to the nanoparticles; and subsequently contacting the DNA barcodes bound to the nanoparticles with the substrate under conditions effective to allow at least a portion of the sequence of the DNA barcodes bound to the nanoparticles to hybridize with complementary oligonucleotides bound to the substrate.

In another aspect of this embodiment, the DNA barcode is amplified prior to the contacting step.

In another aspect of this embodiment, at least two types of particle complex probes are provided, a first type of probe having a specific binding complement to a first binding site of the target analyte and a second type of probe having a specific binding complement to a second binding site of the target analyte.

As discussedf above, the nucleic acid and oligonucleotide comprise genes, viral RNA and DNA, bacterial DNA, fungal DNA, mammalian DNA, cDNA, mRNA, RNA and DNA fragments, oligonucleotides, synthetic oligonucleotides, modified oligonucleotides, single-stranded and double-stranded nucleic acids, natural and synthetic nucleic acids, and aptamers.

In another embodiment of the invention, kits are provided which comprise the particle complex probe described above.

In another aspect of this embodiment, a kit is provided for detecting for the presence or absence of one or more target analytes in a sample, each target analyte having at least two binding sites. The kit comprising:
at least one type of detection probe for each target analyte, each type of detection probe comprising (i) a nanoparticle; (ii) a member of a specific binding pair bound to the nanoparticle; (iii) oligonucleotides bound to the nanoparticle; and (iv) a DNA barcode having a predetermined sequence that is complementary to a least a portion of the oligonucleotides.

In another aspect of this embodiment, the kit comprises:
at least one type of capture probe comprising (i) a substrate; (ii) a first member of a first specific binding pair attached to the substrate, wherein the first member of the fist specific binding pair binds to a first binding site of the target analyte;
at least one type of detection probe comprising (i) a nanoparticle; (ii) a first member of a second specific binding pair attached to the nanoparticle, wherein the first member of the second specific binding pair binds to a second binding site of the target analyte; (iii) at least one type of oligonucleotides bound to the nanoparticle; and (iv) at least one type of DNA barcodes, each type having a predetermined sequence that is complementary to at least a portion of a specific type of oligonucleotides.

Any suitable substrate can be used in the kit. Preferably, the substrate is magnetic such as a magnetic microparticle.

In another aspect of this embodiment, the kit comprises:
at least one type of capture probe comprising (i) a magnetic particle; (ii) a first member of a first specific binding pair attached to the magnetic particle, wherein the first member of the first specific binding pair binds to a first binding site of the target analyte;
at least one type of detection probe comprising (i) a nanoparticle; (ii) a first member of a second specific binding pair attached to the nanoparticle, wherein the first member of the second specific binding pair binds to a second binding site of the target analyte; (iii) at least one type of oligonucleotides bound to the nanoparticle; and (iv) at least one type of DNA barcodes, each type having a predetermined sequence that is complementary to at least a portion of a specific type of oligonucleotides.

In another aspect of this embodiment, the kit comprises:
at least one container including particle complex probes comprising a particle having oligonucleotides bound thereto, a DNA barcode, and an oligonucleotide having bound thereto a specific binding complement to a target analyte, wherein the DNA barcode has a sequence having at least two portions, at least some of the oligonucleotides attached to the particle have a sequence that is complementary to a first portion of a DNA barcode, the oligonucleotides having bound thereto a specific binding complement have a sequence that is complementary to a second portion of a DNA barcode, and wherein the DNA barcode is hybridized to at least to some of the oligonucleotides attached to the particle and to the oligonucleotides having bound thereto the specific binding complement, and an optional substrate for observing a detectable change.

In another aspect of this embodiment, the kit comprises:
at least one or more containers, container holds a type of particle complex probe comprising a particle having oligonucleotides bound thereto, a DNA barcode, and an oligonucleotide having bound thereto a specific binding complement to a specific target analyte, wherein (i) the DNA barcode has a sequence having at least two portions, (ii) at least some of the oligonucleotides attached to the particle have a sequence that is complementary to a first portion of a DNA barcode, (iii) the oligonucleotides having bound thereto a specific binding complement have a sequence that is complementary to a second portion of a DNA barcode, and (iv) the DNA barcode in each type of particle complex probe has a sequence that is different and that serves as an identifier for a particular target analyte; wherein the kit optionally includes a substrate for observing a detectable change.

In another aspect of this embodiment, the kit comprises:
at least one pair of containers and an optional substrate for observing a detectable change,
the first container of the pair includes particle probe comprising a particle having oligonucleotides bound thereto and a DNA barcode having a sequence of at least two portions, wherein at least some of the oligonucleotides attached to the particle have a sequence that is complementary to a first portion of a DNA barcode;
the second container of the pair includes an oligonucleotide having a sequence that is complementary to a second portion of the DNA barcode, the oligonucleotide having a moiety that can be used to covalently link a specific binding pair complement of a target analyte.

In another aspect of this embodiment, the kit comprises:
at least two or more pairs of containers,
the first container of each pair includes particle complex probes having particles having oligonucleotides bound thereto and a DNA barcode having a sequence of at least two portions, wherein at least some of the oligonucleotides bound to the particles have a sequence that is complementary to a first portion of a DNA barcode having at least two portions; and
the second container of each pair contains an oligonucleotide having a sequence that is complementary to a second portion of the DNA barcode, the oligonucleotide having a moiety that can be used to covalently link a specific binding pair complement of a target analyte,
wherein the DNA barcode for type of particle complex probe has a sequence that is different and that serves as an identifier for a target analyte and wherein the kit optionally include a substrate for observing a detectable change.

In another aspect of this embodiment, the kit comprises:
a first container and at least two or more pairs of containers,
the first container includes particle complex probes having particles having oligonucleotides bound thereto;
the first container of the pair includes a DNA barcode having a sequence of at least two portions, wherein at least some of the oligonucleotides bound to the particles have a sequence that is complementary to a first portion of the DNA barcode; and
the second container of each pair contains an oligonucleotide having a sequence that is complementary to a second portion of the DNA barcode, the oligonucleotide having a moiety that can be used to covalently link a specific binding pair complement of a target analyte,
wherein the DNA barcode present in the first container of each pair of containers serves as an identifier for a target analyte and has a sequence that is different from a DNA barcode in another pair of containers, and wherein the kite optionally include a substrate for observing a detectable change.

In another aspect of this embodiment, the kit comprises:
an oligonucleotide sequence that serves as an identifier for the presence of a specific target analyte.

The above kits can include instructions for assembling the assay and for conducting the assay.

It is to be noted that the term "a" or "an" entity refers to one or more of that entity. For example, "a characteristic" refers to one or more characteristics or at least one characteristic. As such, the terms "a" (or "an"), "one or more" and "at least one" are used interchangeably herein. It is also to be noted that the terms "comprising", "including", and "having" have been used interchangeably. The following examples are intended for illustration purposes only, and should not be construed as limiting the spirit or scope of the invention in any way.

### EXAMPLES

### Example 1: Preparation of Oligonucleotide-Modified Gold Nanoparticles

### A. Preparation Of Gold Nanoparticles

Oligonucleotide-modified 13 nm Au particles were prepared by literature methods (~110 oligonucleotides/particle)¹⁸⁻²⁰. Gold colloids (13 nm diameter) were prepared by reduction of HAuCl₄ with citrate as described in Frens, Nature Phys. Sci., 241, 20 (1973) and Grabar, Anal. Chem., 67, 735 (1995). Briefly, all glassware was cleaned in aqua regia (3 parts HCl, 1 part HNO₃), rinsed with Nanopure H₂O, then oven dried prior to use. HAuCl₄ and sodium citrate were purchased from Aldrich Chemical Company. An aqueous solution of HAuCl₄ (1 mM, 500 mL) was brought to a reflux while stirring, and then 50 mL of a 38.8 mM trisodium citrate solution was added quickly, which resulted in a change in solution color from pale yellow to deep red. After the color change, the solution was refluxed for an additional fifteen minutes, allowed to cool to room temperature, and subsequently filtered through a Micron Separations Inc. 0.45 micron nylon filter. Au colloids were characterized by UV-vis spectroscopy using a Hewlett Packard 8452A diode array spectrophotometer and by Transmission Electron Microscopy (TEM) using a Hitachi 8100 transmission electron microscope. A typical solution of 13 nm diameter gold particles exhibited a characteristic surface plasmon band centered at 518 - 520 nm. Gold particles with diameters of 13 nm will produce a visible color change when aggregated with target and probe oligonucleotide sequences in the 10-72 nucleotide range.

### B. Synthesis Of Oligonucleotides

Oligonucleotides were synthesized on a 1 micromole scale using a Milligene Expedite DNA synthesizer in single column mode using phosphoramidite chemistry. Eckstein, F. (ed.) Oligonucleotides and Analogues: A Practical Approach (IRL Press, Oxford, 1991). All solutions were purchased from Milligene (DNA synthesis grade). Average coupling efficiency varied from 98 to 99.8%, and the final dimethoxytrityl (DMT) protecting group was not cleaved from the oligonucleotides to aid in purification.

For 3'-thiol-oligonucleotides, Thiol-Modifier C3 S-S CPG support was purchased from Glen Research and used in the automated synthesizer. The final dimethoxytrityl (DMT) protecting group was not removed to aid in purification. After synthesis, the supported oligonucleotide was placed in 1 mL of concentrated ammonium hydroxide for 16 hours at 55 °C to cleave the oligonucleotide from the solid support and remove the protecting groups from the bases.

After evaporation of the ammonia, the oligonucleotides were purified by preparative reverse-phase HPLC using an HP ODS Hypersil column (5 um, 250 x 4 mm) with 0.03 M triethyl ammonium acetate (TEAA), pH 7 and a 1%/minute gradient of 95% CH₃CN/5% 0.03 M TEAA at a flow rate of 1 mL/minute, while monitoring the UV signal of DNA at 254 nm. The retention time of the DMT protected modified 12-base oligomer was 30 minutes. The DMT was subsequently cleaved by soaking the purified oligonucleotide in an 80 % acetic acid solution for 30 minutes, followed by evaporation; the oligonucleotide was redispersed in 500 uL of water, and the solution was extracted with ethyl acetate (3 x 300 uL). After evaporation of the solvent, the oligonucleotide (10 OD's) was redispersed in 100 uL of a 0.04 M DTT, 0.17 M phosphate buffer (pH 8) solution overnight at 50 °C to cleave the 3' disulfide. Aliquots of this solution (< 10 OD's) were purified through a desalting NAP-5 column. The amount of oligonucleotide was determined by absorbance at 260 nm. Purity was assessed by ion-exchange HPLC using a Dionex Nucleopac PA-100 column (250 x 4 mm) with 10 mM NaOH (pH 12) and a 2%/minute gradient of 10 mM NaOH, 1 M NaCl at a flow rate of 1 mL/minute while monitoring the UV signal of DNA at 254 nm. Three peaks with retention times (Tᵣ) of 18.5, 18.9 and 22 minutes were observed. The main single peak at Tᵣ = 22.0 minutes, which has been attributed to the disulfide, was 79 % by area. The two peaks with shorter retention times of 18.5 and 18.9 minutes were ∼9 % and 12 % by area respectively, and have been attributed to oxidized impurity and residual thiol oligonucleotide.

5'-Alkylthiol modified oligonucleotides were prepared using the following protocol: 1) a CPG-bound, detritylated oligonucleotide was synthesized on an automated DNA synthesizer (Expedite) using standard procedures; 2) the CPG-cartridge was removed and disposable syringes were attached to the ends; 3) 200 uL of a solution containing 20 umole of 5-Thiol-Modifier C6-phosphoramidite (Glen Research) in dry acetonitrile was mixed with 200 uL of standard "tetrazole activator solution" and, *via* one of the syringes, introduced into the cartridge containing the oligonucleotide-CPG; 4) the solution was slowly pumped back and forth through the cartridge for 10 minutes and then ejected followed by washing with dry acetonitrile (2 x 1 mL); 5) the intermediate phosphite was oxidized with 700 uL of 0.02 M iodine in THF/pyridine/water (30 seconds) followed by washing with acetonitrile/pyridine (1:1; 2 x 1 mL) and dry acetonitirile. The trityloligonucleotide derivative was then isolated and purified as described by the 3'-alkylthiol oligonucleotides; then the trityl protecting group was cleaved by adding 15 uL (for 10 OD's) of a 50 mM AgNO₃ solution to the dry oligonucleotide sample for 20 minutes, which resulted in a milky white suspension. The excess silver nitrate was removed by adding 20 uL of a 10 mg/mL solution of DTT (five minute reaction time), which immediately formed a yellow precipitate that was removed by centrifugation. Aliquots of the oligonucleotide solution (< 10 OD's) were then transferred onto a desalting NAP-5 column for purification. The final amount and the purity of the resulting 5' alkylthiol oligonucleotides were assessed using the techniques described above for 3' alkylthiol oligonucleotides. Two major peaks were observed by ion-exchange HPLC with retention times of 19.8 minutes (thiol peak, 16 % by area) and 23.5 minutes (disulfide peak, 82 % by area).

### C. Attachment Of Oligonucleotides To Gold Nanoparticles

A 1 mL solution of the gold colloids (17nM) in water was mixed with excess (3.68 uM) thiol-oligonucleotide (22 bases in length) in water, and the mixture was allowed to stand for 12-24 hours at room temperature. Then, the solution was brought to 0.1 M NaCl, 10 mM phosphate buffer (pH 7) and allowed to stand for 40 hours. This "aging" step was designed to increase the surface coverage by the thiol-oligonucleotides and to displace oligonucleotide bases from the gold surface. The solution was next centrifuged at 14,000 rpm in an Eppendorf Centrifuge 5414 for about 25 minutes to give a very pale pink supernatant containing most of the oligonucleotide (as indicated by the absorbance at 260 nm) along with 7-10% of the colloidal gold (as indicated by the absorbance at 520 nm), and a compact, dark, gelatinous residue at the bottom of the tube. The supernatant was removed, and the residue was resuspended in about 200 µL of buffer (10 mM phosphate, 0.1 M NaCl) and recentrifuged. After removal of the supernatant solution, the residue was taken up in 1.0 mL of buffer (10 mM phosphate, 0.3 M NaCl, 0.01 % NaN₃). The resulting red master solution was stable (*i.e*., remained red and did not aggregate) on standing for months at room temperature, on spotting on silica thin-layer chromatography (TLC) plates (see Example 4), and on addition to 1 M NaCl, 10 mM MgCl₂, or solutions containing high concentrations of salmon sperm DNA.

### Example 2: Preparation of Hapten-modified oligonucleotides

Hapten-modified oligonucleotides were prepared with a biotin-triethylene glycol phosphoramidite for **A1** and 2, 4-dinitrophenyl-triethylene glycol phosphoramidite for **B1** (Glen research) using standard solid-phase DNA synthesis procedures.²¹

Biotin modified oligonucleotides were prepared using the following protocol: A CPG-bound, detritylated oligonucleotide was synthesized on an automated DNA synthesizer (Expedite) using standard procedures²¹. The CPG-cartridge was then removed and disposable syringes were attached to the ends. 200 uL of a solution containing 20 umole of biotin-triethylene glycol phosphoramidite in dry acetonitrile was then mixed with 200 uL of standard "tetrazole activator solution" and, *via* one of the syringes, introduced into the cartridge containing the oligonucleotide-CPG. The solution then was slowly pumped back and forth through the cartridge for 10 minutes and then ejected followed by washing with dry acetonitrile (2 x 1 mL). Thereafter, the intermediate phosphite was oxidized with 700 uL of 0.02 M iodine in THF/pyridine/ water (30 seconds) followed by washing with acetonitrile/pyridine (1:1; 2 x 1 mL) and dry acetonitirile with subsequent drying of the column with a stream of nitrogen. The trityl protecting group was not removed, which aids in purification. The supported oligonucleotide was placed in 1 mL of concentrated ammonium hydroxide for 16 hours at 55 °C to cleave the oligonucleotide from the solid support and remove the protecting groups from the bases. After evaporation of the ammonia, the oligonucleotides were purified by preparative reverse-phase HPLC using an HP ODS Hypersil column (5 um, 250 x 4 mm) with 0.03 M triethyl ammonium acetate (TEAA), pH 7 and a 1%/minute gradient of 95% CH₃CN/5% 0.03 M TEAA at a flow rate of 1 mL/minute, while monitoring the UV signal of DNA at 254 nm. The retention time of the DMT protected oligonucleotides was ~32 minutes. The DMT was subsequently cleaved by soaking the purified oligonucleotide in an 80% acetic acid solution for 30 minutes, followed by evaporation; the oligonucleotide was redispersed in 500 uL of water, and the solution was extracted with ethyl acetate (3 x 300 uL) and dried. The same protocol was used to synthesize DNP modified oligonucleotide using 2, 4-dinitrophenyl-triethylene glycol phosphoramidite.

### Example 3: Assay Using Nanoparticle Complex Probes

The Oligonucleotide-modified 13 nm gold particles were prepared as described in Example 1. Hapten-modified oligonucleotides were prepared as described in Example 2 with a biotin-triethylene glycol phosphoramidite for **A1** and 2, 4-dinitrophenyl-triethylene glycol phosphoramidite for **B1** (Glen research) using standard solid-phase DNA synthesis procedures.²¹ The PBS buffer solution used in this research consists of 0.3 M NaCl and 10 mM phosphate buffer (pH 7). IgE and IgG1 were purchased from Sigma Aldrich (Milwaukee, WI) and dissolved in 0.3 M PBS buffer with 0.05% Tween 20 (final concentration: 4.3x10⁻⁸ b/µl) and background proteins (10 ug/ml of lysozyme, 1% bovine serum albumin, and 5.3 ug/ml of anti-digoxin; 10 uL of each) prior to use.

To prepare the probes, the oligonucleotide modified particles (13 nM, 300 µL) were hybridized with hapten-modified complementary oligonucleotides (10 µL of 10 µM) and biobarcode DNA (10 µL of 10 µM) at room temperature for 2-3 h, sequences given in Figure 1. Unreacted hapten-modified oligonucleotide and biobarcodes were removed via centrifugation (12,000 rpm, 20 min) of the nanoparticle probes and subsequent decanting of the supernatant.

In a typical assay for IgE and/or IgG1, the target proteins (40 µl of 43 µg/ml for each) were added to the solution containing the probes (~13 nM), and the mixture was incubated at 37 °C for 50 minutes to facilitate protein-hapten complexation. To ensure complete reaction among all the components, especially the complementary DNA strands, the solution was incubated to expedite hybridization at -15 °C for 20 minutes (Boekel Tropicooler Hot/Cold Block Incubator) and stored at 4 °C for 24 hours. If the target protein is present, particle aggregation takes place affecting a shift in the gold nanoparticle plasmon band and a red-to-purple color change along with precipitation. The hybridized products were centrifuged (3000 rpm for 2 minutes), and the supernatant containing unreacted elements was decanted prior to analysis. To determine which proteins are present, a melting analysis, which monitors the extinction at 260 nm as a function of temperature is carried out in the solution, Figure 2. When IgG1 is treated with the probes via the aforementioned protocol, the solution turns pinkish-blue, indicating the formation of nanoparticle aggregates. In a control experiment where no target but background proteins are present, there is no discernible precipitation. A melting analysis of the solution shows a sharp transition with a melting temperature (Tm) of 55 °C. This is the expected transition for the IgG1 target, Figure 2A (---). If IgE is added to a fresh solution of probes, the same color change is observed but the melting analysis provides a curve with a Tm of 36 °C, the expected transition for this target, Figure 2A (—). Significantly, when both protein targets are added to the solution of probes, the solution turns dark purple, and the melting analysis exhibits two distinct transactions. The first derivative of this curve shows two peaks centered at 36 and 55 °C, respectively, Figure 2B. This demonstrates that two distinct assemblies form and their melting properties, which derive from the oligonucleotide barcodes, can be used to distinguish two protein targets.

### Example 4: Assay Using Nanoparticle Complex Probes

A variation of this strategy can be used to increase the sensitivity of the aforementioned system and to increase the number of targets that can be interrogated in one solution (Figure 3). With this strategy, the protein targets can be detected indirectly via the DNA biobarcodes. A 12-mer oligonucleotide has 4¹² different sequences, many of which can be used to prepare a barcode for a polyvalent protein of interest via Figure 1A. In this variation of the assay, the melting properties of the aggregates that form are not measured in solution but rather the DNA biobarcodes within the aggregates are separated via centrifugation (3000 rpm for 2 minutes) from the unreacted probes and target molecules. The aggregates are then denatured by adding water to the solution, freeing the complexed DNA. The particles and proteins can be separated from the DNA barcodes with a centrifugal filter device (Millipore Microcon YM-100, 3500 rpm for 25 min). Once the DNA barcodes are isolated, they can be captured on an oligonucleotide array and can be identified using one of the many DNA detection assays (Figure 3). For the examples described herein involving IgG1 and IgE, the barcodes are captured on a microscope slide that has been functionalized with oligonucleotides (250 µm diameter spots) that are complementary to one half of the barcode of interest (**A3** and **B3** in Figure 1). If the barcode is captured by the oligonucleotide array, a DNA-modified particle that is complementary to the remaining portion of the barcode can be hybridized to the array (see experimental section). When developed via the standard scanometric approach ^{[11]} (which involves treatment with photographic developing solution), a flat bed scanner can be used to quantify the results, Figure 4.¹¹ If IgG1 is present, only the spot designed for IgG1 shows measurable signal. Similarly if IgE is the only protein present, the spot designed for it only exhibits signal. Finally, if both proteins are present, both spots exhibit intense signals.

For scanometric DNA biobarcode detection, the DNA/Au nanoparticle assembly was centrifuged (3000 rpm for 2 min) in a polystyrene 1.5 mL vial, and the supernatant was removed. PBS buffer solution (700 µl) was added to the aggregate and the procedure was repeated to ensure isolation of the aggregate from unreacted protein and assay components. Then, 500 µl of water was added to the vial containing the aggregate to denature it. Microarrays were prepared and DNA hybridization methods were used according to literature methods.^{11, 22} The isolated DNA biobarcodes were premixed with **A2**-modified nanoparticles or **B2**-modified nanoparticles (2nM), exposed to the DNA microarray, and incubated in a hybridization chamber (GRACE BIO-LABS) at room temperature for three hours. The array was then washed with 0.3M NaNO₃ and 10nM NaH₂PO₄/Na₂HPO₄ buffer (pH 7) and submerged in Silver Enhancer Solution (Sigma) for three minutes at room temperature. The slide was washed with water and then analyzed with a flat bed scanner.

### Example 5: Assay using nanoparticle complex probe

The Biobarcode PCR (*B*PCR) protocol was performed to detect a protein target, free prostate specific antigen (PSA), at 3 aM sensitivity (Figure 6), which is six orders of magnitude more sensitive than the current conventional clinical assay for detecting PSA (46). The nanoparticle detection probes were prepared by adding polyclonal anti-PSA antibody (7 µg) to an aqueous solution of 13 nm Au nanoparticles (10 ml of 13 nM solution in colloidal suspension, citrate (~38 mM) stabilized) at pH 9.0. After 20 minutes, the anti-PSA modified nanoparticles were reacted with alkylthiol-capped barcode DNA capture strands (0.2 OD; 5' CAA CTT CAT CCA CGT TCA ACG CTA GTG AAC ACA GTT GTG T-A₁₀-SH 3' SEQ ID NO:9) for 12 hours and then salt-stabilized to 0.1 M NaCl/0.01 M phosphate buffer, pH 7. Next, the solution was treated with 1 ml of a 10 % BSA solution for 20 minutes to passivate and stabilize the gold nanoparticles. The final solution was centrifuged for 1 hour at 4 °C (20,000g), and the supernatant was removed. This centrifugation procedure was repeated for further purification. The PSA-specific barcode DNA strands (1 OD; 5' ACA CAA CTG TGT TCA CTA GCG TTG AAC GTG GAT GAA GTT G 3' SEQ ID NO:10) were then hybridized with the barcode DNA capture strands coordinated to the nanoparticles and purified using a similar centrifugation procedure.

Amino-functionalized 1 µm diameter magnetic particles were obtained from Polysciences, Inc. They were then linked to proteins using the commercial glutaraldehyde-amine coupling chemistry. Coupling efficiency was determined to be 90 % by UV-vis spectroscopy by comparing the absorbance at 270 nm before and after protein coupling to the particles. The particles, the magnetic capture probes, were suspended in 40 ml of 0.1 M PBS buffer (pH 7.4) prior to use.

In a typical PSA detection experiment (Figure 6B), an aqueous dispersion of magnetic capture probes functionalized with monoclonal anti-PSA antibodies (50 µl of 3 mg/ml magnetic probe solution) is mixed with an aqueous solution (0.1 M PBS) of free PSA (10 µl of PSA) and stirred at 37 °C for 30 minutes (Step 1 of Figure 6B). PSA bound magnetic detection probe can be easily separated from unbound PSA by applying a magnetic field. To effect magnetic separation, a 1.5 ml tube containing the assay solution is placed in a BioMag^{®} microcentrifuge tube separator (Polysciences, Inc.) at room temperature. After 15 seconds, the magnetic capture probe-PSA hybrids are concentrated on the wall of the tube. The supernatant (solution of unbound PSA molecules) is removed, and the magnetic capture probes, now in the form of a pellet on the side of the tube, are re-suspended in 50 µl of 0.1 M PBS (repeated 2X). Nanoparticle detection probes (50 µl at 1 nM), functionalized with polyclonal anti-PSA antibodies and hybrid barcode DNA strands, are then added. The detection probes react with the PSA immobilized on the capture probes and provide DNA strands for signal amplification and protein identification (Step 2 of Figure 6B). The solution is vigorously stirred at 37 °C for 30 minutes. The magnetic particles were then washed with 0.3 M PBS using the magnetic separator to isolate the magnetic particles. This step is repeated 4 times, each time requiring one minute, removing everything but the magnetic capture probes (along with PSA bound nanoparticle detection probes). After the final wash step, the magnetic capture probes are resuspended in Nanopure (18 M Ohm) water (50 µl) to dehybridize barcode DNA strands from the nanoparticle detection probe surface for 2 minutes. Dehybridized barcode DNA is then easily separated and collected from the probes using the magnetic separator (Step 3 of Figure 6B).

For barcode DNA amplification (Step 4, figure 6B), isolated barcode DNA is added to a PCR reaction mixture (20 µl, final volume) containing the appropriate primers, and the solution is then thermally cycled according to the following procedure. An aliquot of free barcode DNA (8.9µl) is added to the top wax layer of an EasyStaft^{™} Micro 20 PCR Mix-in-a-Tube (Molecular Bio-Products, San Diego, CA) along with 0.3 µl of the appropriate primers (each at 25 µM, Primer 1: 5'CAA CTT CAT CCA CGT TCA AC 3' SEQ ID NO:11, primer 2: 5'ACA CAA CTG TGT TCA CTA GC 3' SEQ ID NO:12), 0.4 µl DMSO (2 % final concentration), and 0.1 µL of Taq DNA polymerase, a polymerase shown to be compatible with the EasyStart^{™} system (5U/µl, Fisher Scientific), for a final volume of 20 µl. The final concentrations of the PCR reaction mix are as follows: Primers 1 and 2, 0.37 µM; dNTP mix, 0.2 mM; PCR buffer, 1X; and MgCl₂, 2 mM. The PCR tubes are then loaded into a thermal cycler (GeneAmp 9700, Applied Biosystems) and subjected to a 7 minute "hot start" at 94 °C, cycled 25 times at 94 °C for 30 seconds, 58 °C for 30 seconds and 72 °C for 1 minute, with a final extension of 72 °C for seven minutes followed by a 4 °C soak.

Control experiments were first performed to assess primer dimer formation after PCR amplification. Dimethyl sulfoxide (DMSO) was added to reduce the melting temperature of spurious hybridized primers and minimize the possibility of primer dimmer formation and amplification (Figure 7, 0 to 2 % from left to right in 0.5 % increments in lanes 1-5, and lanes 6-10). In addition, the number of thermal cycles was set at 25. As seen in Figure 7, there are clear bands with barcode DNA amplicon (lanes 1-5), while there are no observable bands when only primers are thermally cycled in the presence of Taq polymerase (lanes 6-10). Therefore, 2 % DMSO was added to all BPCR reactions since there is no observable band trace for that concentration (Figure 7, lane 10) while amplification was maintained for barcode DNA.

The barcode DNA amplicon was stained with ethidium bromide and mixed with gel loading dye. Gel electrophoresis was then performed to determine whether amplification has taken place (Figure 8, panels A and C). For all electrophoresis experiments, an aliquot (15 µl) of the PCR mixture is stained with ethidium bromide (1 mg), mixed with gel electrophoresis loading dye (3 µl, 6X, Promega, Madison, WI), and gel electrophoresis was performed (2 % agarose gel, 110 V, 35 minutes) in 1X TAB running buffer. A biobarcode standard (1 µl, 6 µM biobarcode duplex) was added to the gel for reference. The biobarcode standard (40-mer) was made by adding the biobarcode DNA to its complementary strand in 0.3 M PBS. All gel images and determinations of band intensities were done using a Kodak DC-120 digital camera and Kodak ID 2.0.2 imaging software. Gel bands were also stained with ethidium bromide after electrophoresis by soaking the gel in ethidium bromide for 35 minutes (0.5 µg/ml in 1X TAE running buffer) and qualitatively similar results to those where ethidium bromide was added to the PCR reaction prior to electrophoresis were obtained.

The relative intensity of the ethidium bromide stained bands allowed for an estimate of the relative concentration of PSA (Figure 8, panels B and D). The stained intensity of PCR amplicons represents PSA concentrations in lanes 3-8 of 300 aM, 3 fM, 30 fM, 300 fM, 3 pM, and 30 pM, respectively. The non-specific binding of the nanoparticle probe to the magnetic probe was negligible, as *B*PCR generated little signal when PSA was absent (Figure 8, panel A, lanes 1 and 2, and panel C, lane 1) fording to panel B, the intensities for control bands are at least 8 times lower than the bands with PSA present. In the graph representing low concentration (Figure 8, panel B, 3 aM to 300 fM, lanes 2-7, respectively) PSA detection, the gel band corresponding to 3 aM (lane 2) has a relative intensity 2.5 times higher than the negative control (lane 1).

### Example 6: Assay using nanoparticle complex probe

Although gel electrophoresis was routinely used to analyze the results of the assay, in general, the scanometric method provides higher sensitivity and is easier to implement than the gel-based method. Therefore, the results of the scanometric assay are reported herein. Chip-immobilized DNA 20-mers, which are complementary with half of the target barcode sequence (40-mer), were used to capture the amplified barcode DNA sequences, and gold nanoparticles were used to label the other half of the sequence in a sandwich assay format. The amplified duplex barcode DNA must be first denatured in order to effect hybridization between the barcode DNA, the chip surface, and gold nanoparticle probes. Thus, the barcode DNA amplicons were removed from the original PCR tube and added (5 µl) to a solution of gold nanoparticle probes (5 µl, 10 nM in 0.3 M PBS). The solution was diluted with 0.3 M PBS (90 µl) to a final volume of 100 µl in a clean 0.2 ml PCR tube. In order to hybridize barcode DNA single strands (40-mer) and nanoparticle bound complements (20-mer), the PCR tubes were added to a thermal cycler, heated to 95 °C for 3 minutes to denature the barcode DNA duplexes, and then cooled to a hybridization temperature of 45 °C for 2 minutes to bind nanoparticle probes to their complementary barcode DNA sequences. This mixture was removed from the PCR tube and added to the microarrayed (GMC 417 Arrayer, Genetic MicroSystems) chip with immobilized capture strands (20-mer). The test solution for each experiment was confined over the active region of the array with a 100 µl hybridization well (Grace BioLabs, Bend, OR) for 45 minutes in a humidity chamber. After hybridization, the chips were rinsed with 0.1 M NaNO₃/0.01 M phosphate buffer, pH 7.0 at 45 °C to remove excess gold particles (repeated 2X). Chips with hybridized nanoparticle probes were then subjected to silver amplification with silver enhancement solution (6 minute reaction time, Ted Pella Inc., Redding, CA), rinsed with Nanopure (18 M Ohm) water, and dried using a benchtop centrifuge. Gold nanoparticle binding followed by silver amplification results in gray spots that can be read with a Verigene ID system (Nanosphere, Inc.) that measures light scattered from the developed spots ^{11, 51}. Target PSA concentrations from 300 fM to 3 aM could be easily detected via this method (Figure 9). The 3 aM sample correlates with eighteen protein molecules in the entire sample. The selectivity for the barcode DNA sequence is excellent as evidenced by the lack of signal from the control spots with noncomplementary capture DNA (5'SH-C6-A10-GGCAGCTCGTGGTGA-3', SEQ ID NO:13) (Figure 9, Spotting template), and the observation that there is little discernible signal when PSA is absent (Figure 9, control).

### Example 7: Theoretical limit of protein detection using BPCR

To examiner the theoretical lower-limit of protein detection using *B*PCR, PCR/gel electrophoresis was performed with a dilution series of barcode DNA concentrations for PCR amplification (Figure 10). The signal when barcode DNA amplicons were present is quite discernible from the control band (lane 10) even when only 30 copies of barcode DNA were added to the PCR reaction (lane 9). Assuming each nanoparticle probe has about 50 barcode DNA strands, *B*PCR can, in theory, generate a detection signal with a single bound nanoparticle probe.

### Example 8: Detection of PSA in complex medium

To demonstrate the applicability of the *B*PCR amplification method in a sample solution more comparable to a clinical setting, the assays as described in Examples 5 and 6 were performed by dissolving the PSA target in goat serum, and the barcodes detected following a *B*PCR amplification step. PSA was successively diluted in 0.1 M PBS and then added to un-diluted goat serum (ICN Biomedicals, Inc., Aurora, Ohio). The goat serum effectively mimics 1X normal saline buffer, (e.g., any biological system, as far as ionic strength and pH).

The data demonstrates that in a complex medium the methods of the invention can detect target analytes (here PSA) at concentrations as low as 30 aM. The signal generated at this concentration is clearly discernible from control experiments (Fig. 11). Background signal can be reduced by introducing an optional step wherein the barcode DNA sample is filtered with a membrane that can remove a majority of contaminating components. This optional filtration step can separate barcode DNA from impurities by any known method, such as, for example, size (molecular weight), shape, charge, or hydrophobicity/hydrophilicity.

### Example 9: Direct detection and measurement of barcode DNA (non-BPCR amplified)

The gold nanoparticle (NP) probes were prepared essentially as described above in Example 5. Briefly, polyclonal antibodies (Abs) to PSA (3.5 µg) were added to an aqueous solution of 30 nm gold NPs (1 ml of 40 pM NP solution) at pH 9.0. After 20 minutes, the Ab modified NPs were reacted with alkylthiol-capped barcode DNA capture strands (1 OD for 30 nm gold particles; 5'- CAA CTT CAT CCA CGT TCA ACG CTA GTG AAC ACA GTT GTGT-A₁₀-(CH₂)₃-SH 3') for 16 hours and then salt-stabilized to 0.1 M NaCl. This solution was treated with 0.3 ml of a 10 % BSA solution for 30 minutes to passivate and stabilize the gold NPs. The final solution was centrifuged for 1 hour at 4 °C (20,000g), and the supernatant was removed. This centrifugation procedure was repeated for further purification. The final NP probes were re-dispersed in 0.1 M NaCl/0.01 M phosphate buffer solution at pH 7.4. The PSA-specific barcode DNA strands (1 OD; 5'-ACA CAA CTG TGT TCA CTA GCG TTG AAC GTG GAT GAA GTT G-3') were then hybridized with the biobarcode DNA capture strands coordinated to the NPs and purified using a similar centrifugation procedure. The oligonucleotide loading was determined by the method of Demers *et al*., see: L. M. Demers et al. Anal. Chem. 72, 5535 (2000). Amino-functionalized 1 µm diameter MMPs were obtained from Polysciences, Inc. MMPs were linked to mAbs to PSA using the commercial glutaraldehyde-amine coupling chemistry. Coupling efficiency was determined to be 90% by UV-vis spectroscopy by comparing the absorbance at 270 nm before and after protein coupling to the MMPs. The MMPs were suspended in 40 ml of 0.1 M PBS buffer (pH 7.4) prior to use.

Chip-based assays were used to measure directly the amount of barcode DNA in solution, generally as described in Example 6, however without use of an amplification step by *B*PCR. Since PCR is not used, this method eliminates the need to denature the duplex DNA formed during PCR amplification. Thus, after protein detection and isolation of barcode DNA, an aliquot of the isolated barcode sample (10 µl) was mixed with 0.6 M PBS (85 µl) and the 13 nm gold detection probe (5 µl, 500 pM final concentration, same sequence as above). This mixture was added to the wells of a Verigene on-chip hybridization chamber under which the appropriate capture strands were arrayed as described above. The sample was incubated for 2 h at 42 °C. After incubation, the reaction mixture was removed and the chips were washed with 0.5 M NaNO₃/0.01 M phosphate buffer to remove excess gold nanoparticles. Surface immobilized gold particles were stained with silver enhancement solution (Ted Pella) for 6 min, washed with Nanopure water, and imaged with the Verigene ID system, all as described above.

Although 30 nm gold NPs were used in this instance, 13 nm gold NPs are contemplated for use with this procedure for direct detection of Barcode DNA. Nevertheless, by increasing the size of NP probes in the protein detection step, the number of DNA strands for each NP can be increased significantly, aiding in the direct detection (in theory, assuming 100 DNA strands are attached to a 13 nm gold NP, there could be as many as 532 DNA strands on each 30 nm gold NP). The size of the particular nanoparticle probe can be adjusted to optimize certain aspects

The results (Fig. 12) demonstrate that the PSA target was directly detectable at concentrations as low as 30 attomolar, using 30 nm gold NP (at 20 pM) without *B*PCR amplification. Although this represents a loss in sensitivity of an order of magnitude relative to the *B*PCR amplification method, this method is still exquisitely sensitive and provides for significant decrease in cost, effort, and time, through elimination of the PCR step.

### Example 10: Direct Detection of Target Nucleic Acid Sequence at ZeptoMolar Concentrations

For this experiment, the DNA sequence associated with the anthrax lethal factor (5'-GGA TTA TTG TTA AAT ATT GAT AAG GAT-3'; SEQ ID NO:14) was chosen as an initial target because this sequence is important for bio-terrorism and bio-warfare applications and is well studied in the literature.^{63,67-69} To each 20 µL test sample, two control DNA sequences were added [1 µL of 10 pM (5'-CTA TTA TAA TAA AAT ATT TAT ATA GCA-3'; SEQ ID NO:15) and 1 µL of 10 pM for control 2 (5'-GAA TTA TAG TTA ACT ATA GCT AAG GAT-3'; SEQ ID NO:16)]. Prior to use, the nanoparticle probes were loaded with barcode DNA by hybridization (20 nm probes at 400 pM; 30 nm probes at 200 pM). Barcode DNA was introduced at 10 µM concentration to effect hybridization in an appropriate hybridization buffer. The particles were subsequently centrifuged and washed with PBS buffer. The probes were then suspended in appropriate storage buffer, and stored until use.

For the MMPs, the polyamine-functionalized polystyrene particles were linked with alkylthiol-capped DNA by reacting them with a sulfosuccinimidyl 4-[p-maleimidophenyl]butyrate (sulfo-SMPB) bifunctional linker that reacts with the primary amines on the MMPs and the thiol groups on the oligonucleotide which form the a specific recognition binding site for target sequence. The MMPs were passivated with bovine serum albumin prior to use by adding 10% BSA to the solution containing them. The probes were then centrifuged, washed with PBS buffer, and resuspended at 2 mg/mL to yield active probes (Figure **13A**).

The assay was performed by adding 50 µL of the MMP probes (at 2 mg/mL) to a solution that contained target DNA in single stranded form. The system was allowed to stand at room temperature for 10 min. Following the 10 min. standing period, 50 µL of the NP probes [50 µL at 400 pM (20 nm NP probe solution) or 50 µL at 200 pM (30 nm NP probe solution)] were added to the solution and allowed to hybridize for 50 min. After hybridization, a magnetic field was applied to the reaction vessel (BioMag multi-6 microcentrifuge tube separator, Polysciences, Incorporated, Warrington, PA), which pulled the target DNA strands sandwiched with MMPs and NPs, as well as unreacted MMPs, to the wall of the reaction vessel. Any remaining unreacted reaction solution components, especially NPs not specifically hybribized to MMPs, were washed away with several washes with PBS buffer. The magnetic field was then removed and 50 µl of NANOpure water (Barnstead International, Dubuque, IA) was added to the reaction vessel and the system was heated to 55°C for 3 min. to release the bar-code DNA. Reintroduction of the magnetic field removed all of the MMPs from solution, leaving barcode DNA for detection.

To analyze the amount and identity of the barcode DNA in the final reaction solution, scanometric methods were used.⁶⁷ Scanometric methods are chip-based DNA detection methods that rely on oligonucleotide-modified gold NP probes (5'-TCT CAA CTC GTA GCT-A₁₀-SH-3'-Au; SEQ ID NO:17) and NP-promoted reduction of silver(I) for signal amplification. For this particular assay, maleimide-modified glass chips were spotted with 5' capture DNA strands (5'-SH-A₁₀-CGT CGC ATT CAG GAT-3'; SEQ ID NO:18) using a DNA microarrayer (spot diameter is 175 µm and the distance between two spots is 375 µm; GMS 417 Arrayer, Genetic MicroSystems, Woburn, Massachusetts). The non-spot area of the surface was passivated with a A₁₀ sequence (10 µM of 5'-SH-AAA AAA AAA A-3'; SEQ ID NO:19) overnight. Once the chip surface was contacted with the barcode DNA solution, NP probes mixed with target sequence solution were added to the barcode/capture DNA-modified chip. The spots on a chip were labeled with NP probes and target DNA strands. The spotted chip was then exposed to silver enhancement solution (Ted Pella, Redding, CA) for further signal enhancement. The developed spots were then read with a Verigene ID (identification) system (Nanosphere, Incorporated, Northbrook, IL). The Verigene ID system measures the scattered light from the developed spots and provides a permanent record for the assay. Figure 14A illustrates 20-nm NP probes used in the detection of "amplified" barcode DNA. The spot intensities for target DNA concentrations from 5 fM to 50 aM are clearly stronger than control spots. To measure spot intensity of each concentration, three spots were patterned on a chip and imaged with the Verigene ID system. The gray levels of the spots were calculated with graphic software (Adobe Photoshop) and the spot intensity graph is shown in Figure 15. This graph shows that 20-nm NP probes can clearly detect target DNA at about 50 aM, but cannot differentiate signal from background at a concentration of 5 aM.

When 30 nm NP probes were used for DNA-BCA detection, target DNA concentration as low as 500 zM was detected (Figure 14B). This difference in detection limit for the 20 and 30 nm NP probes may be due to the difference in the absolute number of barcode DNA strands on NP probes of different surface areas. The intensity graph suggests that 30-nm NP probe system provides a more intense spot signal than 20-nm NP probe system at all sample concentrations (Figure 15). A sample volume of 20 µL at 500 zM represents a total sample number of approximately 10 target DNA strands, providing a sensitivity comparable to assays that employ PCR-based methods coupled with molecular fluorophore probes.⁵⁸⁻⁶⁰

### Example 11

### Simplified Reporter-Based Bio-barcode Assay

The detection of prostate-specific antigen (PSA) will be used as an illustrative example for consistency with Example 8. PSA was chosen for this example as clinically significant levels of PSA are close to the detection limits of the tests that are presently used for this cancer marker and, thus, a method of increased sensitivity is desirable. The generalization of this example to the detection of multiple target analytes in the same medium can be implemented by mixing substrates and detection probes prepared as described below to form the corresponding reagent "cocktails" that are then used as described below. Alternative implementations such as forming spatially separated binding zones for different target analytes on the same substrate are also practical and may be desirable in specific uses of this invention. A magnetic substrate will be described for consistency with the previous Examples, but other types of substrates may be more appropriate for specific uses of this invention.

### a) Preparation of Substrates

Magnetic microparticle detection probes (MMPs) were prepared by linking a monoclonal antibody specific for one epitope of PSA (Maine Biotechnologies, Maine, MA) to amino-functionalized magnetic microparticles (Polysciences, Inc., Warrington, PA) using a standard glutaraldehyde-amine coupling chemistry. Amino-functionalized MMPs (5 mg) were activated with 5 ml of 5% glutaraldehyde in pyridine wash buffer (PWB) for 3 hrs at room temperature. The activated MMPs were then magnetically separated, and the supernatant was removed. This magnetic separation step was repeated twice, and the MMPs were re-suspended in PWB. The monoclonal antibody (750 µg) was then added to MMPs, and the solution was mixed for 10 hrs at room temperature. Non-specific binding sites were blocked by adding 1 mg of bovine serum albumin (BSA) to the MMP solution and mixing for 10 additional hrs at room temperature. The magnetic separation step was repeated twice, and the MMPs were re-suspended in 5 ml of PWB. Then 3 ml of glycine solution (1 M at pH 8.0) was added to the resulting solution to quench all the unreacted glutaraldehydes and stirred for 30 min. After the magnetic separation step (repeated twice), 5 ml of wash buffer was mixed vigorously with monoclonal antibody-functionalized MMPs and the MMPs were magnetically separated again (this washing step was repeated three times). Finally, the MMP probes were resuspended in 0.15 M phosphate-buffered saline (PBS) solution. The coupling efficiency was determined to be 90% by UV-Vis spectroscopy by comparing the absorbance at 280 nm before and after protein coupling to the MMPs.

### b) Preparation of Probes

Detection probes were prepared using one micron diameter amino-functionalized polystyrene microparticles (Polysciences, Inc., Warrington, PA). One milliliter of a 1.25% aqueous suspension of the amino-functionalized polystyrene microparticles was centrifuged for 5 min at 10,000 rpm, and the supernatant was removed. The pellet was then resuspended in PBS and the centrifugation step was repeated once more. The resulting polystyrene particle pellet was re-suspended in 1 ml of 8% glutaraldehyde in PBS at pH 7.4 and mixed for 5 hr on a rocking shaker. The particles were then sedimented for 5 minutes at 10,000 rpm, and the supernatant was discarded (this step was repeated twice). The resulting pellet was re-suspended in PBS and 10 µg of polyclonal antibody for PSA (Maine Biotechnologies, Maine, MA) was added and allowed to react over night on a rocking shaker at room temperature. The amount of antibody added is 20-30 times less than the amount of antibody that is needed to fully modify the particle surface so that surface sites are available for the attachment of large numbers of oligonucleotides to the same particle in the next step. These particles were sedimented for 5 minutes at 10,000 RPM and washed twice with PBS by suspension and sedimentation as before.

Oligonucleotide-functionalized microparticles were prepared by resuspending the pelleted activated microparticles from the previous step in 1 ml of a 100 µM solution of the appropriate 3' amino-functionalized oligonucleotide and allowing them to react over night on a rocking shaker at room temperature. "Barcode" detection probes analogous to those of the previous invention were prepared using the oligonucleotides and methods described in the following paragraph. For detection probes where the identification of the target analyte was not encoded in the nucleotide sequence, fluorescently labeled 3' amino-functionalized 30-base oligonucleotides having arbitrary sequences were used. The reacted particles were sedimented for 5 minutes at 10,000 RPM and washed twice with PBS by suspension and sedimentation as before. The resulting pellet was resuspended in 1 ml of 0.2 M ethanolamine to passivate all unreacted glutaraldehyde sites on the microparticles for 30 min at room temperature, sedimented for 5 minutes at 10,000 RPM, and the supernatant was removed by decantation. Bovine serum albumin (10% BSA) solution was subsequently added to further passive the protein-inactive regions of the particle surface. The centrifugation step was repeated twice to remove the supernatant, and the pellet was re-suspended in 1 ml of 0.15 M PBS solution. Disulfidelinked, fluorescently labeled chemically cleavable oligonucleotides were attached to the activated microparticles in a similar manner except that the initial reaction with an amino-functionalized oligonucleotide was omitted; thioethanolamine was substituted for ethanolamine in the passivation step; and a fluorescently labeled, 3' thiol-functionalized oligonucleotide was coupled to the resulting thiol groups by oxidation to the disulfide.

"Barcode" detection probes analogous to those of the previous invention were prepared by resuspending the pelleted microparticles in 1 ml of a 100 µM solution of the 3' amino-functionalized oligonucleotide 5' CGTCGCATTCAGGATTCTCAACTCGTAGCT-A₁₀-C6-amine 3' (SEQ ID NO:20) to the glutaraldehyde-activated microparticles of the previous step and allowed to react over night on a rocking shaker at room temperature. The same oligonucleotide pair or single oligonucleotide may be used for all target analytes. These particles were sedimented for 5 minutes at 10,000 RPM and washed twice with PBS by suspension and sedimentation as before. The resulting pellet was re-suspended in 1 ml of 0.2 M ethanolamine to passivate all unreacted glutaraldehyde sites on the microparticles for 30 min at room temperature, sedimented for 5 minutes at 10,000 RPM, and the supernatant was removed by decantation. Bovine serum albumin (10% BSA) solution was subsequently added to further passive the protein-inactive regions of the particle surface. The centrifugation step was repeated twice to remove the supernatant, and the pellet was re-suspended in 1 ml of 0.15 M PBS solution. 200 µl of 5 mg/ml polystyrene probe particle suspension was then combined with 200 µl of a 33 µM solution of the compelmentary Alexa Fluor® 647-labeled (Molecular Probes, Eugene OR) labeled oligonucleotide 5' AGCTACGAGTTGAGAATCCTGA-ATGCGACG 3' (SEQ ID NO:21, purchased from Integrated DNA Technologies, Coralville, IA, or synthesized and purified using literature methods.³⁵) and hybridized to the complementary oligonucleotide strands on the polystyrene particles. The particles were then washed by suspension and sedimentation prior to use. The oligonucleotide loading for each particle was determined by sedimenting 100 µl of 500 µg/ml particle suspension, discarding the supernate and dehybridizing by resuspending the pellet in 100 µl of NANOpure water. The particles were removed by sedimentation and the fluorescence of the released oligonucleotides in solution was measured in a 50 µl fluorescence micro-cuvette (Starna Cells, Inc., Atascadero, CA) using a SPEX Fluorolog-3 spectro-fluorometer (JOBIN YVON). Quantitation was via comparison of the measured sample fluorescence against a calibration curve for the pure Alexa Fluor® 647-labeled bar-code DNA solution. The average number of bar-code DNA strands for each polystyrene particle was determined to be ∼ 1.1x10⁵.

### c) PSA Test (Reaction)

10 µl of a clinical sample or a calibration standard containing PSA (Sigma Chemical Company, Milwaukee, WI) was added to 50 µl of MMP probes functionalized with monoclonal anti-PSA antibodies (500 µg/ml) in a 1.5 mL BioMag® microcentrifuge tube and the solution was shaken on an orbital shaker at 37° C for 23 min (Step 1 in Figure 18). The tube containing the assay solution was then placed in a BioMag® microcentrifuge tube separator (Polysciences, Inc.) at room temperature. After 15 seconds, the MMP-PSA hybrids are concentrated on the wall of the tube. The supernatant was removed, and the MMPs are re-suspended in 50 µl of 0.1 M PBS (repeated twice). Detection probes (50 µl at 1.25 mg/ml) of either type described above were then added to the solution (Step 1 in Figure 18) and vigorously stirred at 37° C for 23 minutes. The magnetic particles were then separated from the reaction mixture and washed twice with 0.15 M PBS using the magnetic separator as described above (Step 2 in Figure 18).

### d) PSA Test (Barcode Detection Version)

The magnetic particles were re-suspended in NANOpure (18 MΩ) water (50 µl) to dehybridize the fluorescently labeled bar-code oligonucleotides from the complementary oligonucleotides on the polystyrene probe surface. The magnetic particles were removed from the suspension using the magnetic separator (Step 3 in Figure 18) and the integrated fluorescence emission intensity of the released oligonucleotides over the 660-670nm spectral range was measured using a SPEX Fluorolog-3 spectro-fluorimeter (650nm excitation/665nm emission for Alexa Fluor^{®}647). Integrated emission intensity over the 660-670nm spectral range was determined and used as a measure of PSA concentration in the initial medium.

### e) PSA Test (Chemical Release Version)

The magnetic particles were re-suspended in PBS (50 µl) containing dithiothreitol (DTT) and heated for 10 minutes to 50°C to release the fluorescently labeled oligonucleotides from the polystyrene probe surface. The magnetic particles were removed from the suspension using the magnetic separator (Step 3 in Figure 18) and the integrated fluorescence emission intensity of the released oligonucleotides over the 660-670nm spectral range was measured using a SPEX Fluorolog-3 spectro-fluorimeter (650nm excitation/665nm emission for Alexa Fluor^{®}647). Integrated emission intensity over the 660-670nm spectral range was determined and used as a measure of PSA concentration in the initial medium.

### f) PSA Test (In-Situ Detection Version)

The magnetic particles were re-suspended in PBS and the integrated fluorescence emission intensity of the particle-bound oligonucleotides was measured using a SPEX Fluorolog-3 spectro-fluorimeter (650nm excitation/665nm emission for Alexa Fluor^{®}647). The fluorescence of the magnetic microparticles could also be determined in a flow cytometer according to standard procedures.

### g) PSA Test (Results)

All target analyte concentrations over the 30 aM to 300 fM concentration range tested could be easily differentiated from the corresponding negative control (Figure 19A). Importantly, the improvements of the present inventions described above retained the 30 aM target concentration sensitivity of the previous invention, but allowed the reduction of the total assay time from over four hours to less than 50 minutes and significantly reduced the number of steps and the complexity of the instrumentation required.

### Example 12

### Chemical Release of Particle Bound Molecules

A fluorescent assay was constructed as demonstrated in Figures 20 through 22. Messenger RNA (mRNA) served as the target analyte for detection. Poly-T-labeled magnetic beads captured the 3'-Poly A target analytes to immoblize them against a magnetic support and separate them from the sample solution. The detection reagent used was composed of gold nanoparticles functionalized with an oligonucleotide complimentary to the 5' end of the mRNA targets. The complimentary oligonucleotides functioned as both the reporter moiety and the selective binding compound and were bound to the gold nanoparticles through gold-thiol bonds. The detection reagent was added to the solution allowing binding of the mRNA targets to the complementary reporter moieties to form a sandwich of the Poly-T-labeled beads-the target mRNA analyte-the complementary oligonucleotide binding compound-the gold nanoparticle. After washing, dithiothreitol (DTT) was added to the water containing the sandwich structure formed and heated for 10 minutes to 50°C. The water and heat dehybridized the magnetic and gold nanoparticle structures held together by the target mRNA molecule. The DTT exchanged with the thiolated DNA reporter moieties on the surface of the 13 nm gold nanoparticles releasing the latter into solution for subsequent detection after removal of the magnetic and gold particles. A fluorescent molecule (Alexa 488) was added adjacent to the thiol so that solution liberated probe sequences could be detected using a fluorimeter. As shown in Figure 23, target mRNA concentrations down to 6 pM were detected, representing liberated Alexa 488-labeled-mRNA probe strands at approximately 1 nM concentration; the limit for the fluorimetric detection of the Alexa 488 dye in a 50 microliter volume. This example demonstrates that the use of the presently claimed detection methodology for the detection of nucleic acid targets can be as sensitive as the polymerase chain reaction, without the need for enzymatic amplification of the target sequence.

### References

(1) A. Pandey and M. Mann, Nature 2000, 405, 837-846.
(2) S. Fields and O. K. Song, Nature 1989, 340, 245-246.
(3) M. Ijksma, B. Kamp, J. C. Hoogvliet, W. P. van Bennekom, Anal. Chem. 2001, 73, 901-907.
(4) R. F. Service, Science, 2000, 287, 2136-2138.
(5) H. Zole, Monoclonal Antibodies, Springer-Verlag, New York, 2000, p.1-5.
(6) J. E. Butler, J. Immunoassay, 2000, 21 (2 & 3), 165-209.
(7) P. Herbrink, A. Noorduyn, W. C. Van Dijk, Tech. Diagn. Pathol. 1991, 2, 1-19.
(8) C. A. Mirkin, R. L. Letsinger, R. C. Mucic, J. J. Storhoff, Nature 1996, 382, 607-609
(9) J. J. Storhoff, C. A. Mirkin, Chem. Rev. 1999, 99, 1849-1862.
(10) S. -J. Park, A. A. Lazarides, C. A. Mirkin, P. W. Brazis, C. R. Kannewurf, R. L. Letsinger, Angew. Chem. Int. Ed. 2000, 39, 3845-3848.
(11) T. A. Taton, C. A. Mirkin, and R. L. Letsinger, Science 2000, 289, 1757-1760.
(12) S. -J. Park, A. A. Lazarides, C. A. Mirkin, and R. L. Letsinger, Angew. Chem. Int. Ed. 2001, 40, 2909-2912.
(13) Z. Eshhar, M. Ofarim, and T. Waks, J. Immunol. 1980, 124, 775-780.
(14) M. Wilcheck and E. A. Bayer, Immunol. Today 1984, 5, 39-43
(15) N. Winssinger, J.L. Harris, B.J. Backes, P.G. Schultz, Agnew. Chem. Int. Ed. 2001, 40, 3152-3155
(16) G. MacBeath, A.N. Koehler, S.L. Schreiber, J. Am. Chem. Soc. 1999, 121, 7967-7968.
(17) P. J. Hergenrother, K.M. Depew, S.L. Schreiber, J. Am. Chem. Soc. 2000, 122, 7849-7850.
(18) J. J. Storhoff, R. Elghanian, R.C. Mucic, C.A. Mirkin, R.L. Letsinger, J. Am. Chem. Soc. 1998, 120, 1959-1964.
(19) R.C. Mucic, J.J. Storhoff, C.A. Mirkin, R.L. Letsinger, J. Am. Chem. Soc. 1998, 120,12674-12675.
(20) L.M. Demers, C.A. Mirkin, R.C. Mucic, R.A. Reynolds III, R.L. Letsinger, R. Elghanian, G. Viswanadham, Anal. Chem. 2000, 72, 5535-5541.
(21) T. Brown, D.J.S. Brown, in Oligonucleotides and Analogues (Ed.: F. Eckstein), Oxford University Press, New York, 1991.
(22) L.A. Chrisey, G.U. Lee, and C.E. O'Ferral, Nucl. Acids. Res. 1996, 24, 3031-3039.
(23) Nicewamer-Pena, S.R. Freeman, R.G.; Reiss, B.D.; He, L.; Pena, D.J.; Walton, I.D.; Cromer, R.; Keating, C.D.; Natan M.J. Science 2001, 294, 137.
(24) Ferguson, J.A.; Steemers, F.J.; Walt, D.R. Anal. Chem. 2000, 72, 5618.
(25) Han, M.; Gao, X.; Nie, S. Nature biotech. 2001, 19, 631.
(26) R.K. Saiki et al., Science 1985, 230, 1350.
(27) R.K. Saiki et al., Science 1988, 239, 487.
(28) R.A. Gibbs, Curr. Opin. Biotechnol. 1991, 2, 69.
(29) S. Bortolin, T.K. Christopoulos, M. Verhaegen, Anal. Chem. 1996, 68, 834.
(30) B. Deiman, P. van Aarle, P. Sillekens, Mol. Biotechnol. 2002, 20, 163.
(31) S.E. Stiriba, H. Frey, R. Haag, Angew. Chem. Int. Ed. 2002, 41, 1329.
(32) S.A. Bustin, Journal of Molecular Endocrinology 2002, 29, 23.
(33) G. MacBeath, S.L. Schreiber, Science 2000, 289, 1760.
(34) H. Zhu et al., Science 2001, 293, 2101.
(35) B.B. Haab, M.J. Dunham, P.O. Brown, Genome Biol. 2001, 2(2): RESEARCH 0004.1.
(36) J.-M. Nam, S.-J. Park, C.A. Mirkin, J. Am. Chem. Soc. 2002, 124, 3820.
(37) T. Sano, C.L. Smith, C.R. Cantor, Science 1992, 258, 120.
(38) V. Ruzicka, W. Marz, A. Russ, W. Gross, Science 1993, 260, 698.
(39) A. McKie, D. Samuel, B. Cohen, N.A. Saunders, J. Immunol. Methods 2002, 270, 135.
(40) H. Zhou, R.J. Fisher, T.S. Papas, Nucl. Acids Res. 1993, 21, 6038.
(41) E.R. Hendrickson, T.M. Hatfield-Truby, R.D. Joerger, W.R. Majarian, R.C. Ebersole, Nucl. Acids Res. 1995, 23, 522.
(42) C.M. Niemeyer et al., Nucl. Acids Res. 1999, 27, 4553.
(43) B. Schweitzer et al., Proc. Natl. Acad. Sci. U.S.A. 2000, 97, 10113.
(44) C.M. Neimeyer, R. Wacker, M. Adler, Angew. Chem. Int. Ed. 2002, 40, 3169.
(45) C.M. Niemeyer, Trends Biotechnol. 2002, 20, 395.
(46) H. Yu, E.P. Diamandis, A.F. Prestigiacomo, T.A. Stamey, Clin. Chem. 1995, 41, 430.
(47) W.J. Catalona, et al., J. Am. Med. Assoc. 1995, 274, 1214.
(48) J.L. Stanford, et al., Prostate Cancer Trends 1973-1995, SEER Program, National Cancer Institute. NIH Pub. No. 99-4543. Besthesda, MD, 1999.
(49) J. Moul, J. Urol. 2000, 163, 1632.
(50) L.A. Chrisey, G.U. Lee, C.E. Oferral, Nucl. Acids Res. 1996, 24, 3031.
(51) Y.C. Cao, R. Jin, C.A. Mirkin, Science 2002, 297, 1536-1540.
(52) R. K. Saiki et al., Science 230, 1350 (1985).
(53) S. A. Bustin, Journal of Molecular Endocrinology 29, 23 (2002).
(54) M. U. Kopp, A. J. de Mello, A. Manz, Science 280, 1046 (1998).
(55) T. H. Rider et al., Science 301, 213 (2003).
(56) Y. W. Tang, G. W. Procop, D. H. Pershing. Clin. Chem. 43, 2021 (1997).
(57) G. M. Makrigiorgos, S. Chakrabarti, Y. Zhang, M. Kaur, B. D. Price, Nat. Biotechnol. 20, 936 (2002).
(58) W. P. Halford, Nat. Biotechnol. 17, 835 (1999).
(59) R. Sutthent et al., J. Clin. Microbiol. 41, 1016 (2003).
(60) B. Schweitzer, S. Kingsmore, Curr. Opin. Biotechnol. 12, 21 (2001).
(61) S. R. Nicewarner-Pena et al., Science 294, 137 (2001).
(62) M. Han, X. Gao, S. Nie, Nat. Biotechnol. 19, 631 (2001).
(63) X. Zhao, R. Tapec-Dytioco, W. Tan, J. Am. Chem. Soc. 125, 11474 (2003).
(64) L. A. Lortie et al., J. Clin. Microbiol. 29, 2250 (1991).
(65) L. R. Zeph, X. Y. Lin, G. Stotzky, Curr. Microbiol. 22. 79 (1991).
(66) C. J. Yu et al., J. Am. Chem. Soc., 123, 11155 (2001).
(67) T. A. Taton, C. A. Mirkin, R. L. Letsinger, Science 289, 1757 (2000).
(68) S.-J. Park, T. A. Taton, C. A. Mirkin, Science 295, 1503 (2002).
(69) Y. C. Cao, R. Jin, C. A. Mirkin, Science 297, 1536 (2002).
(70) A. Saghatelian, K. M. Guckian, D. A. Thayer, M. R. Ghadiri, J. Am. Chem. Soc. 125, 344 (2003).
(71) J.-M. Nam, C. S. Thaxton, C. A. Mirkin, Science 301, 1884.
(72) J.-M. Nam, S.-J. Park, C. A. Mirkin, J. Am. Chem. Soc. 124, 3820 (2002).
(73) L. M. Demers et al., Anal. Chem. 72, 5535 (2000).

## Claims

1. A method for detecting for the presence of one or more target analytes in a sample, each target analyte having at least two binding sites for specific binding interactions with specific binding complements, said method comprising the steps of:
a) providing at least- one type of capture substrates, the capture substrates having bound thereto at least one specific binding complement of the specific target analyte that binds to at least a first binding site of the specific target analyte;
b) providing at least one type of microparticle detection probes, the microparticle detection probes comprising a microparticle having bound thereto (i) at least one specific binding complement to a specific target analyte and (ii) one or more DNA barcodes, each DNA barcode labeled with a reporter group, wherein the specific binding complement bound to the microparticle detection probes binds to at least a second binding site of the target analyte;
c) contacting the capture substrates with a sample believed to contain target analytes under conditions effective to allow for binding of the specific target analyte to the specific binding complement bound to the capture substrate so as to immobilize the target analytes onto the capture substrates;
d) contacting the immobilized target analytes with at least one type of microparticle detection probes under conditions effective to allow for binding between the target analytes and the specific binding complement bound to the microparticle detection probes to the analyte and to form a complex in the presence of the target analyte on the capture substrate;
e) optionally isolating and washing the capture substrate to remove unbound microparticle detection probes;
f) subjecting the complex to conditions effective to release the DNA barcodes from microparticles detection probes; and
g) detecting the released DNA barcodes wherein the presence of the released DNA barcodes is indicative of the presence of the complex; wherein the released DNA barcodes are detected by detecting the reporter group of step 1 b(ii) on the released barcodes.

2. The method of claim 1, wherein the target analyte is a protein or hapten and its specific binding complement is an antibody comprising a monoclonal or polyclonal antibody.

3. The method of claim 1, wherein the target analyte is a nucleic acid molecule.

4. The method of any one of claims 1-3, wherein the reporter group comprises biotin, a fluorophore, a chromophore, a redox-active group, a group with an electrical signature, radioactive group, a catalytic group, or Raman label.

5. The method of any one of claims 1-4, wherein the microparticle has a plurality of DNA barcodes attached thereto.

6. The method of any one of claims 1-5, further comprising the step of washing the capture substrate after the target analytes are immobilized thereon and prior to contacting the capture substrate with the microparticle detection probes.

7. The method of claim 5 wherein the released DNA barcodes are immobilized on a substrate prior to said detecting.

8. The method of claim 5, wherein the DNA barcodes are directly bound to the microparticle.

9. The method of claim 8, wherein the DNA barcodes are released from the particle by a chemical releasing agent.

10. The method of claim 9, wherein the chemical releasing agent is dithiothreitol.

11. The method of claim 5, wherein the microparticle further comprises oligonucleotides bound thereto and the DNA barcodes are hybridized to at least a portion of the oligonucleotides bound to the microparticle.

12. The method of claim 11, wherein the DNA barcodes are released by dehybridization.

13. The method of any one of claims 1 - 12, wherein the capture substrate is a magnetic substrate.

14. The method of claim 13, wherein said isolating comprises subjecting the magnetic substrate to a magnetic field.

15. The method of claim 14, wherein subsequent to said isolating and washing step and prior to said detecting step, further comprising subjecting the complex to conditions effective to release the DNA barcodes.

16. The method of claim 15, wherein said isolating comprises filtration, sedimentation, flotation, or hydrodynamics.

17. The method of claim 16, wherein the filtration step comprises a membrane that removes sample components that do not comprise DNA barcodes.

18. The method of any one of claims 1-17, wherein the microparticle is a polymeric, glass, metallic, semiconductor, or ceramic microparticle.

19. The method of claim 18, wherein the polymeric microparticle comprises polystyrene.

20. The method of any one of claims 1-19, wherein the target has more than two binding sites.

21. The method of claim 20 wherein at least two types of microparticle detection probes are provided, the first type of microparticle detection probe having a specific binding complement to a first binding site on the target analyte and the second type of microparticle detection probe having a specific binding complement to a second binding site on the target analyte.

22. The method of claim 21, wherein a plurality of types of microparticle detection probes are provided, each type of microparticle detection probe having a specific binding complement to different binding sites on the target analyte.

23. The method of any one of claims 1-22, wherein the specific binding complement and the target analyte are members of a specific binding pair.

24. The method of claim 23, wherein members of a specific binding pair comprise nucleic acid, oligonucleotide, peptide nucleic acid, polypeptide, antibody, antigen, carbohydrate, protein, peptide, amino acid, hormone, steroid, vitamin, drug, virus, polysacharides, lipids, lipopolysaccharides, glycoproteins, lipoproteins, nucleoproteins, oligonucleotides, antibodies, immunoglobulins, albumin, hemoglobin, coagulation factors, peptide and protein hormones, non-peptide hormones, interleukins, interferons, cytokines, peptides comprising a tumor-specific epitope, cells, cell-surface molecules, microorganisms, fragments, portions, components or products of microorganisms, small organic molecules, nucleic acids and oligonucleotides metabolites of or antibodies to any of the above substances.

25. The method of claim 24, wherein nucleic acid and oligonucleotide comprise genes, viral RNA and DNA, bacterial DNA, fungal DNA, mammalian DNA, cDNA, mRNA, RNA and DNA fragments, oligonucleotides, synthetic oligonucleotides, modified oligonucleotides, single-stranded and double-stranded nucleic acids, and natural and synthetic nucleic acids.

26. The method of claim 23, wherein the specific binding pair is an antibody and an antigen.

27. The method of claim 23, wherein the specific binding pair is a receptor and a ligand.

28. The method of claim 23, wherein the specific binding pair is an enzyme and a competitive inhibitor.

29. The method of claim 23, wherein the specific binding pair is a drug and a target molecule.

30. The method of claim 23, wherein the specific binding pair is two strands of at least partially complementary oligonucleotides.

31. The method according to claim 23, wherein the target analyte is a nucleic acid and the specific binding complement is an oligonucleotide.

32. The method according to claim 23, wherein the target analyte is a protein or hapten and the specific binding complement is an antibody comprising a monoclonal or polyclonal antibody.

33. The method according to claim 23, wherein the target analyto is a sequence from a genomic DNA sample and the specific binding complements are oligonucleotides, the oligonucleotides having a sequence that is complementary to at least a portion of the genomic sequence.

34. The method of claim 33, wherein the genomic DNA is eukaryotic, bacterial, fungal or viral DNA.

35. The method according to claim 23, wherein the target analyte is a sequence from episomal DNA sample and the specific binding complements are oligonucleotides, said oligonucleotides having a sequence that is complementary to at least a portion of the episomal DNA sequence.

36. The method according to claim 23, wherein the specific binding complement and the target analyte are members of an antibody-ligand pair.

37. The method according to any one of claims 1-36, wherein in addition to its first binding site, the target analyte has been modified to include a second binding site.

## Patentansprüche

1. Ein Verfahren zum Nachweisen des Vorliegens von einem oder mehr Zielanalyten in einer Probe, wobei jeder Zielanalyt mindestens zwei Bindungsstellen für spezifische Bindungswechselwirkungen mit spezifischen Bindungskomplementen hat, das Verfahren umfassend die Schritte:
a) Bereitstellen von mindestens einem Typ von Einfangsubstrat, wobei mindestens ein spezifisches Bindungskomplement des spezifischen Zielanalyten, der an mindestens eine erste Bindungsstelle des spezifischen Zielanalyten bindet, an die Einfangsubstrate gebunden ist;
b) Bereitstellen von mindestens einem Typ von Mikropartikel-Nachweissonden, wobei die Mikropartikel-Nachweissonden ein Mikropartikel umfassen, an den (i) mindestens ein spezifisches Bindungskomplement für ein spezifisches Zielanalyt und (ii) ein oder mehr DNS-Barcodes, jeder DNS-Barcode markiert mit einer Reportergruppe, gebunden sind, wobei das an die Mikropartikel-Nachweissonden gebundene spezifische Bindungskomplement mindestens an eine zweite Bindungsstelle des Zielanalyten bindet;
c) Kontaktieren der Einfangsubstrate mit einer Probe, von der angenommen wird, dass sie Zielanalyten enthält, unter Bedingungen, die wirksam sind, die Bindung des spezifischen Zielanalyten an das ans Einfangsubstrat gebundene spezifische Bindungskomplement zu erlauben, so dass die Zielanalyte auf den Einfangsubstraten immobilisiert werden;
d) Kontaktieren der immobilisierten Zielanalyten mit mindestens einem Typ von Mikropartikel-Nachweissonden unter Bedingungen, die wirksam sind, die Bindung zwischen den Zielanalyten und dem an die Mikropartikel-Nachweissonden gebundenen spezifischen Bindungskomplement an den Analyten zu erlauben und einen Komplex in der Gegenwart des Zielanalyten auf dem Einfangsubstrat zu bilden;
e) optional Isolieren und Waschen des Einfangsubstrates, um nicht gebundene Mikropartikel-Nachweissonden zu entfernen;
f) Unterwerfen des Komplexes unter Bedingungen, die wirksam sind, die DNS-Barcodes von den Mikropartikel-Nachweissonden zu entfernen; und
g) Nachweisen der freigesetzten DNS-Barcodes, wobei das Vorhandensein der freigesetzten DNS-Barcodes bezeichnend ist für das Vorhandensein des Komplexes; wobei die freigesetzten DNS-Barcodes nachgewiesen werden durch den Nachweis der Reportergruppe aus Schritt 1 b(ii) auf den freigesetzten Barcodes.

2. Das Verfahren aus Anspruch 1, wobei der Zielanalyt ein Protein oder Hapten ist und sein spezifisches Bindungskomplement ein Antikörper, umfassend einen monoklonalen oder polyklonalen Antikörper, ist.

3. Das Verfahren aus Anspruch 1, wobei der Zielanalyt ein Nukleinsäuremolekül ist.

4. Das Verfahren aus irgendeinem der Ansprüche 1-3, wobei die Reportergruppe Biotin, ein Fluorophor, ein Chromophor, eine redoxaktive Gruppe, eine Gruppe mit einer elektrischen Signatur, radioaktive Gruppe, eine katalytische Gruppe oder Raman-Markierung umfasst.

5. Das Verfahren aus irgendeinem der Ansprüche 1-4, wobei am Mikropartikel eine Vielzahl von DNS-Barcodes angebracht ist.

6. Das Verfahren aus irgendeinem der Ansprüche 1-5, weiter umfassend den Schritt des Waschens des Einfangsubstrates, nachdem die Zielanalyten darauf immobilisiert sind, und vor dem Kontaktieren des Einfangsubstrates mit den Mikropartikel-Nachweissonden.

7. Das Verfahren aus Anspruch 5, wobei die freigesetzten DNS-Barcodes vor dem Nachweisen auf einem Substrat immobilisiert werden.

8. Das Verfahren aus Anspruch 5, wobei die DNS-Barcodes direkt ans Mikropartikel gebunden sind.

9. Das Verfahren aus Anspruch 8, wobei die DNS-Barcodes durch ein chemisches Freisetzungsagens vom Partikel freigesetzt werden.

10. Das Verfahren aus Anspruch 9, wobei das chemische Freisetzungsagens Dithiothreitol ist.

11. Das Verfahren aus Anspruch 5, wobei das Mikropartikel weiter daran gebundene Oligonukleotide umfasst und die DNS-Barcodes an zumindest einen Teil der ans Mikropartikel gebundenen Oligonukleotide hybridisiert sind.

12. Das Verfahren aus Anspruch 11, wobei die DNS-Barcodes durch Dehybridisierung freigesetzt werden.

13. Das Verfahren aus irgendeinem der Ansprüche 1-12, wobei das Einfangsubstrat ein magnetisches Substrat ist.

14. Das Verfahren aus Anspruch 13, wobei das Isolieren Unterwerfen des magnetischen Substrats unter ein Magnetfeld umfasst.

15. Das Verfahren aus Anspruch 14, wobei nachfolgend auf den Isolier- und Waschschritt und vor dem Nachweisschritt weiter umfassend Unterwerfen des Komplexes unter Bedingungen, die wirksam sind, die DNS-Barcodes freizusetzen.

16. Das Verfahren aus Anspruch 15, wobei das Isolieren Filtration, Sedimentation, Flotation oder Hydrodynamik umfasst.

17. Das Verfahren aus Anspruch 16, wobei der Filtrationsschritt eine Membran umfasst, die Probenkomponenten entfernt, die keine DNS-Barcodes umfassen.

18. Das Verfahren aus irgendeinem der Ansprüche 1-17, wobei das Mikropartikel ein Polymer-, Glas-, Metall-, Halbleiter- oder Keramikmikropartikel ist.

19. Das Verfahren aus Anspruch 18, wobei das Polymermikropartikel Polystyrol umfasst.

20. Das Verfahren aus irgendeinem der Ansprüche 1-19, wobei das Ziel mehr als zwei Bindungsstellen hat.

21. Das Verfahren aus Anspruch 20, wobei mindestens zwei Typen von Mikropartikel-Nachweissonden bereitgestellt werden, wobei der erste Typ von Mikropartikel-Nachweissonde ein spezifisches Bindungskomplement für eine erste Bindungsstelle auf dem Zielanalyt hat und der zweite Typ von Mikropartikel-Nachweissonde ein spezifisches Bindungskomplement für eine zweite Bindungsstelle auf dem Zielanalyt hat.

22. Das Verfahren aus Anspruch 21, wobei eine Vielzahl von Typen von Mikropartikel-Nachweissonden bereitgestellt wird, wobei jeder Typ von Mikropartikel-Nachweissonde ein spezifisches Bindungskomplement für verschiedene Bindungsstellen auf dem Zielanalyt hat.

23. Das Verfahren aus irgendeinem der Ansprüche 1-22, wobei das spezifische Bindungskomplement und der Zielanalyt Teile eines spezifischen Bindungspaares sind.

24. Das Verfahren aus Anspruch 23, wobei Teile eines spezifischen Bindungspaares Nukleinsäure, Oligonukleotid, Peptidnukleinsäure, Polypeptid, Antikörper, Antigen, Kohlenhydrat, Protein, Peptid, Aminosäure, Hormon, Steroid, Vitamin, Medikament, Virus, Polysaccharide, Lipide, Lipopolysaccharide, Glykoproteine, Lipoproteine, Nukleoproteine, Oligonukleotide, Antikörper, Immunoglobuline, Albumin, Hämoglobin, Gerinnungsfaktoren, Peptid- und Proteinhormone, Nicht-Peptid-Hormone, Interleukine, Interferone, Cytokine, Peptide umfassend ein tumorspezifisches Epitop, Zellen, Zelloberflächenmoleküle, Mikroorganismen, Fragmente, Teile, Bestandteile oder Produkte von Mikroorganismen, kleine organische Moleküle, Nukleinsäuren und Oligonukleotide, Metaboliten von oder Antikörper gegen irgendeine der obigen Substanzen umfassen.

25. Das Verfahren aus Anspruch 24, wobei Nukleinsäure und Oligonukleotid Gene, virale RNS und DNS, bakterielle DNS, Pilz-DNS, Säugetier-DNS, cDNA, mRNA, RNS- und DNS-Fragmente, Oligonukleotide, synthetische Oligonukleotide, modifizierte Oligonukleotide, einzelsträngige und doppelsträngige Nukleinsäuren und natürliche und synthetische Nukleinsäuren umfassen.

26. Das Verfahren aus Anspruch 23, wobei das spezifische Bindungspaar ein Antikörper und ein Antigen ist.

27. Das Verfahren aus Anspruch 23, wobei das spezifische Bindungspaar ein Rezeptor und ein Ligand ist.

28. Das Verfahren aus Anspruch 23, wobei das spezifische Bindungspaar ein Enzym und ein kompetitiver Inhibitor ist.

29. Das Verfahren aus Anspruch 23, wobei das spezifische Bindungspaar ein Medikament und ein Zielmolekül ist.

30. Das Verfahren aus Anspruch 23, wobei das spezifische Bindungspaar zwei Stränge von zumindest teilweise komplementären Oligonukleotiden ist.

31. Das Verfahren gemäß Anspruch 23, wobei der Zielanalyt eine Nukleinsäure ist und das spezifische Bindungskomplement ein Oligonukleotid ist.

32. Das Verfahren gemäß Anspruch 23, wobei der Zielanalyt ein Protein oder Hapten ist und das spezifische Bindungskomplement ein Antikörper, umfassend einen monoklonalen oder polyklonalen Antikörper, ist.

33. Das Verfahren gemäß Anspruch 23, wobei der Zielanalyt eine Sequenz aus einer genomischen DNS-Probe ist und die spezifischen Bindungskomplemente Oligonukleotide sind, wobei die Oligonukleotide eine Sequenz haben, die zumindest zu einem Teil der genomischen Sequenz komplementär ist.

34. Das Verfahren aus Anspruch 33, wobei die genomische DNS eukaryotisch, bakteriell, aus einem Pilz oder viral ist.

35. Das Verfahren gemäß Anspruch 23, wobei der Zielanalyt eine Sequenz aus episomaler DNS-Probe ist und die spezifischen Bindungskomplemente Oligonukleotide sind, wobei die Oligonukleotide eine Sequenz haben, die zumindest zu einem Teil der episomalen DNS-Sequenz komplementär ist.

36. Das Verfahren gemäß Anspruch 23, wobei das spezifische Bindungskomplement und der Zielanalyt Teile eines Antikörper-Ligand-Paares sind.

37. Das Verfahren gemäß irgendeinem der Ansprüche 1-36, wobei der Zielanalyt modifiziert worden ist, um zusätzlich zu seiner ersten Bindungsstelle eine zweite Bindungsstelle zu umfassen.

## Revendications

1. Procédé de détection de la présence d'un ou de plusieurs analytes cibles dans un échantillon, chaque analyte cible ayant au moins deux sites de liaison pour des interactions de liaison spécifiques avec des compléments de liaison spécifiques, ledit procédé comprenant les étapes consistant à :
a) fournir au moins un type de substrats de capture, au moins un complément de liaison spécifique de l'analyte cible spécifique qui se lie à au moins un premier site de liaison de l'analyte cible spécifique étant lié aux substrats de capture ;
b) fournir au moins un type de sondes de détection de microparticules, les sondes de détection de microparticules comprenant une microparticule à laquelle sont liés (i) au moins un complément de liaison spécifique d'un analyte cible spécifique et (ii) un ou plusieurs codes à barres d'ADN, chaque code à barres d'ADN étant marqué avec un groupe rapporteur, le complément de liaison spécifique lié aux sondes de détection de microparticules se liant au moins à un second site de liaison de l'analyte cible ;
c) mettre en contact les substrats de capture avec un échantillon supposé contenir des analytes cibles dans des conditions efficaces pour permettre la liaison de l'analyte cible spécifique au complément de liaison spécifique lié au substrat de capture pour immobiliser les analytes cibles sur les substrats de capture ;
d) mettre en contact les analytes cibles immobilisés avec au moins un type de sondes de détection de microparticules dans des conditions efficaces pour permettre la liaison entre les analytes cibles et le complément de liaison spécifique lié aux sondes de détection de microparticules à l'analyte et pour former un complexe en présence de l'analyte cible sur le substrat de capture ;
e) facultativement, isoler et laver le substrat de capture pour éliminer les sondes de détection de microparticules non liées ;
f) soumettre le complexe à des conditions efficaces pour libérer les codes à barres d'ADN des sondes de détection de microparticules ; et
g) détecter les codes à barres d'ADN libérés, la présence des codes à barres d'ADN libérés indiquant la présence du complexe ; les codes à barres d'ADN libérés étant détectés par détection du groupe rapporteur de l'étape 1b(ii) sur les codes à barres libérés.

2. Procédé selon la revendication 1, dans lequel l'analyte cible est une protéine ou un haptène et son complément de liaison spécifique est un anticorps comprenant un anticorps monoclonal ou polyclonal.

3. Procédé selon la revendication 1, dans lequel l'analyte cible est une molécule d'acide nucléique.

4. Procédé selon l'une quelconque des revendications 1 à 3, dans lequel le groupe rapporteur comprend la biotine, un fluorophore, un chromophore, un groupe à activité redox, un groupe avec une signature électrique, groupe radioactif, un groupe catalytique, ou marqueur de Raman.

5. Procédé selon l'une quelconque des revendications 1 à 4, dans lequel une pluralité de codes à barres d'ADN sont attachés à la microparticule.

6. Procédé selon l'une quelconque des revendications 1 à 5, comprenant en outre l'étape consistant à laver le substrat de capture après l'immobilisation des analytes cibles sur celui-ci et avant la mise en contact du substrat de capture avec les sondes de détection de microparticules.

7. Procédé selon la revendication 5, dans lequel les codes à barres d'ADN libérés sont immobilisés sur un substrat avant ladite détection.

8. Procédé selon la revendication 5, dans lequel les codes à barres d'ADN sont directement liés à la microparticule.

9. Procédé selon la revendication 8, dans lequel les codes à barres d'ADN sont libérés de la particule par un agent de libération chimique.

10. Procédé selon la revendication 9, dans lequel l'agent de libération chimique est le dithiothréitol.

11. Procédé selon la revendication 5, dans lequel la microparticule comprend en outre des oligonucléotides liés à celle-ci et les codes à barres d'ADN sont hybridés à au moins une partie des oligonucléotides liés à la microparticule.

12. Procédé selon la revendication 11, dans lequel les codes à barres d'ADN sont libérés par déshybridation.

13. Procédé selon l'une quelconque des revendications 1 à 12, dans lequel le substrat de capture est un substrat magnétique.

14. Procédé selon la revendication 13, dans lequel ledit isolement comprend la soumission du substrat magnétique à un champ magnétique.

15. Procédé selon la revendication 14, dans lequel, après ladite étape d'isolement et de lavage et avant ladite étape de détection, comprenant en outre la soumission du complexe à des conditions efficaces pour libérer les codes à barres d'ADN.

16. Procédé selon la revendication 15, dans lequel ledit isolement comprend la filtration, la sédimentation, la flottation ou l'hydrodynamique.

17. Procédé selon la revendication 16, dans lequel l'étape de filtration comprend une membrane qui élimine les composants d'échantillon qui ne comprennent pas de codes à barres d'ADN.

18. Procédé selon l'une quelconque des revendications 1 à 17, dans lequel la microparticule est une microparticule polymère, en verre, métallique, semiconductrice ou en céramique.

19. Procédé selon la revendication 18, dans lequel la microparticule polymère comprend du polystyrène.

20. Procédé selon l'une quelconque des revendications 1 à 19, dans lequel la cible a plus de deux sites de liaison.

21. Procédé selon la revendication 20, dans lequel au moins deux types de sondes de détection de microparticules sont fournis, le premier type de sonde de détection de microparticules ayant un complément de liaison spécifique d'un premier site de liaison sur l'analyte cible et le second type de sonde de détection de microparticules ayant un complément de liaison spécifique d'un second site de liaison sur l'analyte cible.

22. Procédé selon la revendication 21, dans lequel une pluralité de types de sondes de détection de microparticules sont fournis, chaque type de sondes de détection de microparticules ayant un complément de liaison spécifique de différents sites de liaison sur l'analyte cible.

23. Procédé selon l'une quelconque des revendications 1 à 22, dans lequel le complément de liaison spécifique et l'analyte cible sont les membres d'une paire de liaison spécifique.

24. Procédé selon la revendication 23, dans lequel les membres d'une paire de liaison spécifique comprennent un acide nucléique, un oligonucléotide, un acide nucléique peptidique, un polypeptide, un anticorps, un antigène, un hydrate de carbone, une protéine, un peptide, un acide aminé, une hormone, un stéroïde, une vitamine, un médicament, un virus, des polysaccharides, des lipides, des lipopolysaccharides, des glycoprotéines, des lipoprotéines, des nucléoprotéines, des oligonucléotides, des anticorps, des immunoglobulines, l'albumine, l'hémoglobine, des facteurs de coagulation, des hormones peptidiques et protéiques, des hormones non peptidiques, des interleukines, des interférons, des cytokines, des peptides comprenant un épitope spécifique des tumeurs, des cellules, des molécules de surface cellulaire, des microorganismes, des fragments, des parties, des composants ou des produits de microorganismes, des petites molécules organiques, des acides nucléiques et des oligonucléotides, des métabolites ou des anticorps contre l'une quelconque des substances ci-dessus.

25. Procédé selon la revendication 24, dans lequel l'acide nucléique et l'oligonucléotide comprennent des gènes, de l'ARN viral et de l'ADN viral, de l'ADN bactérien, de l'ADN fongique, de l'ADN de mammifère, de l'ADNc, de l'ARNm, des fragments d'ARN et d'ADN, des oligonucléotides, des oligonucléotides synthétiques, des oligonucléotides modifiés, des acides nucléiques simple brin et double brin, et des acides nucléiques naturels et synthétiques.

26. Procédé selon la revendication 23, dans lequel la paire de liaison spécifique est constituée par un anticorps et un antigène.

27. Procédé selon la revendication 23, dans lequel la paire de liaison spécifique est constituée par un récepteur et un ligand.

28. Procédé selon la revendication 23, dans lequel la paire de liaison spécifique est constituée par une enzyme et un inhibiteur compétitif.

29. Procédé selon la revendication 23, dans lequel la paire de liaison spécifique est constituée par un médicament et une molécule cible.

30. Procédé selon la revendication 23, dans lequel la paire de liaison spécifique est deux brins d'oligonucléotides au moins partiellement complémentaires.

31. Procédé selon la revendication 23, dans lequel l'analyte cible est un acide nucléique et le complément de liaison spécifique est un oligonucléotide.

32. Procédé selon la revendication 23, dans lequel l'analyte cible est une protéine ou un haptène et le complément de liaison spécifique est un anticorps comprenant un anticorps monoclonal ou polyclonal.

33. Procédé selon la revendication 23, dans lequel l'analyte cible est une séquence d'un échantillon d'ADN génomique et les compléments de liaison spécifiques sont des oligonucléotides, les oligonucléotides ayant une séquence qui est complémentaire d'au moins une partie de la séquence génomique.

34. Procédé selon la revendication 33, dans lequel l'ADN génomique est de l'ADN eucaryote, bactérien, fongique ou viral.

35. Procédé selon la revendication 23, dans lequel l'analyte cible est une séquence d'un échantillon d'ADN épisomique et les compléments de liaison spécifiques sont des oligonucléotides, lesdits oligonucléotides ayant une séquence qui est complémentaire d'au moins une partie de la séquence d'ADN épisomique.

36. Procédé selon la revendication 23, dans lequel le complément de liaison spécifique et l'analyte cible sont les membres d'une paire anticorps-ligand.

37. Procédé selon l'une quelconque des revendications 1 à 36, dans lequel outre son premier site de liaison, l'analyte cible a été modifié pour inclure un second site de liaison.
